(19) Europäisches Patentamt
European Patent Office
Office européen des brevets

(11) Publication number : 0 333 185 B1

(12) # EUROPEAN PATENT SPECIFICATION

(45) Date of publication of patent specification : **28.12.94 Bulletin 94/52**

(51) Int. Cl.$^5$ : **G03C 7/34,** // C07D213/65

(21) Application number : **89104700.3**

(22) Date of filing : **16.03.89**

(54) **Cyan dye-forming coupler and silver halide photosensitive material containing the same.**

(30) Priority : **16.03.88 JP 62825/88**

(43) Date of publication of application : **20.09.89 Bulletin 89/38**

(45) Publication of the grant of the patent : **28.12.94 Bulletin 94/52**

(84) Designated Contracting States : **CH DE FR GB IT LI NL**

(56) References cited :
**GB-A- 564 713**
**GB-A- 1 591 641**
**US-A- 4 346 161**

(73) Proprietor : **FUJI PHOTO FILM CO., LTD.
No. 210, Nakanuma
Minami-Ashigara-shi
Kanagawa-ken (JP)**

(72) Inventor : **Aoki, Kozo c/o Fuji Photo Film Co., Ltd.
210, Nakanuma
Minami-Ashigara-shi Kanagawa-ken (JP)**
Inventor : **Ono, Michio c/o Fuji Photo Film Co., Ltd.
210, Nakanuma
Minami-Ashigara-shi Kanagawa-ken (JP)**
Inventor : **Yamakawa, Katsuyoshi c/o Fuji Photo Film Co., Ltd
210, Nakanuma
Minami-Ashigara-shi Kanagawa-ken (JP)**
Inventor : **Yamazaki, Shigeru c/o Fuji Photo Film Co., Ltd.
210, Nakanuma
Minami-Ashigara-shi Kanagawa-ken (JP)**
Inventor : **Sakanoue, Kei c/o Fuji Photo Film Co., Ltd.
210, Nakanuma
Minami-Ashigara-shi Kanagawa-ken (JP)**

(74) Representative : **Patentanwälte Grünecker, Kinkeldey, Stockmair & Partner
Maximilianstrasse 58
D-80538 München (DE)**

**EP 0 333 185 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel cyan dye-forming couplers usable for forming silver halide color photographic materials and silver halide photosensitive materials containing them.

A color image is formed by exposing a silver halide photosensitive material and then subjecting it to a color developing treatment in which a developing agent such as an aromatic primary amine oxidized with the silver halide is reacted with a dye-forming coupler. In this process, colors are reproduced by a subtractive color photography in which blue, green and red colors are reproduced by forming images with yellow, magenta and cyan dyes which are complementary colors.

Phenols and naphthols are often used as cyan color image-forming couplers. There are some problems in the stability of the color images formed with the phenols or naphthols. For example, color images formed with 2-acylaminophenol cyan couplers described in US-A-2,367,531, 2,369,929, 2,423,730 and 2,801,171 usually have a poor fastness to heat, those formed with 2,5-diacrylaminophenol cyan couplers described in US-A-2,772,162 and 2,895,826 usually have a poor fastness to light, and those formed with 1-hydroxy-2-naphthamide cyan coupler usually have poor fastness to both light and heat (particularly heat and humidity.)

Couplers free from these defcets of the cyan dye-forming couplers have been developed. They include, for example, 5-hydroxy-6-acylaminocarbostyryl cyan couplers described in US-A- 4,327,173 and 4,564,586; and 4-hydroxy-5-acylaminoxyindole couplers and 4-hydroxy-5-acylamino-2,3-dihydro-1,3-benzimidazole-2-on couplers described in US-A- No. 4,430,423. These couplers have excellent light resistance and fastness to heat. They are specific couplers having a hetero atom in the color-developing mother nucleus. The rings having a color-developing dissociating group have the same valence as that of phenol.

As for the couplers having a hetero atom in the ring having the group which can be dissociated, only 3-hydroxypyridine and 2,6-dihydroxypyridine are described in US-A- 2,293,004. However, when 3-hydroxypyridine described in this U.S. Patent is used, the absorption wave length is quite short and the absorption peak is broad. Further 3-hydroxypyridine is soluble in water. Therefore, 3-hydroxypyridine could not be used as the so-called cyan coupler.

The object of the present invention is to provide new cyan couplers having a hetero atom in a ring having a group which can be dissociated. The new cyan couplers of the present invention have excellent light resistance and fastness to heat like 5-hydroxy-6-acylaminocarbostyryl cyan couplers described in US-A- 4,327,173 and 4,564,586 and 4-hydroxy-5-acylaminoxyindole couplers described in US-A- 4,430,423. They have also other advantages that the absorption characters of the developed dye are excellent, namely the absorption waves are sharp, and that the color reproducibility can be improved or, in other words, the visible absorption in a short wave length region unnecessary in the reproduction of colors is only slight and the color reproduction region is widened.

Fig. 1 shows absorption spectra of dyes obtained with Coupler (1) and Comparative Coupler (C-1).

The present invention relates to cyan dye-forming couplers of the following formula (I):

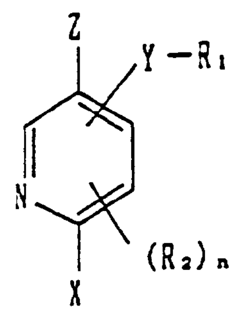

(I)

wherein $R_1$ represents an aliphatic group, an aromatic group or a heterocyclic group; $R_2$ represents a group or atom which can be bonded with the pyridine ring; X represents a hydrogen atom or a group which can be coupled off; Y represents a divalent bonding group having at least one amido bond and/or ester bond;
Z represents a dissociation group having an acid dissociation constant PKa of 5 to 12; and n represents 0, 1 or 2 with the proviso that when n is 2, two $R_2$'s does or does not be the same or different groups or atoms and they do or do not combine to form a ring, when n is 1 or more, $R_1$ may form a ring together with $R_2$; at least one of $R_1$, $R_2$ and X may have one or more pyridyl residues of general formula (I) from which $R_1$, $R_2$ or X has been removed as the substituent(s); and when n is 2, one of $R_2$'s is a hydroxyl group and the hydroxyl group is at o- or p-position with respect to the nitrogen atom of the pyridyl ring, another $R_2$ can be bonded with the

nitrogen atom of the pyridyl group and the hydroxyl group at o- or p-position can form a ketone by the tautomerism.

The present invention relates also to silver halide photosensitive materials containing at least one cyan dye-forming coupler of the above formula (I).

Now the description will be made on the cyan couplers represented by the above formula (I).

$R_1$ in the formula (I) represents an aliphatic group having preferably 1 to 36 carbon atoms, an aromatic group having preferably 6 to 36 carbon atoms (such as phenyl or naphthyl group) or a heterocyclic group (such as 3-pyridyl, 2-furyl or morpholino group).

The term 'aliphatic groups' herein indicates straight chain, branched or cyclic aliphatic hydrocarbon groups. They include saturated and unsaturated aliphatic hydrocarbon groups such as alkyl, alkenyl and alkynyl groups. Typical examples of them include methyl, ethyl, butyl, dodecyl, octadecyl, eicosenyl, isopropyl, tert-butyl, tert-octyl, ter-dodecyl, cyclohexyl, cyclopentyl, allyl, vinyl, 2-hexadecenyl and propargyl groups.

The term 'aromatic groups' herein indicates monocyclic or condensed polycyclic aromatic hydrocarbon groups. The term 'heterocyclic groups' herein incidates cyclic groups consisting of not only carbon atoms but also at least one hetero atom such as O, S, N or P in the ring. The heterocyclic groups comprise a 3 to 8-membered ring, preferably 5 or 6-membered ring and, if desired, the heterocyclic ring may be condensed with each other or the heterocyclic ring may be condensed with the benzene ring. They include also non-aromatic cyclic groups.

The aliphatic groups, aromatic groups and heterocyclic groups $R_1$ may be further substituted with an alkyl group, aryl group, heterocyclic group or alkoxy group (such as methoxy or 2-methoxyethoxy group), aryloxy group (such as 2,4-di-tert-amylphenoxy, 2-chlorophenoxy or 4-cyanophenoxy group), alkenyloxy group (such as 2-propenyloxy group), amino group (such as butylamino dimethylamino, anilino or N-methylanilino group), acyl group (such as acetyl or benzoyl group), ester group (such as butoxycarbonyl, phenoxycarbonyl, acetoxy, benzoyloxy, butoxysulfonyl or toluenesulfonyloxy group), amido group (such as acetylamino, ethylcarbamoyl, dimethylcarbamoyl, methanesulfonamido or butylsulfamoyl group), sulfamido group (such ad dipropylsulfamoylamino group), imido group (such as succinimido or hydantoinyl group), ureido group (such as phenylureido or dimethylureido group), aliphatic or aromatic sulfonyl group (such as methanesulfonyl or phenylsulfonyl group), aliphatic or aromatic thio group (such as ethylthio or phenylthio group), hydroxyl group, cyano group, carboxyl group, nitro group, sulfo group or halogen atom.

$R_2$ of the above formula (I) is a group which can be bonded with the pyridine ring. Preferred examples of $R_2$ include aliphatic groups (such as methyl, ethyl, butyl and dodecyl groups), aromatic groups (such as phenyl and naphthyl groups), heterocyclic groups (such as 3-pyridyl and 2-furyl groups), alkoxy groups (such as methoxy and 2-methoxyethoxy groups), aryloxy groups (such as 2,4-di-tert-amylphenoxy, 2-chlorophenoxy and 4-cyanophenoxy groups), alkenyloxy groups (such as 2-propenyloxy group), amino groups (such as butylamino, dimethylamino, anilino and N-methylanilino groups), acyl groups (such as acetyl and benzoyl groups), ester groups (such as butoxycarbonyl, phenoxycarbonyl, acetoxy, benzoyloxy, butoxysulfonyl and toluenesulfonyloxy groups), amido groups (such as acetylamino, ethylcarbamoyl, dimethylcarbamoyl, methanesulfonamido and butylsulfamoyl groups), sulfamido groups (such as succinimido and hydantoinyl groups), ureido groups, aliphatic and aromatic sulfonyl groups, aliphatic and aromatic thio groups, hydroxyl group, cyano group, carboxyl group and halogen atoms.

Examples of the groups listed above are the same as those listed for $R_1$. $R_2$ can be bonded with the nitrogen atom of the pyridine ring. In this case, $R_2$ represents an aliphatic group, aromatic group, heterocyclic group, acyl group, sulfonyl group, aliphatic or aromatic oxy group, amino group or the like. When a hydroxyl group is present at an o-position or p-position with respect to the nitrogen atom of the pyridine ring, the hydroxyl group can also be shown as a cyclic ketone by the tautomerism.

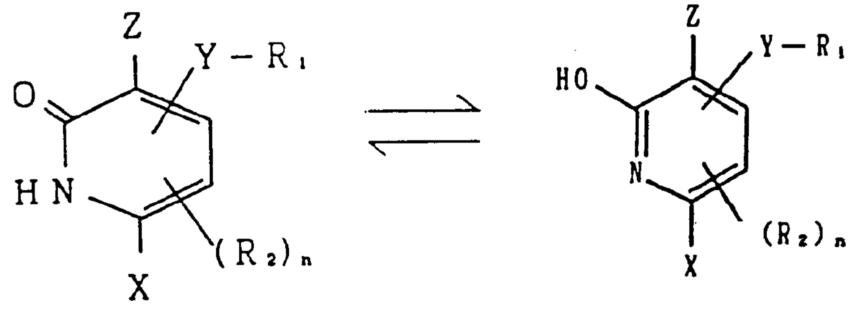

When X in the formula (I) represents a coupling-off group, it includes, for example, a group which bonds

the coupling active carbon atom with an aliphatic, aromatic or heterocyclic group, or with an aliphatic, aromatic or heterocyclic sulfonyl group or an aliphatic, aromatic or heterocyclic carbonyl group through an oxygen, nitrogen, sulfur or carbon atom, or it is a halogen atom or an aromatic azo group. The aliphatic, aromatic or heterocyclic group in the coupling-off group may be substituted with substituent(s) as described above for $R_1$. When it has two or more substituents, they may be the same or different. Further these substituents may have a substituent usable as those of $R_1$.

The coupling-off groups include, for example, halogen atoms (such as fluorine, chlorine and bromine atoms), alkoxy groups (such as ethoxy, dodecyloxy, methoxyethylcarbamoylmethoxy, carboxypropyloxy and methylsulfonylethoxy groups), aryloxy groups (such as 4-chlorophenoxy, 4-methoxyphenoxy and 4-carboxyphenoxy groups), acyloxy groups (such as acetoxy, tetradecanoyloxy and benzoyloxy groups), aliphatic or aromatic sulfonyloxy groups (such as methanesulfonyloxy and toluenesulfonyloxy groups), acylamino groups (such as dichloroacetylamino and heptafluorobutyrylamino groups), aliphatic or aromatic sulfonamido groups (such as methanesulfonamino and p-toluenesulfonylamino groups), alkoxycarbonyloxy groups (such as ethoxycarbonyloxy and benzyloxycarbonyloxy groups), aryloxycarbonyloxy groups (such as phenoxycarbonyloxy groups), aliphatic, aromatic or heterocyclic thio groups (such as ethylthio, phenylthio and tetrazolylthio groups), carbamoylamino groups (such as N-methylcarbamoylamino and N-phenylcarbamoylamino groups), five-membered or six-membered nitrogen-containing heterocyclic groups (such as imidazolyl, pyrazolyl, triazolyl, tetrazolyl and 1,2-dihydro-2-oxo-1-pyridyl groups), imido groups (such as succinimido and hydantoinyl groups), and aromatic azo groups such as phenylazo group). These groups may be substituted with the groups acceptable as the substituents for $R_1$. The coupling-off groups bonded through carbon atom include bis-type couplers formed by condensing tetravalent couplers with an aldehyde or ketone. The coupling-off groups of the present invention may contain a photographically useful group such as as that useful for inhibiting or accelerating the development.

Y of the formula (I) is a divalent bonding group having at least an amido bond or an ester bond. They include, for example, the following groups:

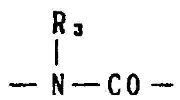 、 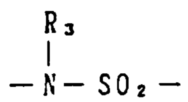 、 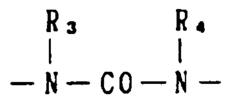 、

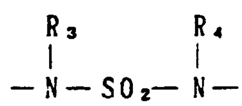 、 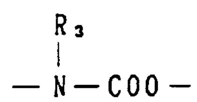 、

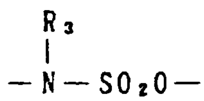 、 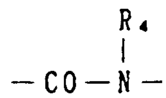

and

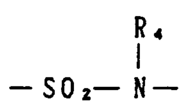

wherein $R_3$ and $R_4$ each represent a hydrogen atom, an aliphatic group, an aromatic group or a heterocyclic group.

It is preferred that the nitrogen atom of the pyridine ring in the formula (I) has no substituent. Y of the formula (I) is preferably

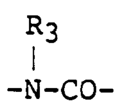 or 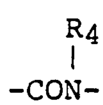

and more preferably -NHCO- which is bonded with the pyridine ring through the nitrogen atom.

X of the formula (I) is preferably a hydrogen atom, halogen atom, aliphatic or aromatic oxy group, aliphatic or aromatic thio group, aliphatic or aromatic oxycarbonyloxy group, aliphatic or aromatic acyloxy group or aliphatic or aromatic sulfonyloxy group.

When $R_1$ and $R_2$ or $R_2$'s of the formula (I) form a ring, the ring is preferably 5 to 7-membered one, more preferably 5- or 6-membered one. The ring may comprise an N or O atom in addition to carbon atoms and it may be aromatic or non-aromatic.

Examples for Z are a hydroxyl group, a sulfonamido group which can be substituted with an aliphatic, aromatic or heterocyclic group or an active methine group having at least 1, preferably 2 electron-attractive groups (such as acyl and cyano groups).

Z is preferably a hydroxyl group or aromatic sulfonamido group. Among them, the hydroxyl group is particularly preferred.

-Y-$R_1$ is arranged in 2-position or 5-position with respect to N of the pyridine ring. A coupler of the present invention wherein -Y-$R_1$ is arranged at 2-position is particularly preferred, since the absorption of the azomethine dye derived from this coupler is sharp and a high molecular extinction coefficient is obtained.

The coupler of the formula (I) has preferably a so-called ballast group having 8 or more carbon atoms in at least one of $R_1$, $R_2$, X and Z in order to introduce it stably into the color photographic sensitive material.

At least one of $R_1$, $R_2$ and X in the formula (I) for the coupler of the present invention may have one or more pyridyl groups of the formula (I) from which $R_1$, $R_2$ or X has been removed. Namely, at least one of $R_1$, $R_2$ and X of the coupler of the present invention has a pyridyl group of the following formula to form an oligomer or polymer:

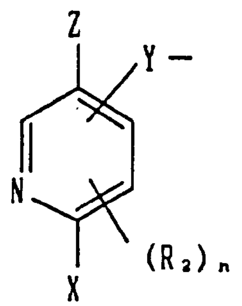

,

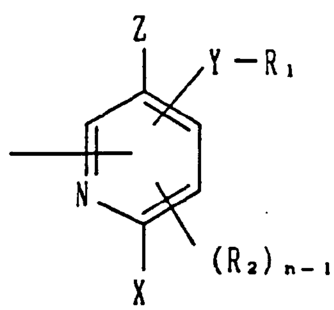

or

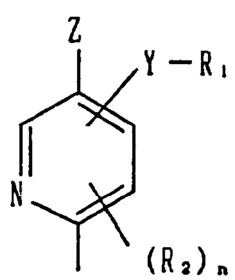

Among the couplers of the present invention, those of the formula (I) having one pyridyl group from which $R_1$, $R_2$ or X has been removed will be referred to as dimers and those having two such pyridyl groups will be referred to as trimers. The polymers include, for example, vinyl polymers having at least one of the above-described three pyridyl groups in $R_1$, $R_2$ or X. The number-average degree of polymerization of the polymer is, for example, about 10 to 1,000.

Examples of the cyan couplers of the present invention will be given below:

(1)

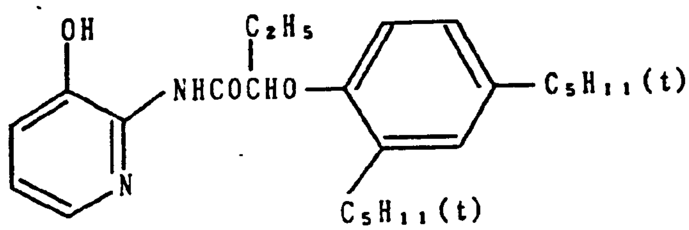

(2)

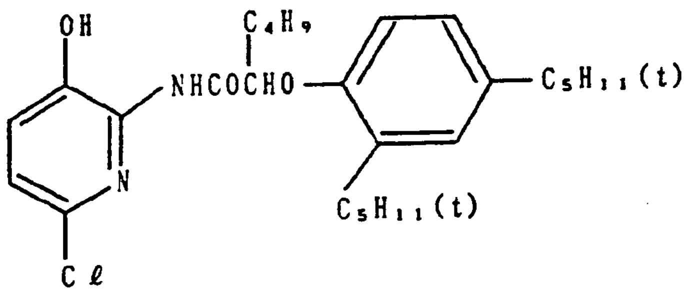

(3)

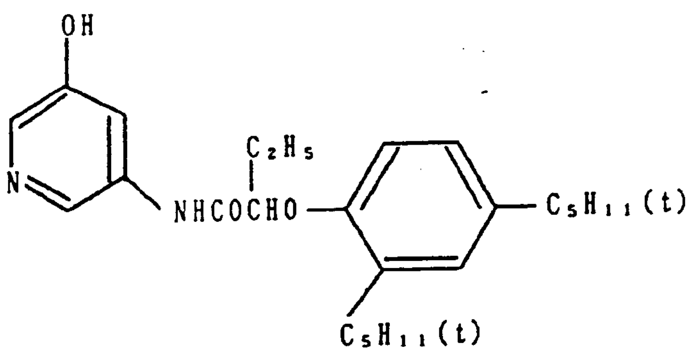

(4)

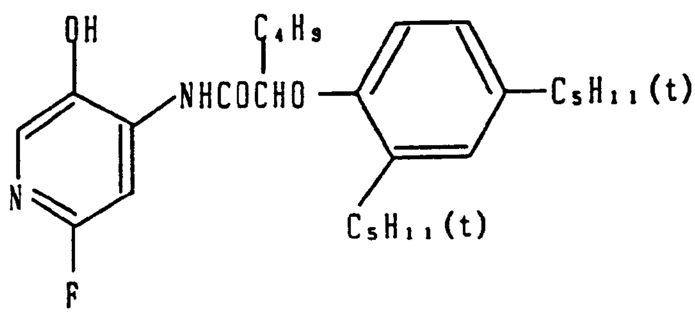

(5)

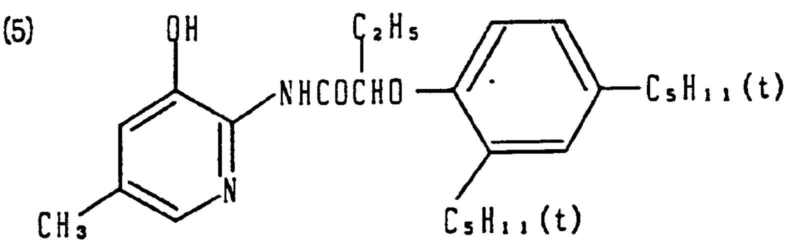

(6)

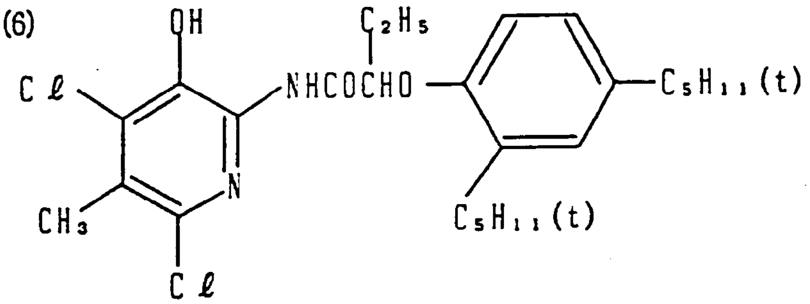

(7)

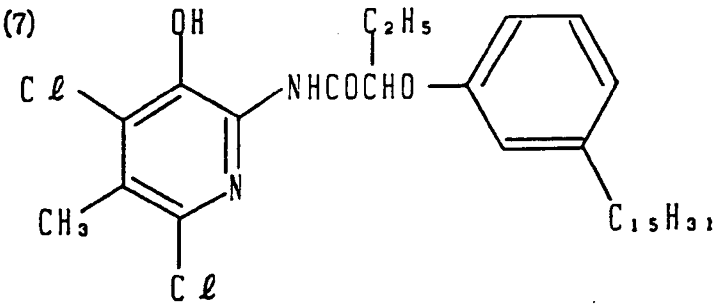

(8)

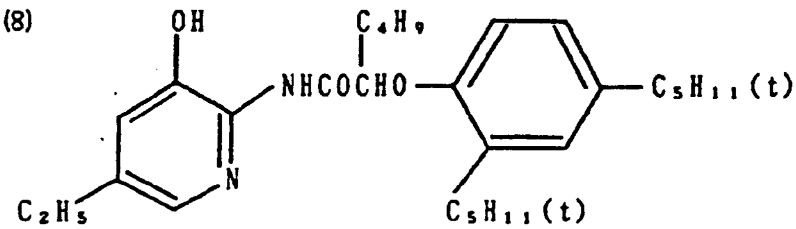

(9)

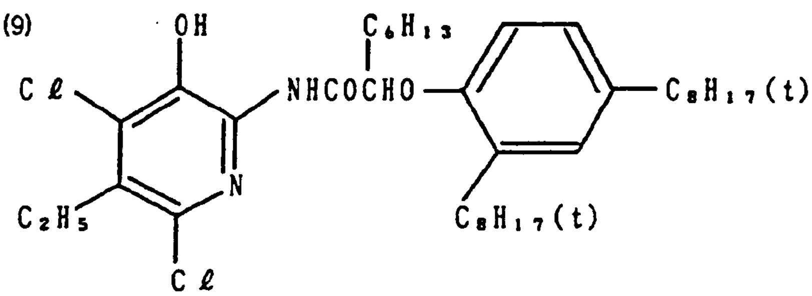

(10)

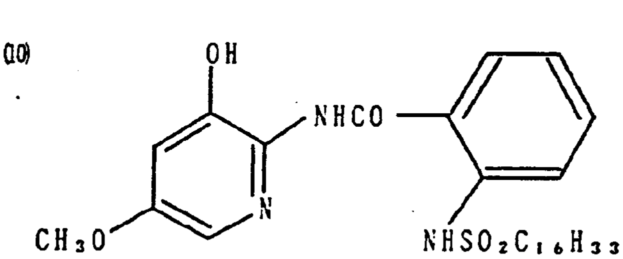

(11)

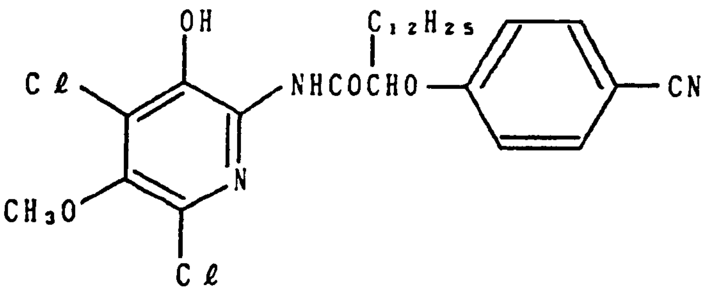

(12)

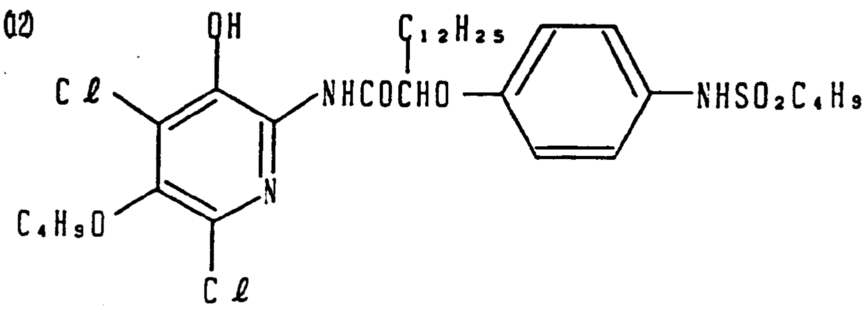

(13)

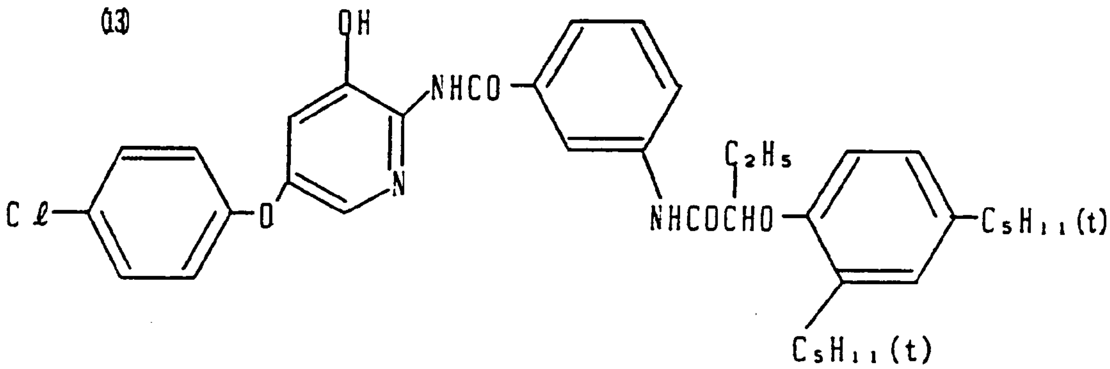

(14)

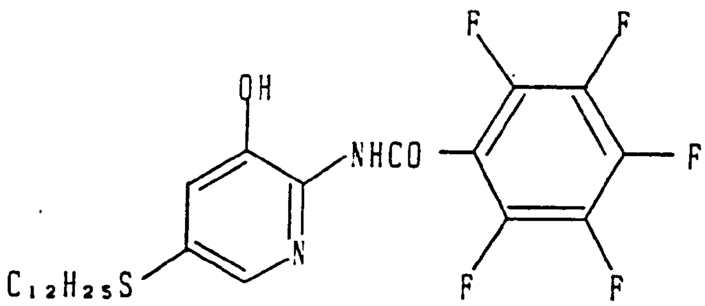

(15)

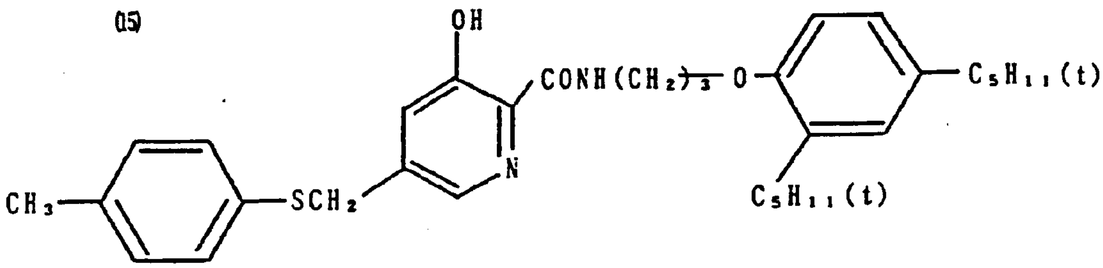

(16)

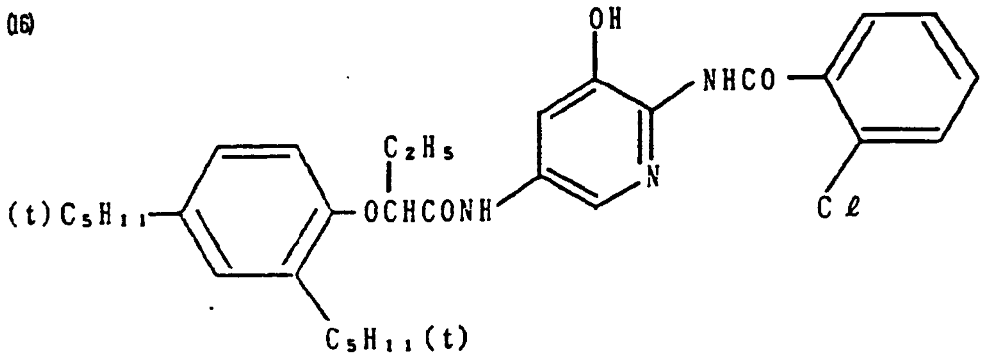

(17)

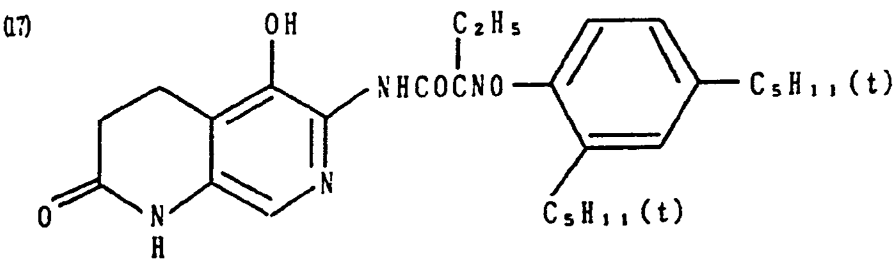

(18)

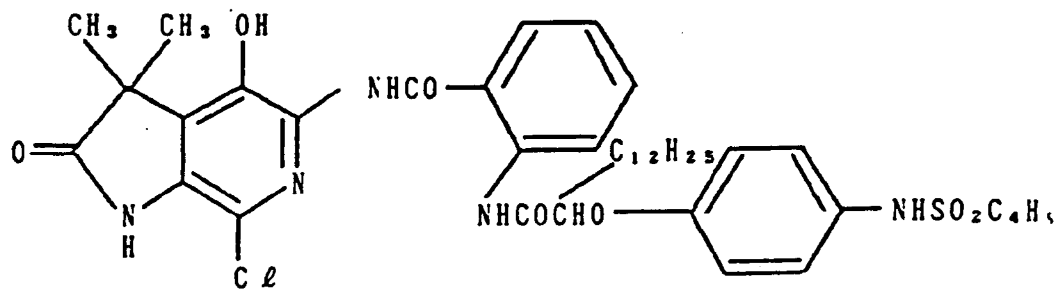

(19)

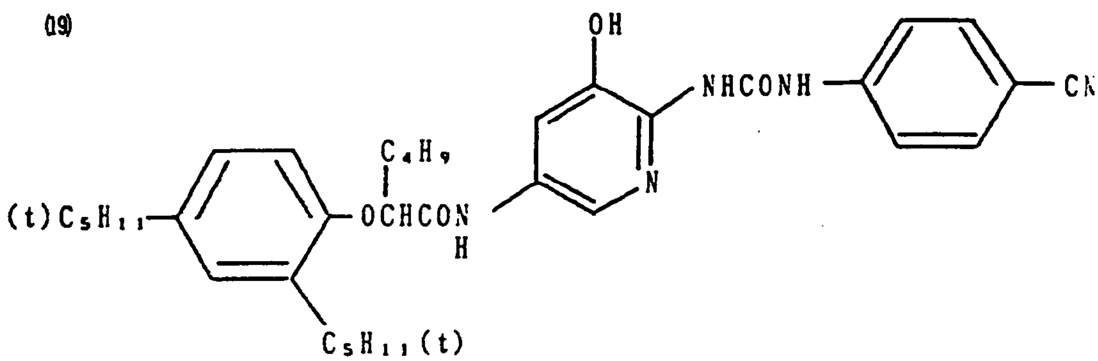

(20)

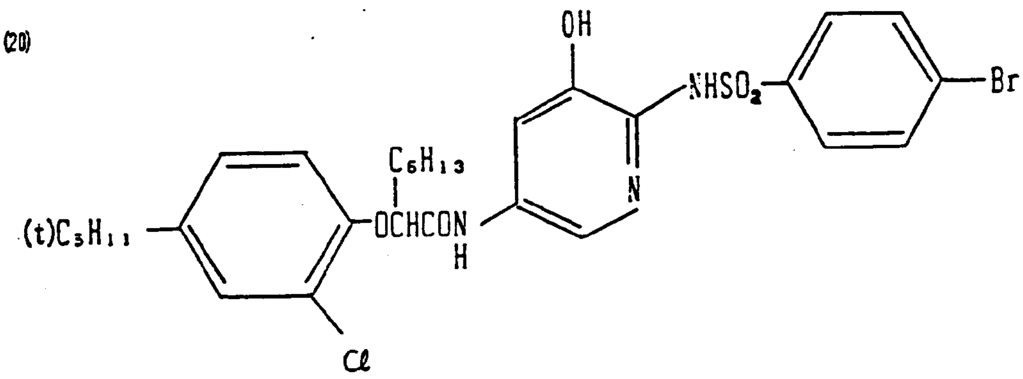

(21)

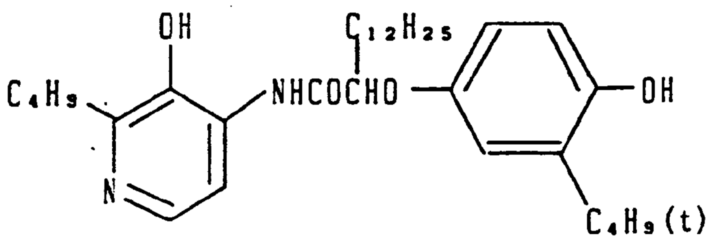

(22)

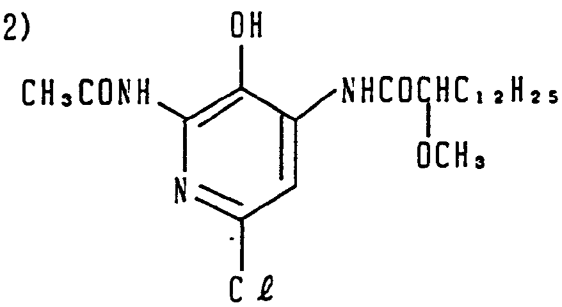

(23)

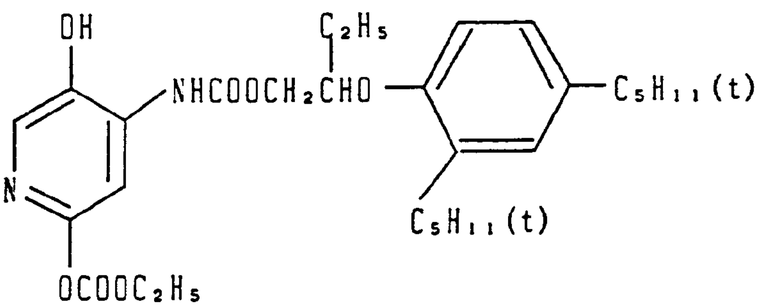

(24)

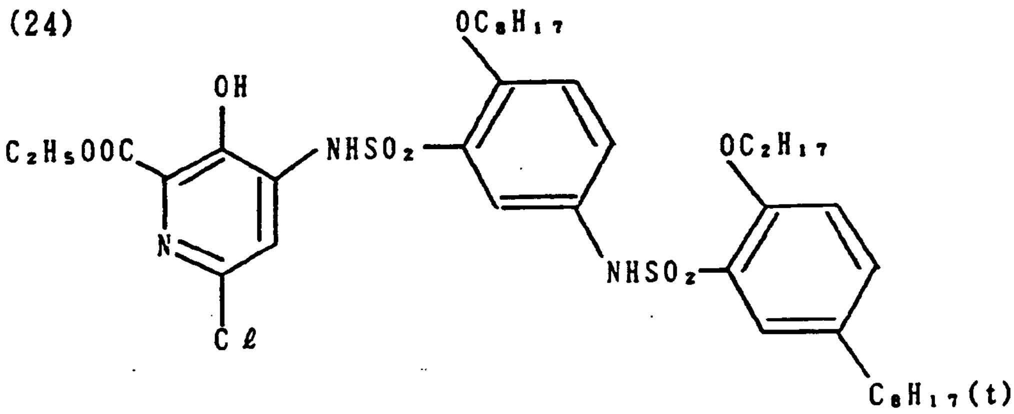

(25)

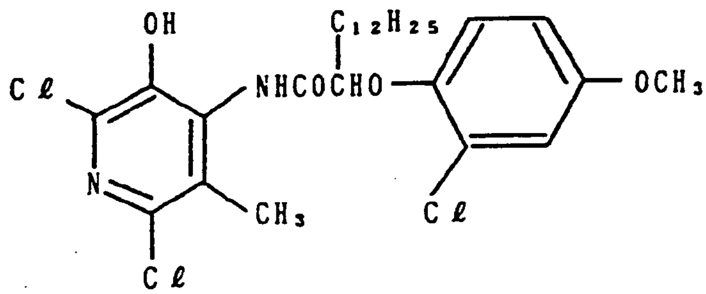

(26)

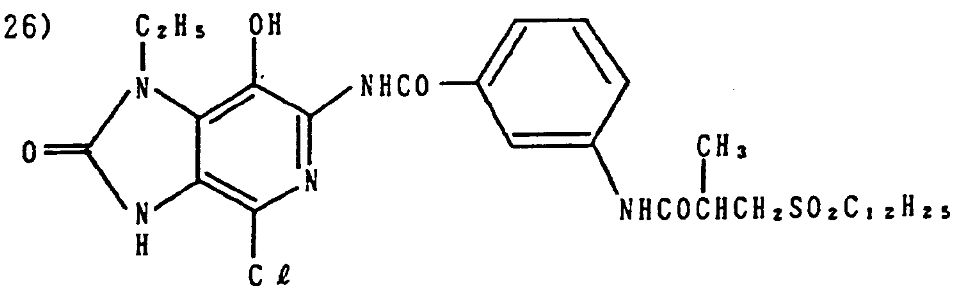

(27)

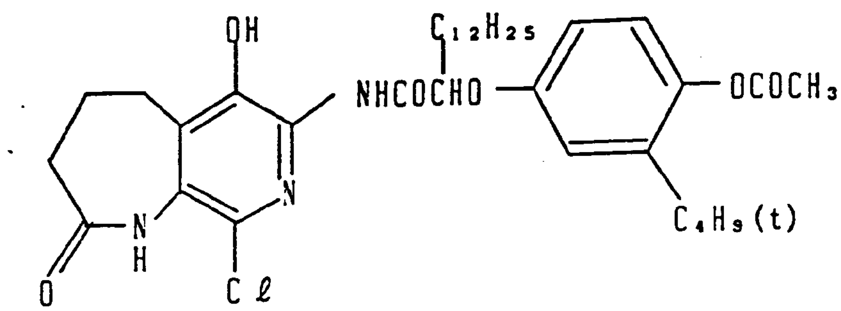

(28)

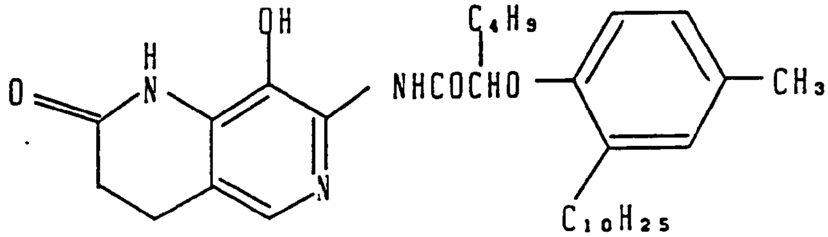

(29)

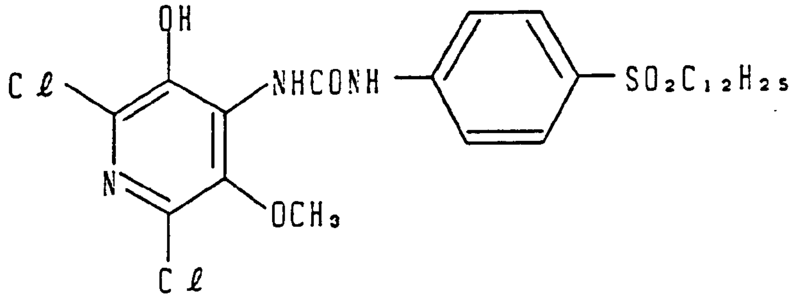

(30)

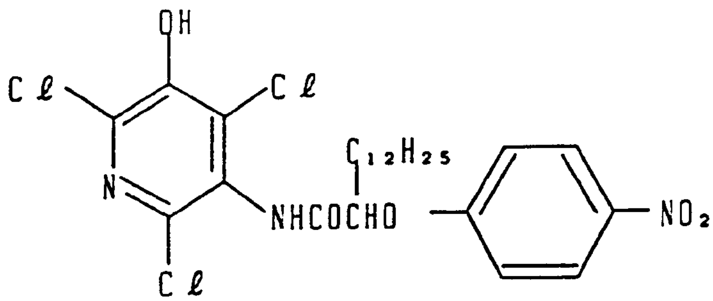

(31)

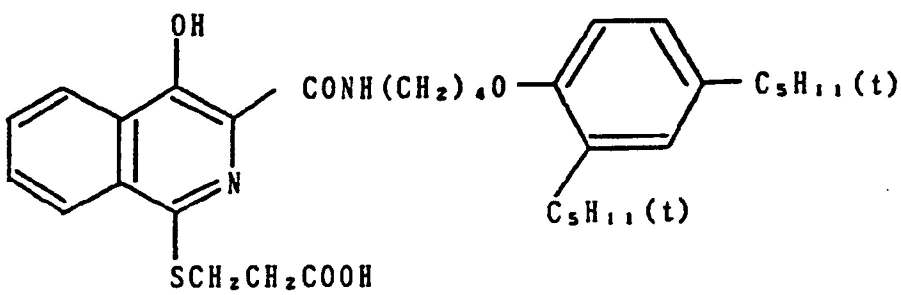

(32)

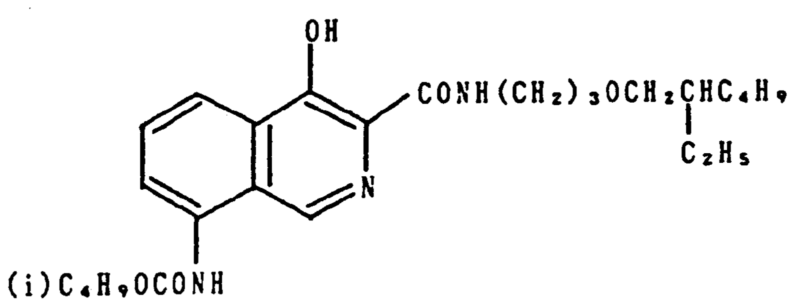

(33)

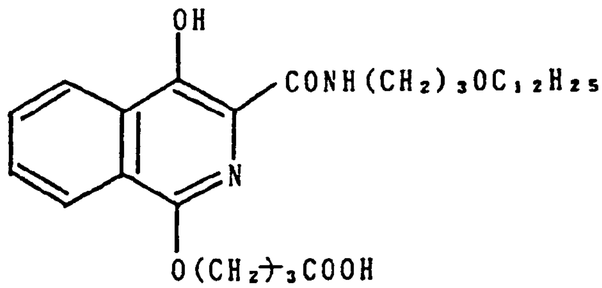

(34)

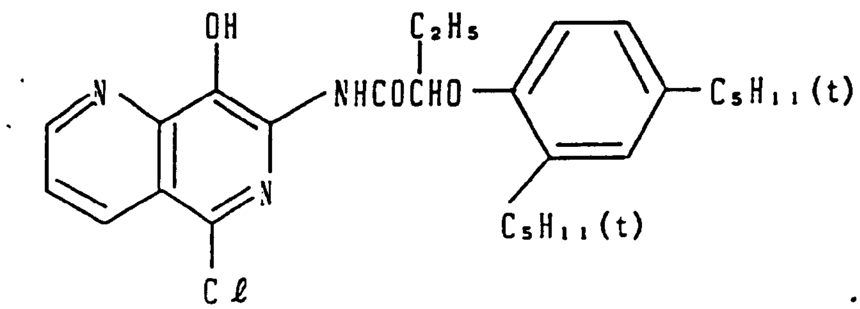

(35)

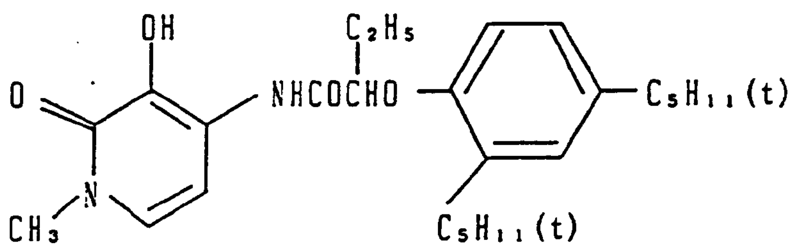

(36)

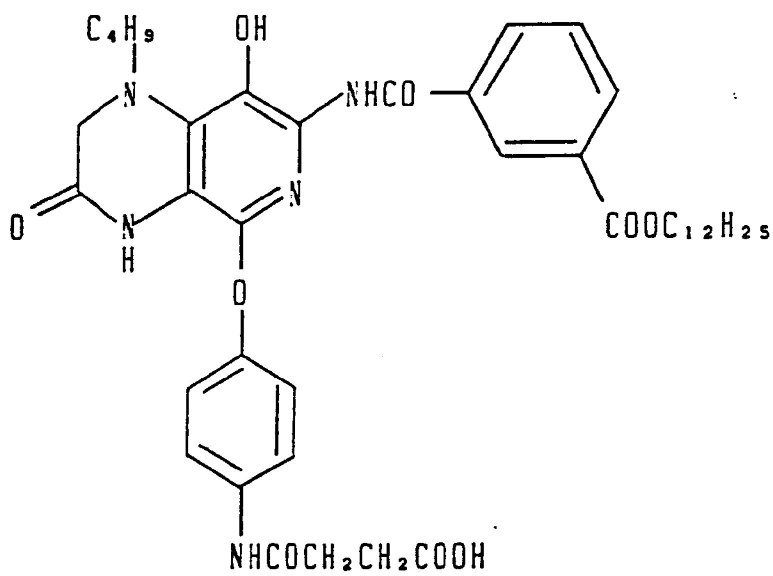

(37)

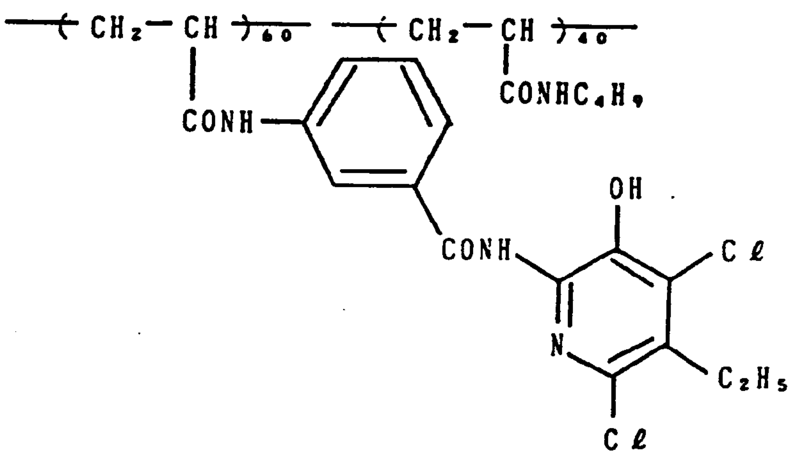

(38)

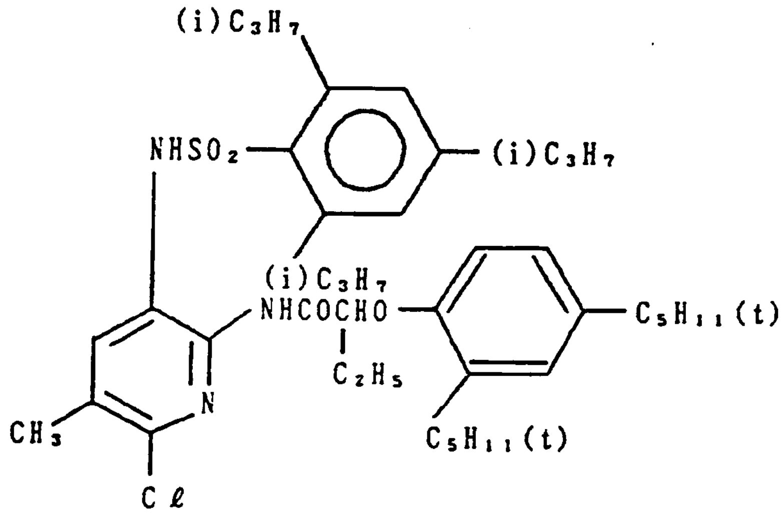

(39)

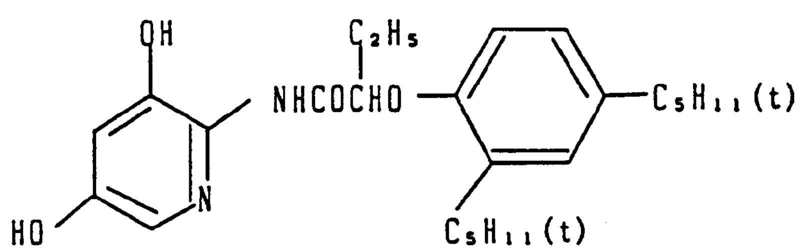

(40)

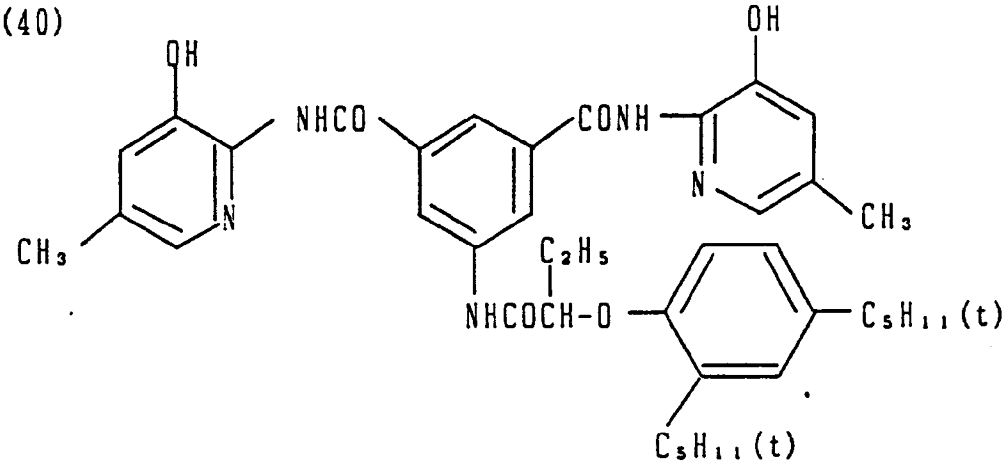

41

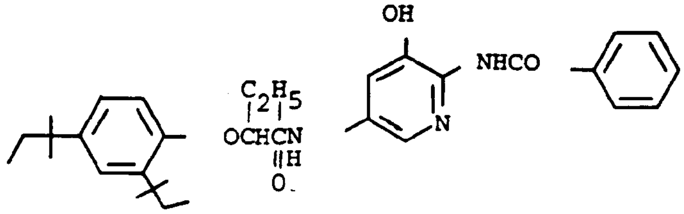

42

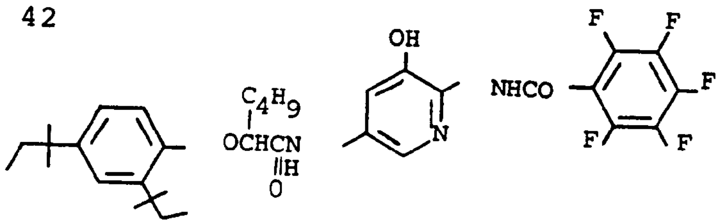

43

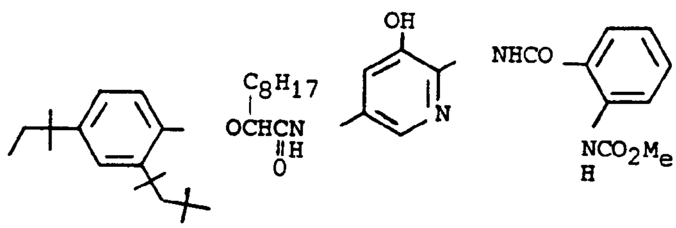

44

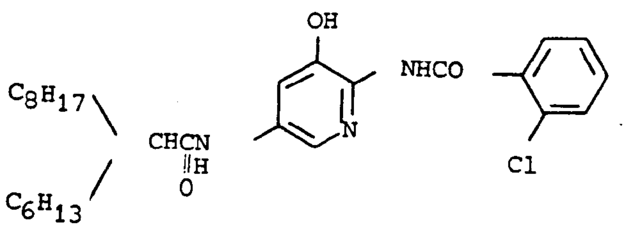

45

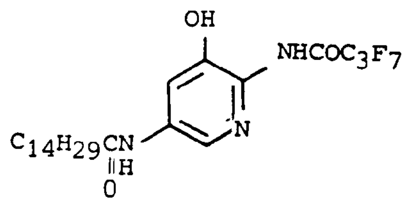

46

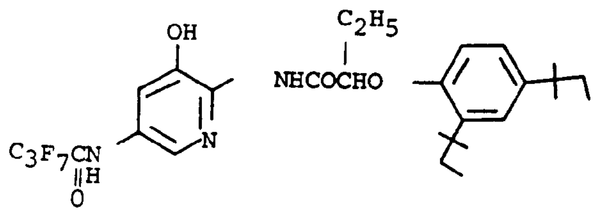

47

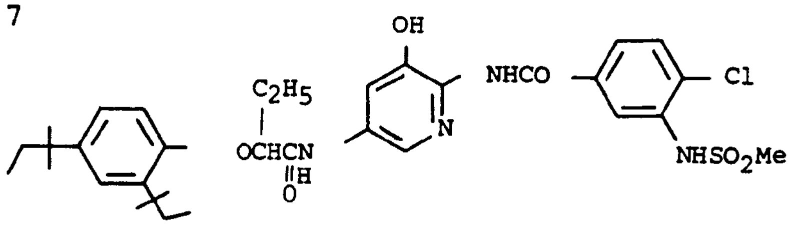

48

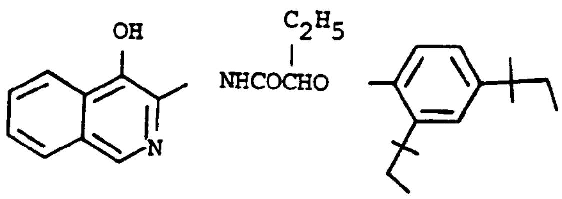

49

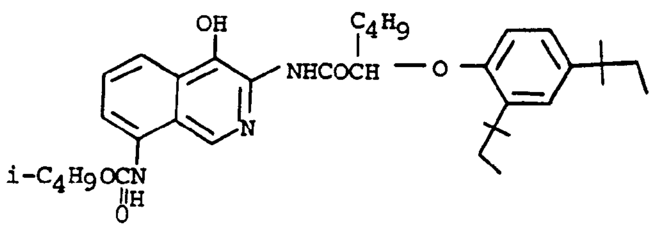

50

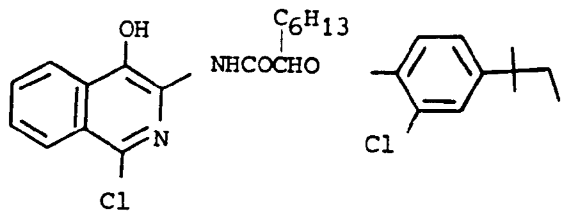

51

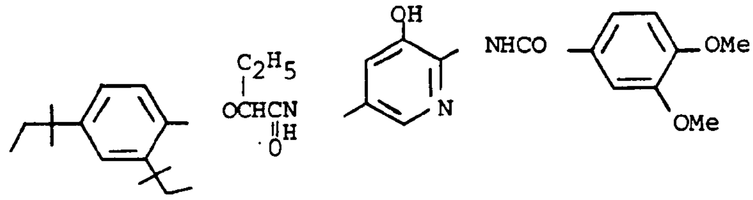

52

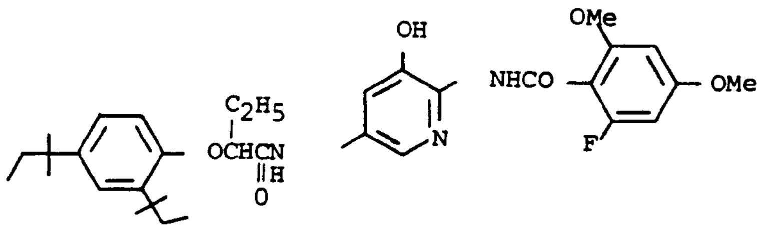

53

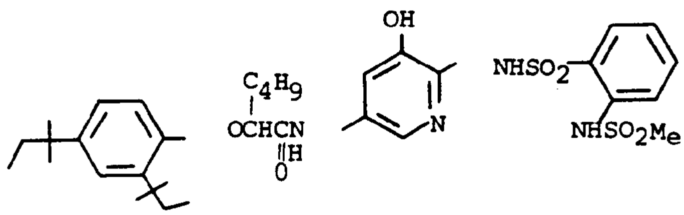

54

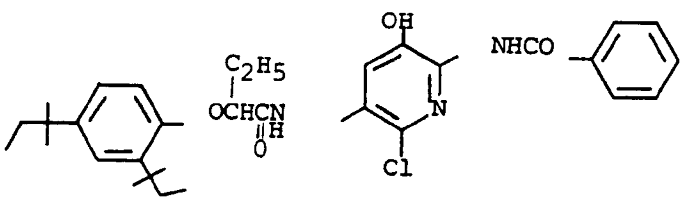

55

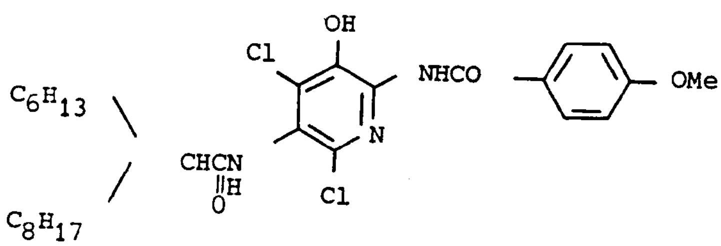

56

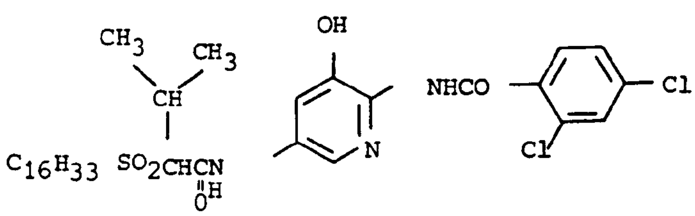

Now the description will be made on the production process of the present invention.

The couplers of the present invention can be produced by, for example, a process shown in the following scheme:

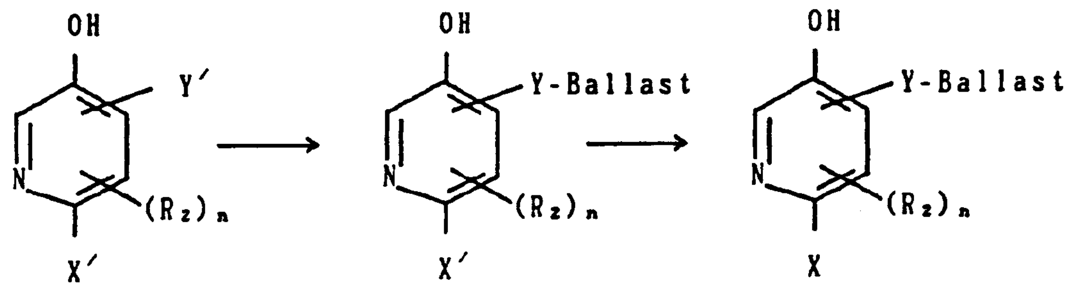

wherein Y′ represents an amino group or carbonyl group, $R_2$ and n are defined above, and X′ represents a hydrogen atom or halogen atom.

When Y′ is an amino group, the compound is reacted with an acid chloride and, on the contrary, when Y′ is a carboxyl group, the compound is reacted with an amine to form a bond by a known dehydration condensation process and a ballast group is introduced therein. If necessary, a coupling-off group (X) can be introduced therein before or preferably after the introduction of the ballast group.

The 3-hydroxypyridine derivatives used as the starting compound can be produced by a process described in Beilsteins Handbuch der Organischen Chemie or literatures referred to therein.

Processes for producing the couplers of the present invention will be illustrated in the following production examples:

Production Example 1 (Coupler (1))

11.0 g of 2-amino-3-hydroxypyridine was dissolved in 100 ml of acetonitrile and 20 ml of dimethylacetamide. 38.9 g of 2-(2′,4′-di-tert-amylphenoxy)butyric chloride was added dropwise to the solution under heating and reflux. After completion of the addition, the mixture was refluxed for additional 2 hrs and then cooled. The reaction mixture was dissolved in 250 ml of ethyl acetate. 250 ml of water was added to the solution. The solution was neutralized and then divided into layers. The ethyl acetate layer was further washed with water and then the solvent was distilled off under reduced pressure. 200 ml of acetonitrile was added to the residue to obtain a solution, which was cooled to form crystals. The crystals were taken by filtration to obtain 35.1 g of coupler (1). Melting point: 80 to 83°C.

Elementary analysis:

| | |
|---|---|
| Found: | C 72.59%, H 8.69% N 6.72% |
| Calculated: | C 72.78%, H 8.80%, N 6.79% |

Production Example 2 (Coupler (2))

2-{2′-(2″,4″-di-tert-amylphenoxy)-hexanoic amide}-3-hydroxypyridine was obtained in the same manner as that of Production Example 1 except that 2-(2′,4′-di-tert-amylphenoxy)butyric chloride was replaced with an equimolar amount of 2-(2′,4′-di-tert-amylphenoxy)hexanoic chloride.

25g of 2-{2′-(2″,4″-di-tert-amylphenoxy)hexanoic amide}-3-hydroxypyridine obtained as described above was dissolved in 200 ml of methylene chloride. 8.4 g of sulfuryl chloride was added dropwise thereto under stirring at room temperature. 20 min after completion of the addition, the liquid reaction mixture was poured into water. After extraction with methylene chloride followed by concentration, the residue was purified according to silica gel column chromatography to obtain 18 g of coupler (2) as an oily substance.

Production Example 3 (Coupler (3))

Production of 3-[2′-(2″,5″-di-tert-amylphenoxy)butanoic amide]-5-methoxypyridine

10 g of 3-amino-5-methoxypyridine was dissolved in 30 ml of dimethylacetamide and 13.5 ml of triethylamine. 27.3 g of 2-(2-(2,5-di-tert-amylphenoxy)-butanoic chloride was added dropwise to the solution under stirring. After conducting the reaction for 20 min, the reaction liquor was poured into water and then extracted

with ethyl acetate. After drying over anhydrous sodium sulfate followed by concentration and purification according to silica gel column chromatography, 15 g of 3-[2′-(2″,5″-di-tert-amylphenoxy)butanoic amide]-5-methoxypyridine was obtained as an oily substance.

Production of coupler (3)

9 g of 3-[2′-(2″,5″-di-tert-amylphenoxy)-butanoic amide]-5-methoxylpyridine produced as described above and 3 g of methylmercaptan were dissolved in DMF. 2.1 g of 60% sodium hydride was slowly added to the solution under stirring while cooling with ice. After completion of the addition, the mixture was stirred at 80°C for 2 hrs and the liquid reaction mixture was poured into water. The mixture was acidified with acetic acid and then subjected to extraction with ethyl acetate. The organic layer was dried over anhydrous sodium sulfate and concentrated. The residue was purified according to silica gel column chromatography to obtain 4.2 g of the intended compound (coupler (3)) in the form of a white powder.

Production Example 4 (Coupler (5))

25.5 g of 6-amino-3-picoline was dissolved in 100 ml of acetonitrile. 30.5 g of pivalic chloride was added dropwise to the solution at room temperature. After completion of the addition, the mixture was heated under reflux for 30 min and then cooled. It was poured into 100 ml of water and adjusted to pH 8 with sodium carbonate. After extraction with chloroform, the solvent was distilled off under reduced pressure to obtain 44.7 g of crystalline 6-pivaloylamino-3-picoline. This compound was suspended in 500 ml of chloroform and then 44 g of m-chloroperbenzoic acid was added in portions thereto. After completion of the addition, the mixture was stirred for 2 hrs and then left to stand overnight. It was concentrated under reduced pressure and then dissolved in ethyl acetate. The solution was washed with an aqueous sodium carbonate solution. The solvent was distilled off under reduced pressure to obtain 44.5 g of solid 6-pivaloylamino-3-picoline oxide. It was dissolved in 200 ml of chloroform. 54 ml of acetic anhydride was added to the solution. 43 g Of bromine diluted with 100 ml of chloroform was added dropwise thereto under cooling with ice. After completion of the addition, the mixture was left to stand at room temperature for 30 min. 23 g of sodium acetate was added thereto and the mixture was heated under reflux for 3 hrs. A small amount of water was added thereto. After neutralization with sodium carbonate, the chloroform layer was taken and concentrated under reduced pressure. The residue was taken according to silica gel column chromatography (solvent: 5% methanol, chloroform) to obtain 28 g of 5-bromo-6-pivaloylamino-3-picoline oxide. It was dissolved in 80 ml of methanol. 0.15 mol of sodium methoxide was added to the solution and the mixture was heated under reflux for 40 hrs. After cooling followed by neutralization with oxalic acid and concentration, the product was dissolved in chloroform and an insoluble matter was removed. The solvent was distilled under reduced pressure to obtain 26 g of 5-methoxy-6-pivaloylamino-3-picoline oxide. This product was added to 200 ml of methanol and 50 ml of 6 N hydrochloric acid and the mixture was heated under reflux for 52 hrs. After neutralization of hydrochloric acid followed by extraction with chloroform, the solvent was distilled off under reduced pressure. 200 ml of acetonitrile was added thereto and 33.8 g of 2-(2′-(2′,4′-di-tert-amylphenoxy)butyric chloride was added dropwise thereto under heating and reflux. The mixture was cooled and crystals thus formed were collected by filtration to obtain 35.4 g of 5-methoxy-6-{2′-(2′,4′-di-tert-amylphenoxy)butyroylamino}-3-picoline oxide. It was dissolved in 500 ml of chloroform. 55 g of phosphorus trichloride was added to the solution at room temperature. The mixture was heated under reflux for 1 hr. After cooling, the mixture was poured into 500 g of ice. pH of the mixture was adjusted at 10 with aqueous ammonia. After extraction with ethyl acetate, the solvent was distilled off under reduced pressure to obtain 31.8 g of 5-methoxy-6-{2-(2′,4′-tert-amylphenoxy)butyroylamino}-3-picoline. It was dissolved in 200 ml of chloroform. 22.5 g of boron tribromide was added dropwise thereto under cooling with ice. Then the temperature was slowly elevated to room temperature and the stirring was continued at room temperature for 2 hrs. The reaction mixture was poured in ice/water and then neutralized with sodium carbonate. After extraction with chloroform, the solvent was distilled off under reduced pressure. The residue was taken according to silica gel column chromatography (solvent: chloroform). After recrystallization from ethyl acetate/acetonitrile, 22.6 g of Coupler (5) was obtained. Melting point: 91 to 95°C.

Elementary analysis:

| | |
|---|---|
| Found: | C 73.13%, H 8.79%, N 6.62% |
| Calculated: | C 73.20%, H 8.98%, N 6.57% |

Production Example 5 (Coupler (6))

19.6 g of 5-hydroxy-6-{2-(2′,4′-tert-amylphenoxy)-hexanoylamino}-3-picoline was formed in the same manner as that of Production Example 4 except that 2-(2′,4′-di-tert-amylphenoxy)butyric chloride was replaced with an equimolar amount of 2-(2′-,4′-di-tert-amylphenoxy)hexaconic chloride. This compound was dissolved in 80 ml of methylene chloride. 12.4 g of sulfuryl chloride was added dropwise thereto at room temperature and the mixture was stirred for 1 hr. After completion of the stirring, the liquid reaction mixture was poured into water. After extraction with methylene chloride followed by concentration, the residue was purified according to silica gel column chromatography to obtain 14.1 g of Coupler (6).

Production Example 6 (Coupler (7))

Coupler (7) was formed in the same manner as that of Production Example 5 except that 2-(2′,4′-di-tert-amylphenoxy)hexanoic chloride was replaced with an equimolar amount of 2-(3-n-pentadecylphenoxy)butyric chloride.

Production Example 7 (Coupler (8))

Coupler (8) was formed in the same manner as that of Production Example 4 except that 6-amino-3-picoline was replaced with an equimolar amount of 6-amino-3-ethylpyridine and that 2-(2′,4′-di-tert-amylphenoxy)butyric chloride was replaced with an equimolar amount of 2-(2′,4′-di-tert-amylphenoxy)hexanoic chloride.

Production Example 8 (Coupler (9))

5-Ethyl-3-hydroxy-2-{2-(2′,4′-di-tert-octylphenoxy)-octanoylamino}pyridine was formed in the same manner as that of Production Example 7 except that 2-(2′,4′-di-tert-amylphenoxy)hexanoic chloride was replaced with an equimolar amount of 2-(2′,4′-di-tert-octylphenoxy)octanoic chloride. It was then chlorinated with sulfuryl chloride in methylene chloride to form Coupler (9).

Production Example 9 (Coupler (16))

12.0 g of Coupler (3) produced in Production Example 3 was dissolved in 40 ml of acetic acid and 15 ml of acetic anhydride. 2.3 ml of concentrated nitric acid was added dropwise to the solution under cooling with water. The mixture was stirred at room temperature for additional 6 hrs. It was poured into ice/water and neutralized with sodium carbonate. After extraction with ethyl acetate, the solvent was distilled off under reduced pressure. The residue was dissolved in 100 ml of ethanol. 50 ml of water was added to the solution and then 10 g of sodium hydrosulfite was added in portions thereto under heating and reflux. After conducting the reflux until the yellow color of the reaction mixture substantially disappeared, it was cooled. Water was added to the reaction mixture and the crystals thus formed were taken by filtration. The crystals were dissolved in 30 ml of dimethylacetamide and 100 ml of acetonitrile. 5.3 g of o-chlorobenzoyl chloride was added dropwise thereto at 60°C. The mixture was refluxed for 6 hrs, then cooled and neutralized with an aqueous sodium hydroxide solution. The crystals thus formed were taken by filtration and recrystallized from acetonitrile to obtain 9.8 g of Coupler (16). Melting point: 125 to 128°C.

Elementary analysis:

| | |
|---|---|
| Found: | C 67.71%, H 7.03%, N 7.60% |
| Calculated: | C 67.89%, H 7.12%, N 7.42% |

Production Example 10 (Coupler (17))

16.4 g of 5-hydroxy-1,2,3,4-tetrahydro-1,7-naphthylidine-2-on was dispersed in 50 ml of acetic acid and 20 ml of acetic anhydride. 7.9 ml of concentrated nitric acid was added dropwise to the dispersion under cooling with water and the mixture was stirred at room temperature for 3 hrs. The reaction mixture was poured in ice/water and neutralized with sodium carbonate. After extraction with ethyl acetate, the solvent was distilled off under

reduced pressure. The resulting residue was dissolved in 300 ml of ethanol. 150 ml of an aqueous solution of 40 g of sodium hydrosulfite was slowly added thereto and the mixture was heated under reflux for 30 min. After cooling to room temperature, the mixture was poured into 450 ml of water. The crystals thus formed were taken by filtration and dissolved in 50 ml of dimethylacetamide and 150 ml of acetonitrile. 31.1 g of 2-(2,4-di-tert-amylphenoxy)butyric chloride was added dropwise to the solution under heating and reflux. After completion of the addition, the mixture was refluxed for additional 3 hrs. The mixture was cooled to room temperature. 300 ml of ethyl acetate and 300 ml of water were added thereto. After the neutralization, the mixture was divided into layers. The ethyl acetate layer was further washed with water and dried over Glauber's salt. The solvent was distilled off under reduded pressure. The residue was purified according to silica gel column chromatography (chloroform/ethyl acetate = 5/1) and then crystallized from a solvent mixture of hexane and ethyl acetate to obtain 26.4 g of intended Coupler (17).

Production Example 11 (Coupler (19))

3-{2′-(2″,4″-di-tert-amylphenoxy)hexanoylamino}-5-hydroxypyridine was produced in the same manner as that of the production of Coupler (3) except that 2-(2′,4′-di-tert-amylphenoxy)butanoic chloride used in Production Example 3 was replaced with an equimolar amount of 2-(2′,4′-di-tert-amylphenoxy)hexanoic chloride. 12.0 g of the product was dissolved in 40 ml of acetic acid and 15 ml of acetic anhydride. 2.2 ml of concentrated nitric acid was added dropwise to the solution under cooling with water. The mixture was stirred at room temperature for 6 hrs, then poured into ice/water and neutralized with sodium carbonate. After extraction with ethyl acetate, the solvent was distilled off under reduced pressure. The residue was dissolved in 100 ml of ethanol. 50 ml of water was added thereto. 10 g of sodium hydrosulfite was added in portions thereto under heating and reflux. After conducting the reflux until the yellow color of the reaction mixture substantially disappeared, it was cooled. The crystals thus formed were taken by filtration. 9.8 g of the crystals were dispersed in 80 ml of toluene. 3.2 g of p-cyanophenyl isocyanate was added to the dispersion and the mixture was stirred at 80°C for 3 hrs. The reaction mixture was poured into water. After extraction with ethyl acetate, the solvent was distilled off under reduced pressure. The residue was purified according to silica gel column chromatography to obtain 8.3 g of Coupler (19).

Production Example 12 (Coupler (20))

3-{2′-(2″-chloro-4″-tert-amylphenoxy)hexanoylamino}-5-hydroxypyridine was produced in the same manner as that of the production of Coupler (3) except that 2′-(2′,4′-tert-amylphenoxy)butanoic chloride used in Production Example 3 was replaced with an equimolar amount of 2-(2′-chloro-4′-di-tert-amylphenoxy)hexanoic chloride. 12 g of the product was dissolved in 40 ml of acetic acid and 15 ml of acetic anhydride. 2.2 ml of concentrated nitric acid was added dropwise to the solution under cooling with water. The mixture was stirred at room temperature for 6 hrs, poured into ice/water and neutralized with sodium carbonate. After extraction with ethyl acetate, the solvent was distilled off under reduced pressure. The residue was dissolved in 100 ml of ethanol. 50 ml of water was added thereto. 12 g of sodium hydrosulfite was added thereto in portions under heating and reflux. After conducting the reflux until the yellow color of the reaction mixture substantially disappeared, it was cooled. The crystals thus formed were taken by filtration. 8 g of the crystals were dissolved in 30 ml of acetonitrile. 1.7 ml of pyridine was added to the solution. A solution of 4.6 g of p-bromobenzenesulfonyl chloride in 10 ml of acetonitrile was added dropwise thereto under heating and reflux. After completion of the addition, the reaction mixture was refluxed for 3 hrs, cooled and poured into water. After extraction with ethyl acetate, the solvent was distilled off and the residue was purified according to silica gel column chromatography to obtain 6.1 g of Coupler (20).

Production Example 13 (Coupler (26))

3-hydroxy-5-phenoxycarbonylaminopyridine was produced in the same manner as that of the production of Coupler (3) (Production Example 3) except that 2-(2′,4′-di-tert-amylphenoxy)butanoic chloride was replaced with an equimolar amount of phenyl chloroformate. 20 g of the product was dissolved in 80 ml of acetic acid and 20 ml of acetic anhydride. 5.5 ml of concentration nitric acid was added dropwise to the solution under cooling with ice. The mixture was stirred at room temperature for 4 hrs, poured into ice-water and neutralized with sodium carbonate. After extraction with ethyl acetate, the solvent was distilled off under reduced pressure. The residue was dissolved in 100 ml of ethanol. 100 ml of water was added to the solution and then 20 g of sodium hydrosulfite was added thereto in small portions under heating and reflux. After conducting the reflux until the yellow color of the reaction mixture substantially disappeared, it was cooled. 100 ml of water was added

thereto and crystals thus formed were taken by filtration. 18 g of the crystals were dissolved in 80 ml of acetonitrile and 40 ml of dimethylacetamide. 33.6 g of 3-(1′-dodecylsulfonyl-2′-butanoylamino)benzoyl chloride was added to the solution and the mixture was stirred at 50°C for 1 hr. After completion of the reaction, the mixture was poured into water. 9 g of sodium acetate was added thereto. After extraction with ethyl acetate, the solvent was distilled off and the residue was dissolved in 200 ml of methylene chloride. 11 g of sulfuryl chloride was added dropwise to the solution at room temperature and the mixture was stirred for 3 hrs. The liquid reaction mixture was poured into water. After extraction with methylene chloride, the solvent was distilled off and the residue was purified according to column chromatography to obtain 12.4 g of 2-chloro-6-(1′-dodecylsulfonyl-2′-butanoylamino)-5-hydroxy-3-phenoxycarbonyl-aminopyridine. 12 g of the product was dissolved in 50 ml of chloroform. 1.1 ml of concentrated nitric acid was added dropwise to the solution at room temperature and the mixture was stirred at room temperature for 3 hrs. The reaction mixture was poured into water. After extraction with chloroform, the solvent was distilled off. The residue was dissolved in 200ml of ethanol. 1 g of Raney nickel and 1.1 g of acetaldehyde were added to the solution and the catalytic hydrogenation reaction was conducted in an autoclave. The catalyst was removed by filtration. The product was poured into water and the crystals thus formed were taken by filtration. The crude crystals were recrystallized from acetonitrile to obtain 6 g of Coupler (26).

Production Example 14 (Coupler (41))

Coupler (41) was prepared according to the method shown in the following synthesis scheme:

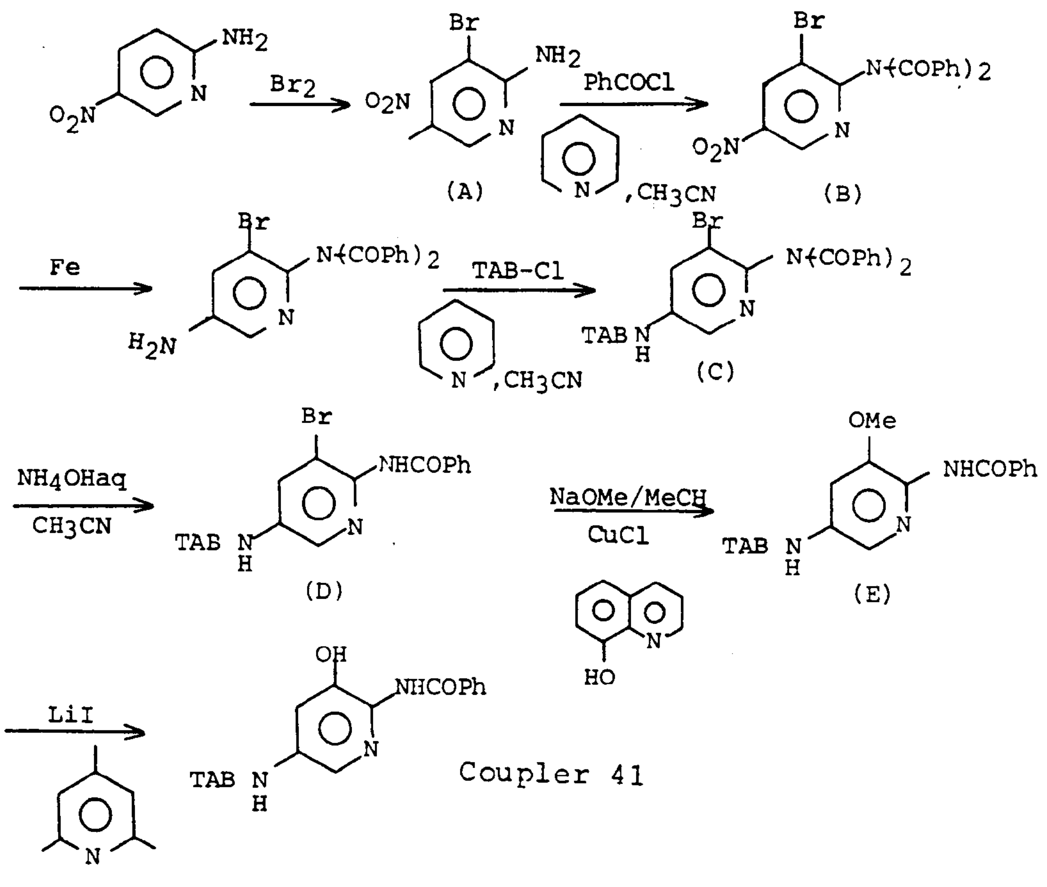

(A) (B) (C) (D) (E)
Coupler 41

Bromine (62.6 ml, 1.2 mol) was added dropwise to 2-amino-5-nitropyridine (154.1 g, 1.1 mol) in THF (300 ml) at a room temperature. The resulting mixture was stirred for 1 hr and then poured into water (3 ℓ). The crystals thus formed were taken by filtration. The resultant crude crystals were recrystallized from acetonitrile (500 ml) to obtain 2-amino-3-bromo-5-nitropyridine (A) (194.4 g, 80%).

Pyridine (20 ml) was added to 2-amino-3-bromo-5-nitropyridine (A) (21.8 g, 0.1 mol) in acetonitrile (300 ml) at a room temperature in a stream of nitrogen followed by addition of henzoylchloride (24.4 g, 0.2 mol) and agitation for 3 hrs. Then dilute hydrochloric acid (0.1 N) was added to the resulting mixture. After extraction with ethyl acetate, the resulting ethyl acetate solution was dried over anhydrous sodium sulfate. The solvent

was distilled off under reduced pressure. The residue was recrystallized from methanol to obtain 3-bromo-2-benzoylamino-5-nitropyridine (B) (28.7 g 89%).

An isopropyl alcohol (300 ml) solution containing reduced iron (42.6 g, 0.76 mol), water (42.6 ml) and ammonium chloride (4.3 g, 81 mmol) was refluxed under heating for 10 min. Then 3-bromo-2-benzoylamino-5-nitropyridine (B) (42.6 g, 100 mmol) was added to the resulting solution and refluxed under heating for 30 min. The reaction mixture was filtered with celite and the solvent was distilled off under reduced pressure. After azeatropic distillation with benzen was conducted in twice, 2-(2′,4′-di-tert-amylphenoxy)butanoyl chloride (33.8 g, 100 mol), acetonitrile (300 ml) and pyridine (10 ml) were added to the residue and stirred for 1 hr. Dilute hydrochloric acid (1 N) was added to the resulting mixture. After extraction with ethyl acetate in twice, the thus obtained ethyl acetate phase was dried over anhydrous sodium sulfate. The solvent was distilled off under reduced pressure. The residue was recrystallized from acetonitrile to obtain 5-(2-{2′-4′-di-tert-amylphenoxy}butanoylamino)-2-dibenzoylamino-3-bromopyridine (C).

28% aqueous ammonia (20 ml) was added to an acetonitrile (300 ml) solution containing the above compound (C) followed by agitation for 15 min. The mixture was poured into water and extracted with ethyl acetate in twice. The resulting ethyl acetate solution was washed with dilute hydrochloric acid (0.1 N) and aqueous solution saturated with sodium chloride and dried over anhydrous sodium sulfate. The solvent was distilled off under reduced pressure. The residue was recrystallized from acetonitrile to obtain 5-(2-{2′,4′-di-tert-amylphenoxy}butanoylamino)-2-benzoylamino-3-bromopyridine (D) (19.9 g, 34% on the basis of (B)).

In the stream of nitrogen, 28% sodium methoxide in methanol (7.1 ml, 36.9 mmol), anhydrous cuprous chloride (332 mg, 3.4 mol) and 8-hydroxy quinoline were added to a methanol solution containing compound (D) (19.9 g, 33.5 mmol) followed by reflux under heating for 3 hrs. Dilute hydrochloric acid was added to the reaction mixture and extracted with ethyl acetate in twice. The resulting ethyl acetate solution was washed with an aqueous solution saturated with sodium chloride and dried over anhydrous sodium sulfate. The solvent was distilled off and the resulting residue was recrystallized from acetonitrile to obtain 5-(2-{2′,4′-di-tert-amylphenoxy}butanoylamino)-2-benzoylamino-3-methoxypyridine (E) (10.4 g, 57%).

In a stream of nitrogen, lithium iodide (4.0 g, 29.6 mmol) was added to a 2,4,6-collidine solution containing compound (E) (3.9 g, 7.2 mmol) followed by reflux under heating for 3 hrs. After cooling, dilute hydrochloric acid (0.1 N) was added to the reaction mixture. After extraction with ethyl acetate in twice, the resulting ethyl acetate solution was washed with an aqueous solution saturated with sodium chloride and dried over anhydrous magnesium sulfate. The solvent was distilled off under reduced pressure. The residue was recrystallized from acetonitrile to obtain coupler (41) (2.9 g, 76%).

Most of the couplers of the present invention are soluble in an oil. Usually it is preferred that the coupler is dissolved in a high-boiling solvent (if necessary, in combination with a low-boiling solvent), the solution is emulsified or dispersed in an aqueous gelatin solution and the emulsion or suspension is added to a silver halide emulsion. When the coupler is soluble in an aqueous alkali solution, it is dissolved therein together with a developing agent and other additives and the solution is used for forming an image by so-called coupler-in-developer process.

Further the coupler can be used together with the developing agent and the alkali (if necessary in the presence of an organic solvent) for an oxidation coupling with an oxidizing agent (such as a persulfate, silver nitrate, nitrous acid or a salt thereof). When X of general formula (I) is hydrogen atom, it can form a colorant by condensation with a p-nitrosoaniline in the presence of an alkali or acetic anhydride. The cyan dye thus formed is usable widely as, for example, dye for filter, paint, ink, image-formation information recording and printing.

When the coupler is added to the silver halide emulsion, a hydroquinone derivative, U.V. absorber or known fading inhibitor can be used, if necessary, for controlling or improving the color developability.

The above-described high-boiling solvents are those having a melting point of 100°C or less (preferably 80°C or less) and a boiling point of 140°C or higher (preferably 160°C or higher) and in which the coupler is soluble. They include, for example, phosphoric esters (such as tricresyl phosphate, trioctyl phosphate and tricyclohexyl phosphate), organic acid esters (such as dibutyl phthalate, dioctyl phthalate, dicyclohexyl phthalate, dodecyl benzoate and bis(2-ethylhexyl) sebacate), ethers (including epoxy compounds), amides and amines. They further include cyclic compounds. In addition, high-boiling organic solvents used in an oil-in-water dispersion method can also be used.

Now the description will be made on the silver halide photosensitive materials for color photographs.

The cyan dye-forming coupler of above formula (I) is contained in at least one layer of the silver halide photosensitive material for color photographs of the present invention.

The silver halide photosensitive material for color photographs of the present invention can be a multi-layer, multi-color photographic material comprising at least two layers having different spectral sensitivities formed on a support. Multi-layer, natural color photographic materials comprise at least one red-sensitive emulsion layer, at least one green-sensitive emulsion layer and at least one blue-sensitive emulsion layer formed on a

support. The arrangement of the layers may be in any order selected suitably. A preferred arrangement of the layers is a structure of a red-sensitive layer, a green-sensitive layer and a blue-sensitive layer in order from the support; blue-sensitive layer, green-sensitive layer and red-sensitive layer in order from the support; or blue-sensitive layer, red-sensitive layer and green-sensitive layer in order from the support. Any color-sensitive emulsion layer may comprise two or more sub-emulsion layers sensitive to the same color but having different sensitivity in order to improve the sensitivity. Further, an emulsion layer may comprise three sub-layers in order to improve the graininess. A non-photosensitive layer may be placed between or among two or more emulsion layers sensitive to the same color. In another structure, an emulsion layer sensitive to a different color may be placed between two emulsion layers sensitive to the same color. A reflective layer comprising, for example, fine silver halide grains may be placed below a high-sensitivity layer, particularly high blue-sensitive layer to improve the sensitivity. A yellow filter layer may also be formed.

Usually the red-sensitive emulsion layer contains a cyan-forming coupler, the green-sensitive emulsion layer contains a magenta-forming coupler and the blue-sensitive emulsion layer contains a yellow forming coupler. A cyan coupler of above general formula (I) can be used as the whole cyan-forming coupler or as a part thereof. If necessary, other combinations are also possible. For example, an infrared ray-sensitive layer can be used for the exposure of pseudo color prints or semiconductor laser. In a typical example, the cyan coupler of the present invention is incorporated as the main coupler in the red-sensitive emulsion layer or a layer adjacent thereto. Further the cyan coupler of the present invention can be incorporated as the main coupler in the blue-sensitive, green-sensitive or red-sensitive emulsion layer. In addition, it is usable in combination with another main coupler.

The standard amount of the cyan coupler of the present invention used is in the range of 0.001 to 1 mol, preferably 0.003 to 0.3 mol, per mol of the photosensitive silver halide.

The cyan coupler of the present invention is usable also in combination with the magenta coupler or yellow coupler in an emulsion layer sensitive to a color different from the color to which the cyan-coupler-containing emulsion layer is sensitive. Another known cyan coupler can also be incorporated in the same layer or another layer so far as the effect of the present invention is not impaired. The cyan coupler, magenta coupler and yellow coupler usable in combination with the coupler of the present invention include those of general formulae (I) through (V) shown on pages 4 and 5 of EP-A-0231832. In particular, the cyan couplers are described on pages 12 to 24, magenta couplers are described on pages 25 to 43 and yellow couplers are described on pages 44 to 64 of that specification.

The color photosensitive material of the present invention is usable in various fields. For example, it is usable as a material for ordinary or movie negative color films, and reversal color films for slides and televisions. It is usable also as a material for color papers, positive color films and reversal color papers. The photosensitive material of the present invention is usable also as a black-and-white photosensitive material according to a technique of mixing three color couplers as described in Research Disclosure 17123 (July, 1978).

The silver halide emulsions used for forming the photosensitive material of the present invention are not particularly limited and those known in the art can be used. For example, the silver halide emulsions described in JP-A- 61-198236 (US-A- 4,707,436) are usable. In particular, silver halides described from line 10, upper right column, to line 7, lower left column, page 5 of the Patent Gazette; silver halide emulsions described from line 8, lower left column, page 5 to line 3, lower right column, page 7; couplers described from line 4, lower right column, page 7 to line 2, lower left column, page 8; and additives such as color antifoggant described from line 3, lower left column, page 8 to line 11, upper left column, page 9 are usable.

In particular, when the silver halide photosensitive material of the present invention is used for a direct positive color film or a direct positive color paper, direct positive silver iodide of internal latent image type is preferably used as silver halide. Further, when the silver halide photosensitive material of the present invention is used for a reversal color film, silver bromoiodide (content of silver iodide is preferably from 2 to 15 mol.%) is preferably used as silver halide. Moreover, when the silver halide photosensitive material of the present invention is used for a color paper or a photosensitive material for color printing, a silver halide emulsion containing silver chloride or silver chlorobromide which substantially does not contain silver iodide is preferably used. The silver iodide content of the above silver chlorobromide substantially not containing silver iodide is 1 mol.% or less, preferably 0.2 mol.% or less. Although halogen composition of the emulsion may be identical or different between grains, an emulsion having an identical halogen composition between grains easily makes properties of grains uniform. As for halogen distribution in grains contained in a silver halide emulsion, suitable grains can be selected from the following group: so-called uniform structure grains with homogeneous composition; so-called core/shell structure grains of which halogen composition is different between core part or internal part of a silver halide grain and shell part (consisting of one or more layers) surrounding the core part; and grains having a non-lamellar part(s) with different halogen composition in an internal part of the grains or on the surface of the grains (when the grains have the non-lamellar part on the surface, such a part is conjugated at the edge or the corner, or on the plain surface). The two latter grains are advantageous rather than the former one ( the uniform structure grains) for high sensitivity, and they are also preferred from the view point of the pressure resistance. When the silver halide grains have the above-mentioned structures, the boundary between two different halogen compositions may be clear, or unclear boundary may be formed by mixture of crystals with different compositions, or the composition of the boundary may be continuously changed by intention.

The silver bromide/silver chloride ratio of the silver chlorobromide emulsion can be optionally selected. This ratio can be changed widely according to the purpose and the silver chloride content is preferably 2% or more.

So-called high silver chloride content emulsion with high silver chloride concentration is preferably used for photosensitive materials suitable to a rapid processing. The silver chloride content thereof is preferably 90 mol.% or more, more preferably 95 mol.% or more.

In the high silver chloride content emulsion, silver bromide localizing layer is preferably located in an interal part of a silver halide grain and/or on the surface thereof in the state of lamellar or non-lamellar. As for halogen composition of the localizing layer, silver bromide content is preferably at least 10 mol.%, more preferably more that 20 mol.%. The localizing layer may be present in the grain core, at the edge or corner of the grain surface, or on the grain surface and is preferably be conjugated by epitaxial growth on the corner of the grain.

The uniform structure grains of which halogen distribution in grains is small is preferably used for a high silver chloride emulsion with 90 mol.% or make silver chloride content for the purpose of inhibiting reduction of sensitivity when a photosensitive material is pressed.

It is advantageous to increase further the silver chloride content in the silver halide emulsion for the purpose of reducing replenisher for development. In this case, a pure silver chloride emulsion containing 98 to 99.9 mol.% of silver chloride is also used preferably.

Mean particle size of silver halide grains contained in the silver halide emulsion of the present invention, which is an average member of the particle size calculated in terms of diameter of circle obtained from projected area of particles, is preferably from 0.1μm to 2 μm.

As for particle size distribution of the grains, so-called monodispersed emulsion with degree of variability of 20% or less, preferably 15% or less (the degree is obtained by division of standard deviation of particle size by average particle size) is preferred. In this case, the above-mentioned monodispersed emulsions may be blended and contained in an emulsion layer, or the emulsions may be contained in two or more emulsion layers in order to obtain wide latitude.

Silver halide grains in the photographic emulsion may be so-called regular grains which have a regular crystal shape such as cubic, octahedron or tetradecahedron, grains having an irregular crystal shape such as sphere or tabula, or grains having a composite shape thereof. Further silver halide grains may be a mixture of grains with various crystal shape. In the present invention, an emulsion containing 50% or more, preferably 70% or more, more preferably 90% or more of the grains having a regular crystal shape.

An emulsion containing more than 50% (on the basis of projected area of the whole grains) of flat grains which have an aspect ratio (diameter calculated in term of circle/thickness) of 5 or more, preferably 8 or more, is also used preferably.

Silver halide emulsion used for the present invention may contain various polyvalent metal ion inpurities during process for forming emulsion grains or physical ripening. Examples of compounds used include cadmium, zinc, lead, copper and thallium salts, and salts or complex salts of VIII group elements such as iron, ruthenium, rhodium, palladium, osmium, iridium and platinum. Particularly, elements belonging to VIII group of the periodic table are preferably used. An additive amount of the above salts may vary widely according to the purpose and preferably ranges from $10^{-9}$ to $10^{-2}$ mol.% on the basis of silver halide.

The silver halide emulsion used in the present invention may be chemically or spectrally sensitized.

In the case of chemical sensitization, sulfur sensitization typically using unstable sulfur compounds, noble metal sensitization such as gold sensitization, or reduction sensitization or combinations thereof may be used.

Compounds described in JP-A- 62-215272 (p.18, right-lower column to p.22, right-upper column) are preferably used.

The coupler of formula (I) of the present invention can be introduced in the photosensitive material by various known dispersion methods such as oil-in-water dispersion method. High-boiling solvents usable in this dispersion method are described in, for example, US-A- 2,322,027.

The high-boiling organic solvents having a boiling point under atmospheric pressure of at least 175°C used in the oil-in-water dispersion method include, for example, phthalic esters (such as dibutyl phthalate, dicyclohexyl phthalate, di-2-ethylhexyl phthalate, decyl phthalate, bis(2,4-di-t-amylphenyl) phthalate, bis(2,4-di-t-amylphenyl) isophthalate and bis(1,1-diethylpropyl) phthalate), phosphoric and phosphonic esters (such as triphenyl phosphate, tricresyl phosphate, 2-ethylhexyldiphenyl phosphate, tricyclohexyl phosphate, tri-2-ethylhexyl phosphate, tridodecyl phosphate, tributoxyethyl phosphate, trichloropropyl phosphate and di-2-ethylhexylphenyl phosphonate), benzoic esters (such as 2-ethylhexyl benzoate, dodecyl benzoate and 2-ethylhexyl-p-hydroxybenzoate), amides (such as N,N-diethyldodecaneamide, N,N-diethyllaurylamide and N-tetradecylpyrrolidone), alcohols and phenols (such as isostearyl alcohol and 2,4-di-tert-amylphenol), aliphatic carboxylic esters (such as bis(2-ethylhexyl) sebacate, dioctyl azelate, glycerol tributyrate, isostearyl lactate and trioctyl citrate), aniline derivatives (such as N,N-dibutyl-2-butoxy-5-tert-octylaniline), and hydrocarbons (such as paraffin, dodecylbenzene and diisopropylnaphthalene). Assistant solvents usable herein include, for example, organic solvents having a boiling point of at least about 30°C, preferably in the range of 50°C to about 160°C. Typical examples of them include ethyl acetate, butyl acetate, ethyl propionate, methyl ethyl ketone, cyclohexanone, 2-ethoxyethyl acetate and dimethylformamide.

When the coupler of the formula (I) of the present invention is to be dispersed by the oil-in-water dispersion method, the high-boiling organic solvent used therein is preferably at least one of the compounds of following formulae (VI) and (VII) and particularly further with an assistant solvent (such as ethyl acetate). The weight ratio of the high-boiling organic solvent to the coupler is preferably 0.6 or less and more preferably 0.4 or less. Namely, the assistant solvent can be used alone for the dispersion to obtain excellent results.

Formula (VI):

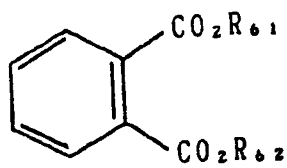

wherein $R_{61}$ and $R_{62}$ may be the same or different and they each represent an alkyl group, cycloalkyl group, alkenyl group or aryl group, with the proviso that the number of the total carbon atoms in $R_{61}$ and $R_{62}$ groups is 4 to 30.

Formula (VII):

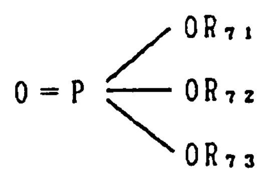

wherein $R_{71}$, $R_{72}$ and $R_{73}$ may be the same or different and they each represent an alkyl group, cycloalkyl group, alkenyl group or aryl group, with the proviso that the number of the total carbon atoms in $R_{71}$, $R_{72}$ and $R_{73}$ is 12 to 60.

The steps of the latex dispersion method, the effects thereof and the kinds of the latexes usable for the impregnation are described in, for example, US-A- 4,199,363, and DE-A- 2,541,274 and 2,541,230.

The suitable supports usable in the present invention are described on page 28 of above-described RD. No. 17643, and from the right column, page 647 to the left column, page 648 of RD. No. 18716.

The color photographic material according to the present invention can be developed by an ordinary method described on pages 28 to 29 of above-described RD. No. 17643 and from the left column to the right column, page 651 of RD. No. 18716.

The silver halide photosensitive material of the present invention for color photographs is usually subjected to a desilverization treatment, washed with water and/or subjected to a stabilization step. The amount of water used in the washing step is variable over a wide range depending on the characters of the photosensitive material (due to couplers, etc. used), the use, temperature of water, number of washing tanks (number of stages), feeding system (either countercurrent or down-flor system) and various other conditions. Among them, the relationship between the number of the tanks for washing water and the amount of water in the multi-stage countercurrent system can be determined by a method disclosed in 'Journal of the Society of Motion Picture and Television Engineers' vol. 64, page 248 to 253 (May, 1955).

Although the amount of water used for the washing can be remarkably reduced by the multi-step countercurrent method described in the above-mentioned literature, a problem is posed that bacteria propagate because of a prolonged residence time of water in the tanks and, therefore, the suspended matter thus formed contaminates the photosensitive material. In the treatment of the color photosensitive material of the present invention, this problem can be solved by a method described in JP-A- 62-288838 wherein the amount of calcium ion and magnesium ion is reduced. Further germicides can be used. The germicides usable herein include, for example, isothiazolone compounds and thiabendazoles described in JP-A- 57-8542, chlorine-containing germicides such as chlorinated sodium isocyanurate, and germicides described in Hiroshi Horiguchi 'Bokin Bokabizai no Kagaku', 'Biseibutsu no Mekkin, Sakkin, Bokabi Gijutsu' edited by Eisei Gijutsu-kai and 'Bokin Bokabizai Jiten' edited by Nippon Bokin Bokabi Gakkai such as benzotriazole.

pH of water used for the washing in the treatment of the photosensitive material of the present invention is 4 to 9, preferably 5 to 8. The temperature of water and the washing time are variable depending on the characters of the photosensitive material and the use thereof. Usually, the temperature and the time are 15 to 45°C and 20 sec to 10 min, preferably 25 to 40°C and 30 sec to 5 min, respectively. Further the photosensitive material of the present invention can be treated directly with a stabilizer in place of washing with water. In the stabilization treatment, any of known methods described in JP-A- 57-8,543 58-14,834 and 60-200,345 can be employed.

If necessary, the photosensitive material is stabilized after the washing with water. The stabilization bath usable herein is, for example, a bath containing formalin and a surfactant usually used as the final bath for photographic color sensitive materials. The stabilization bath may contain also chelating agents and antifungal agents.

The overflow due to the washing with water and/or feeding of the stabilizer can be reused in other steps such as the desilverization step.

The silver halide color photosensitive material of the present invention may contain a color developing agent in order to facilitate or accelerate the treatment. The color developing agents to be contained therein are preferably in the form of precursors thereof. They include, for example, indoaniline compounds described in US-A- 3,342,597, Schiff base-type compounds described in US-A- 3,342,599 and Research Disclosure Nos. 14,850 and 15,159, aldole compounds described in Research Disclosure No. 13,924, metal salt complexes described in US-A- 3,719,492 and urethane compounds described in JP-A- 53-135,628.

The silver halide color photosensitive material of the present invention may contain a 1-phenyl-3-pyrazolidone compound in order to accelerate the color development, if necessary. Typical examples of them are described in JP-A- 56-64,339, 57-144,547 and 58-115,438.

The temperature of the liquids used for the treatments in the present invention is 10 to 50°C, usually 33 to 38°C. A higher temperature can also be employed in order to accelerate the treatments or to reduce the treatment time or, on the contrary, a lower temperature can be employed in order to improve the quality of the image or stability of the treating solution. Cobalt intensifiers or hydrogen peroxide intensifiers described in DE-A- 2,226,770 and US-A- 3,674,499 can be used for saving silder in the photosensitive material.

The silver halide photosensitive material of the present invention is usable also as a heat development-type photosensitive material described in US-A- 4,500,626, JP-A- 60-133,449, 59-218443 and 61-238056 and EP-A-210,660.

The following examples will further illustrate the present invention.

Example 1

2.0 g of Coupler (1) of the present invention was dissolved in 20 ml of ethyl acetate and 15 ml of ethanol. A solution of 3 g of sodium carbonate in 30 ml of water was added thereto. 2.4 g of 4-(ethyl-methanesulfonamido-ethylamino)-o-toluidine monohydrate sesquisulfate was added to the mixture under stirring. A solution of 2.7 g of ammonium persulfate in 16 ml of water was added dropwise thereto. The mixture was stirred at room temperature for 30 min. 100 ml of water was added to the mixture to divide it into layers. The ethyl acetate layer was washed with water three times and, dried over anhydrous sodium sulfate. The solvent was distilled off at ambient temperature under reduced pressure. The residue was dissolved in a small amount of chloroform immediately thereafter and the solution was passed through a silica gel column to take a fraction of cyan color. The solvent was distilled off at ambient temperature under reduced pressure to obtain a powdery navy blue dye.

For comparison, dyes were produced from Couplers (2) and (5) and Compartative Couplers (C-1) and (C-2) which will be shown below in the same manner as above.

The absorption spectrum of each dye solution is shown in Table 1. The spectra of Coupler (1) and Comparative Coupler (C-1) are shown in Fig. 1.

| Coupler used | Maximum absorption (nm) | Half band width (nm) | ε |
|---|---|---|---|
| Coupler (1) of the present invention | 620.8 | 80.4 | 58600 |
| Coupler (2) of the present invention | 620.6 | 80.3 | 58400 |
| Coupler (5) of the present invention | 620.1 | 81.2 | 54600 |
| Comparative Coupler (C-1) | 622.4 | 114.7 | 29400 |
| Comparative Coupler (C-2) | 621.0 | 116.2 | 20800 |

It is apparent from Fig. 1 and Table 1 that the absorption of the dye obtained from the coupler of the present invention was quite sharp and had a vivid hue and a high molar extinction coefficient (ε).

Comparative Coupler (C-1)

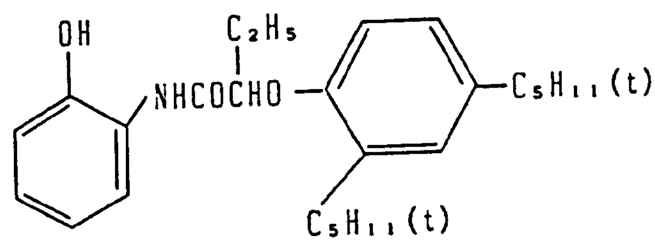

Comparative Coupler (C-2)

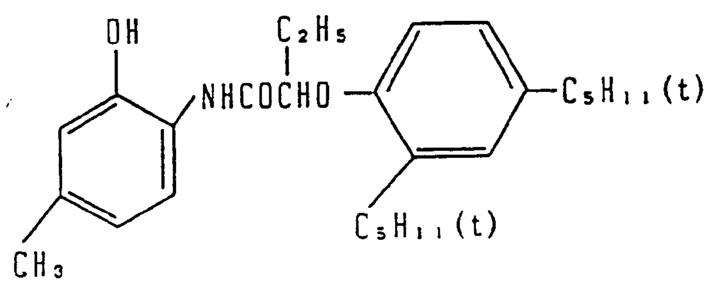

Example 2

A solution prepared by heating 10 g of Coupler (1) of the present invention, 10 g of dibutyl phthalate and 20 ml of ethyl acetate at 50°C was dispersed in 80 g of gelatin solution containing 8 ml of 1% aqueous sodium dodecylbenzenesulfonate solution to form an emulsion.

Then the emulsion was mixed with 145 g (7 g in terms of Ag) of a red-sensitive silver chlorobromide emulsion (Br content: 50%). Sodium dodecylbenzenesulfonate as the coating aid was added thereto and the mixture was applied to the surface of a paper support the both surface of which had been laminated with polyethylene films. The amount of the coupler to be coated was 400 mg/$m^2$. A protective gelatin layer (gelatin: 1 g/$m^2$) was formed thereon to form Sample A.

The same procedure as above was repeated except that Coupler (1) was replaced with an equimolar amount of Coupler (2), (3), (5), (16), (17) or (26) to form Samples B to G, respectively. For comparison, Samples H to L were prepared in the same manner as above except that Comparative Couplers (C-1), (C-2), (C-3), (C-4) and (C-5) were used, respectively.

The samples were exposed with a sensitometric continuous wedge and then subjected to the following developing treatment:

Color development step (33°C)

1. Color development (3 min 30 sec)
2. Bleach-fixing (1 min 30 sec)
3. Washing with water (2 min 30 sec)

The treating solutions used in the respective steps were as follows:

Color developer:

| | |
|---|---|
| benzyl alcohol | 15.0 ml |
| diethylene glycol | 8.0 ml |
| ethylenediaminetetraacetic acid | 5.0 g |
| sodium sulfite | 2.0 g |
| anhydrous potassium carbonate | 30 g |
| hydroxylamine sulfate | 3.0 g |
| potassium bromide | 0.6 g |
| 4-amino-N-ethyl-N-(ß-methanesulfonamidoethyl)-m-toluidine sesquisulfate monohydrate | 5.0 g |
| water | ad 1 ℓ (-H 10.2) |

Comparative Couplers:

(C - 3)

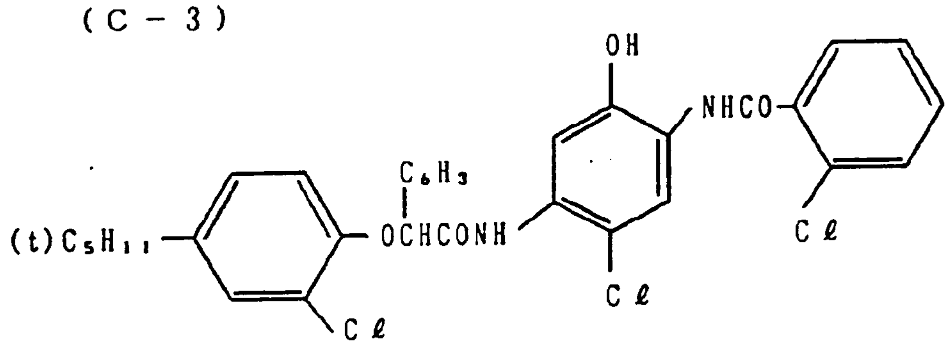

(C - 4)

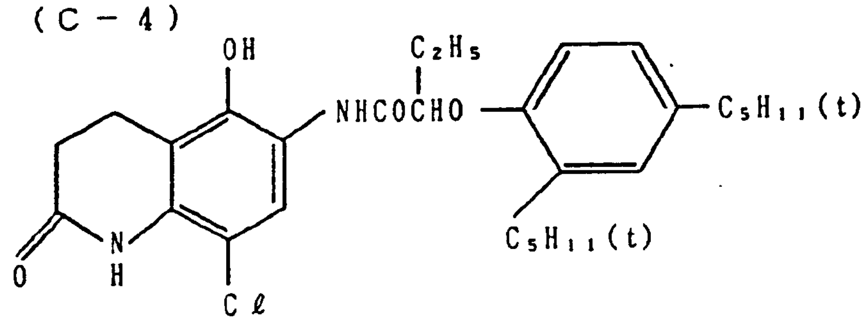

(C - 5)

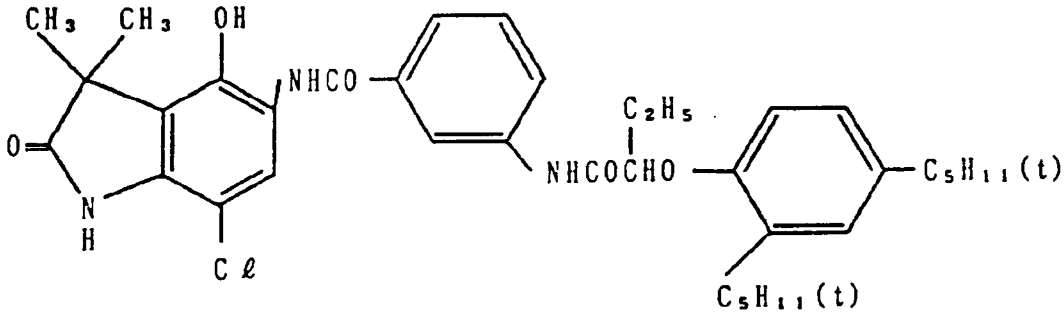

Bleach-fixing solution:

| | |
|---|---|
| ethylenediaminetetraacetic acid | 4.0 g |
| ferric ethylenediaminetetraacetate | 40 g |
| sodium sulfite | 5.0 g |
| (70%) sodium thiosulfate | 150 ml |
| water | ad 1 |

Samples treated with the couplers of the present invention had a vivid cyan or blue hue.

Then the fastness of the treated samples was examined. Dye densities kept after leaving the samples to stand at a dark place at 100°C for 10 days or after exposing them to a light with a xenon fadeometer (100,000 1x) are shown in Table 2.

Table 2

| Sample No. | Coupler | 100°C, 10 days | Xenon, 1 week | Remarks |
|---|---|---|---|---|
| A | 1 | 0.32 | 0.50 | present invention |
| B | 2 | 0.33 | 0.52 | " |
| C | 3 | 0.80 | 0.68 | " |
| D | 5 | 0.48 | 0.56 | " |
| E | 16 | 0.89 | 0.72 | " |
| F | 17 | 0.91 | 0.89 | " |
| G | 26 | 0.88 | 0.90 | " |
| H | C-1 | 0.38 | 0.48 | comparative example |
| I | C-2 | 0.40 | 0.78 | " |
| J | C-3 | 0.85 | 0.69 | " |
| K | C-4 | 0.88 | 0.86 | " |
| L | C-5 | 0.82 | 0.88 | " |

It is apparent from Table 2 that the couplers of the present invention had the fastness similar to that of corresponding couplers having a carbon ring (comparsion of A with B and H, D with I, E with J, F with K and G with L).

Example 3

A multilayer silver halide photosensitive material 101 having the following layer structure formed on a paper support the both surface of which had been laminated with polyethylene was prepared:

Sodium salts of 1-oxy-3,5-dichloro-s-triazine was used in each layers as a gelatin hardener.

The following compounds were used as spectral sensitizing dye:

Blue-sensitive emulsion layer

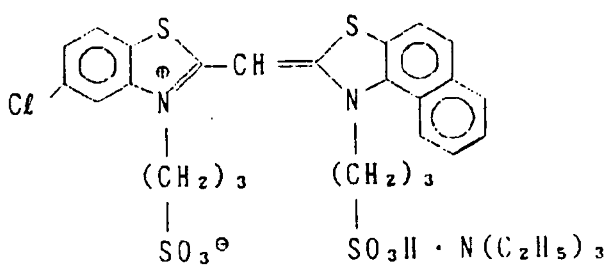

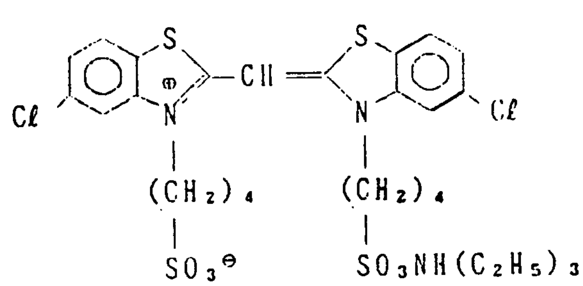

(Large size emulsion: 2.0 x $10^{-4}$ mole/l mole silver halide
Small size emulsion : 2.5 x $10^{-4}$ mole/l mole silver halide)

Green-sensitive emulsion layer

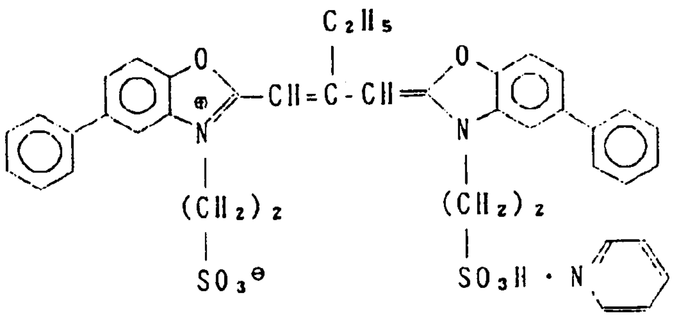

(Large size emulsion: 4.0 x $10^{-4}$ mole/l mole silver halide
Small size emulsion : 5.6 x $10^{-4}$ mole/l mole silver halide) and

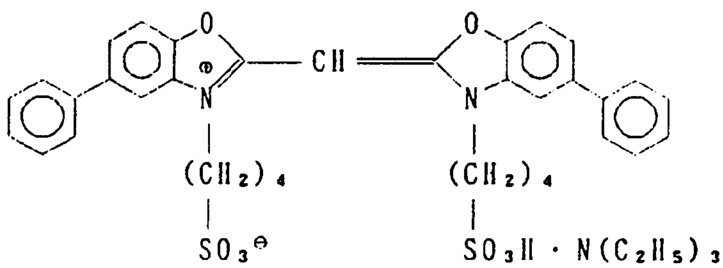

(Large size emulsion: 7.0 x $10^{-5}$ mole/l mole silver halide
Small size emulsion : 1.0 x $10^{-5}$ mole/l mole silver halide)

Red-sensitive emulsion layer

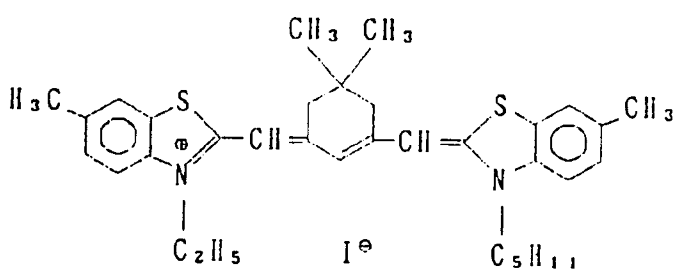

(Large size emulsion: 0.9 x $10^{-4}$ mole/l mole silver halide
Small size emulsion : 1.1 x $10^{-4}$ mole/l mole silver halide)

2.6 x $10^{-3}$ mole/1 mole silver halide of the following compound was added to the red-sensitive emulsion layer.

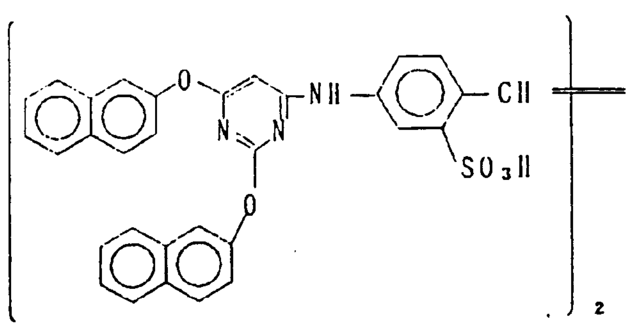

$8.5 \times 10^{-5}$, $7.7 \times 10^{-4}$ and $2.5 \times 10^{-4}$ (mole/l mole silver halide) of 1-(5-methylureidophenyl)-5-mercaptotetrazale were added to the blue-sensitive, green-sensitive and red-sensitive emulsion layers, respectinely.

The following dyes were added to emulsion layers in order for inhibition of irradiation.

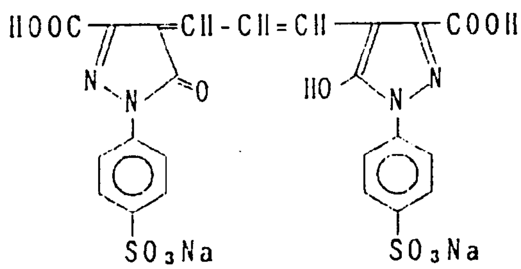

and

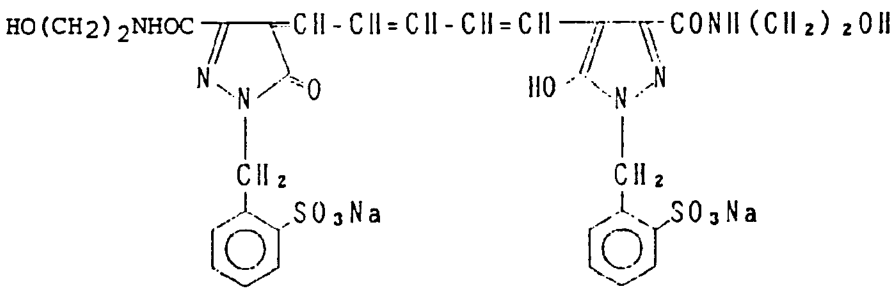

(layer structure)

The compositions of the respective layers will be shown below. The numerals stands for the applied amount ($g/m^2$). The amount of the silver halide emulsion is given in terms of silver.

Support:

paper laminated with polyethylene (The polyethylene layer adjacent to the first layer contained a white pigment ($TiO_2$) and a blue dye:)

| | |
|---|---|
| The first layer (blue-sensitive layer) | |
| silver chlorobromide emulsion | 0.30 |
| gelatin | 1.86 |
| yellow coupler (ExY) | 0.82 |
| color image stabilizer (Cpd-1) | 0.19 |
| solvent (Solv-3) | 0.35 |
| color image stabilizer (Cpd-7) | 0.06 |
| The second layer (color mixing-inhibiting layer) | |
| gelatin | 0.99 |
| color mixing inhibitor (Cpd-5) | 0.08 |
| solvent (Solv-1) | 0.16 |
| solvent (Solv-4) | 0.08 |
| The third layer (green-sensitive layer) | |

silver chlorobromide emulsion (1:3 (Ag molar ratio) mixture of cubic grains with 0.55 μm and 0.39 μm of an average praticle size. Degrees of variability of particle size distribution

were 0.10 and 0.08. 0.8 mol.% of AgBr localized on the grain surface.)

| | |
|---|---|
| | 0.12 |
| gelatin | 1.24 |
| magenta coupler (ExM) | 0.20 |
| color image stabilizer (Cpd-3) | 0.15 |
| color image stabilizer (Cpd-4) | 0.02 |
| color image stabilizer (Cpd-2) | 0.03 |
| solvent (Solv-2) | 0.40 |

The fourth layer (U. V. absorption layer)

| | |
|---|---|
| gelatin | 1.58 |
| U. V. absorber (UV-1) | 0.47 |
| color mixing inhibitor (Cpd-5) | 0.05 |
| solvent (Solv-5) | 0.24 |

The fifth layer (red-sensitive layer)

silver chlorobromide emulsion

(1:4 (Ag molar ratio) mixture of cubic grains with 0.58 μm and 0.45 μm of an average particle size. Degrees of variability of particle size distribution were 0.09 and 0.11. 0.6 mol.% of AgBr localized on a part of the grain surface.)

| | |
|---|---|
| | 0.23 |
| gelatin | 1.34 |
| cyan coupler (C-1) | 0.15 |
| cyan coupler (C-2) | 0.18 |
| color image stabilizer (Cpd-6) | 0.02 |
| color image stabilizer (Cpd-8) | 0.04 |
| color image stabilizer (Cpd-7) | 0.40 |
| solvent (Solv-6) | 0.15 |

The sixth layer (U.V. absorption layer)

| | |
|---|---|
| gelatin | 0.53 |

| | |
|---|---|
| U. V. absorber (UV-1) | 0.16 |
| color mixing inhibitor (Cpd-5) | 0.02 |
| solvent (Solv-5) | 0.08 |

The seventh layer (protective layer)

| | |
|---|---|
| acid-treated gelatin | 1.33 |
| acrylic-modified polyvinyl alcohol copolymer (degree of modofication: 17%) | 0.17 |
| liquid paraffin | 0.03 |

(ExY) Yellow coupler

1:1 (molar ratio) mixture of

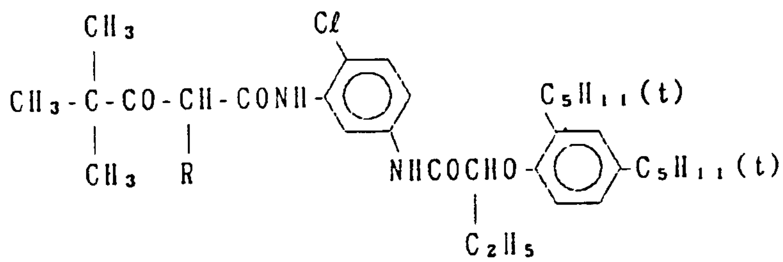

and

R = 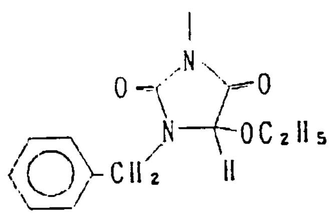

R = 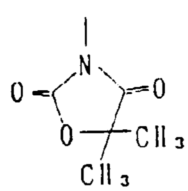

(ExM) Magenta coupler

1:1 (molar ratio) mixture of

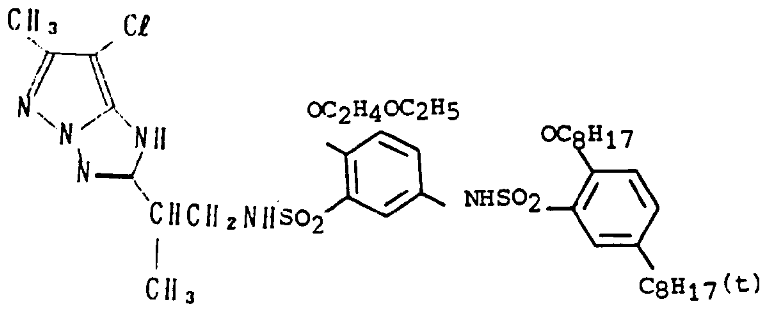

and

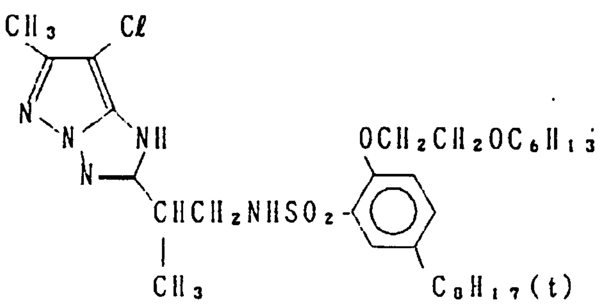

(Cpd-1) color image stabilizer

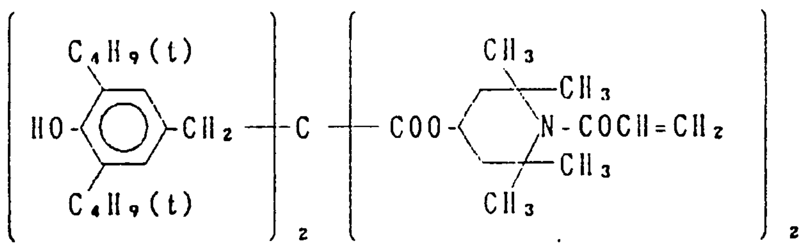

(Cpd-3) color image stabilizer

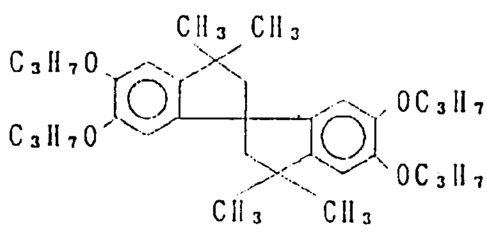

(Cpd-5) color mixing inhibitor

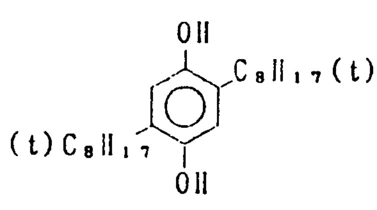

(Cpd-4) color image stabilizer

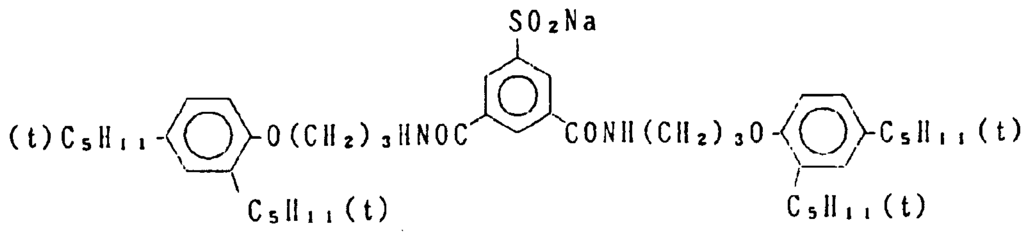

(Cpd-2) color image stabilizer

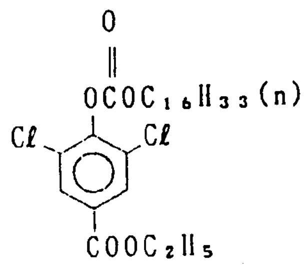

(Cpd-6) color image stabilizer

2:4:4 (weight ratio) mixture of

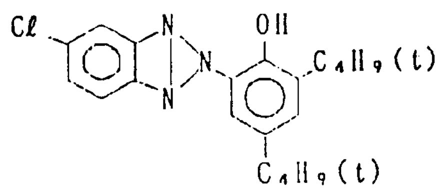

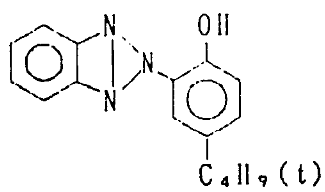

and

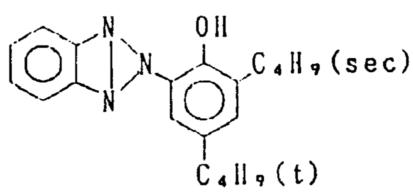

(Cpd-7) color image stabilizer

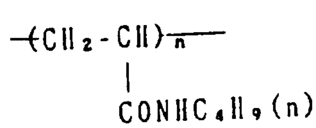

Average molecular weight 60,000

(Cpd-8) color image stabilizer

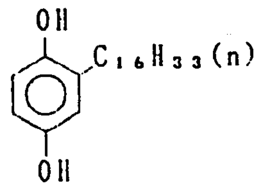

(UV-1) U. V. absorber

4:2:4 (weight ratio) mixture of

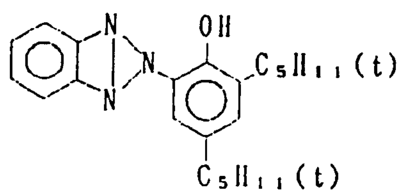

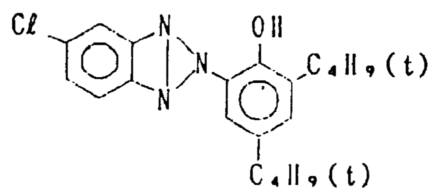

and

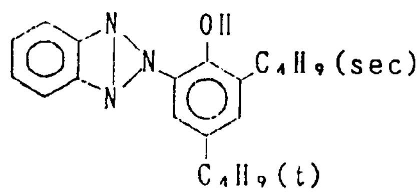

(Solv-1) Solvent

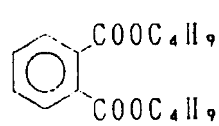

(Solv-2) Solvent

2:1 (volume ratio) mixture

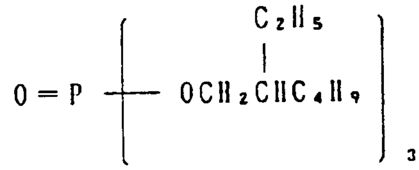

and

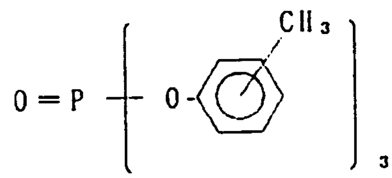

(Solv-3) Solvent

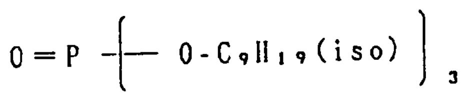

(Solv-4) Solvent

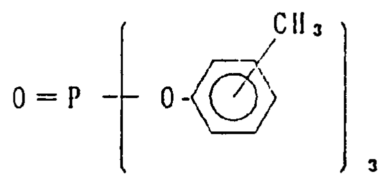

(Solv-5) Solvent

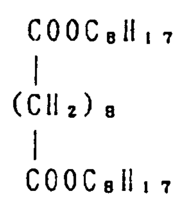

(Solv-6) Solvent

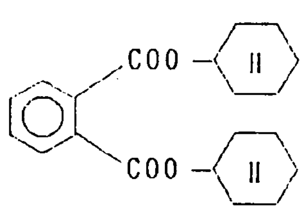

Samples 102 to 108

Samples were prepared in the same manner as that of the preparation of Sample 101 except that Couplers (C-1) and (C-2) in the fifth layer of Sample 101 (Comparative) were replaced with an equimoplar amount of (1), (5), (6), (9), (16), (17) or (26). They will be referred to as Samples 102 to 108, respectively, (present invention).

The photosensitive materials were exposed through an optical wedge and then treated in the following steps:

| Treatment steps | Temperature | Time | Replenisher* | Tank volume |
|---|---|---|---|---|
| Color development | 35°C | 45 sec | 161 ml | 17 ℓ |
| Bleach-fixing | 30 to 35°C | 45 sec | 215 ml | 17 ℓ |
| Rinse (1) | 30 to 35°C | 20 sec | - | 10 ℓ |
| Rinse (2) | 30 to 35°C | 20 sec | - | 10 ℓ |
| Rinse (3) | 30 to 35°C | 20 sec | 350 ml | 10 ℓ |
| Drying | 70 to 80°C | 60 sec | | |

* Amount of replenisher are expressed on the basis of $1m^3$ of photosensitive material.

(The countercurrent system was employed in the three tanks in the rinse steps (1) to (3).)

The compositions of the treating solutions were as follows:

| Color developer | Tank liquid | Replenisher |
|---|---|---|
| water | 800 ml | 800 ml |
| ethylenediamine-N,N,N,N-tetramethylenephosphoric acid | 1.5 g | 2.0 g |
| triethanolamine | 8.0 g | 12.0 g |
| sodium chloride | 1.4 g | - |
| potassium carbonate | 25 g | 25 g |
| N-ethyl-N-(β-methane-sulfonamidoethyl)-3-methyl-4-aminoaniline sulfate | 5.0 g | 7.0 g |
| N,N-bis(carboxymethyl)hydrazine | 5.5 g | 7.0 g |
| fluorescent brightening agent (WHITEX 4B manufactured by Sumitomo Chemical) | 2.0 g | |
| water | ad 1,000 ml | |
| pH (25°C) | 10.05 | 10.45 |

<u>Bleach-fixing solution</u> (Tank liquid has the same compostion as that of replenisher)

| | |
|---|---|
| water | 400 ml |
| ammonium thiosulfate (70%) | 100 ml |
| sodium sulfite | 17 g |
| iron (III) ammonium ethylene-diaminetetraacetate | 55 g |
| disodium ethylene-diaminetetraacetate | 5 g |
| ammonium bromide | 40 g |
| water | <u>ad</u> 1,000 ml |
| pH (25°C) | 6.0 |

<u>Rinse solution</u>

ion-exchanged water (Ca, Mg: ppm or less) Samples 102 to 108 prepared by using the couplers of the present invention had a color reproducibility superior to that of Comparative Sample 101.

Example 4

The first layer to the fourteenth layer were coated on the front side of a paper support (thickness: 100μ ) (polyethylene has been laminated on the both surfaces of the support) and the fifteenth layer and the sixteenth layer were coated on the back side of the paper support to form a color photographic photosensitive material sample 201. The polyethylene layer laminated on the front side contained titanium dioxide (4 g/m$^2$) as a white pigment and a small amount of ultramarine (0.003 g/m$^2$) as a bluing dye. (Chromaticity (L*, a*, b*) on the front side of the laminated paper support: 88.0, -0.20. -0.75)

Photosensitive layer composition)

The compositions and the applied amount (g/m$^2$) of respective layers will be shown below. Emulsions utilized to each layer were prepared on the basis of the method for preparation of emulsion EM1 expect that a lippmann emulsion which had not been subjected to surface chemical sensitization was used as an emulsion of the fourteenth layer. The first layer (anti-halation layer)

| | |
|---|---|
| black colloidal silver | 0.10 |
| gelatin | 0.70 |

The second layer (interlayer)

| | |
|---|---|
| gelatin | 0.70 |

The third layer (low speed red-sensitive layer)

| | |
|---|---|
| silver bromide (mean particle size: 0.25μ, size distribution (degree of variability) 8%, octahedron) spectrally sensitized with red sensitizing dyes (ExS-1,2,3) | 0.04 |
| silver chlorobromide (silver chloride content: 5 mol.%, mean particle size: 0.40μ, size distribution: 10%, octahedron) spectrally sensitized with red sensitizing dyes (ExS-1,2,3) | 0.08 |
| gelatin | 1.00 |
| cyan coupler (ExC-1:ExC-2:ExC-3=1:1:0.2) | 0.30 |
| decoloration inhibitor (Cpd-1:Cpd-2:Cpd-3:Cpd4=1:1:1:1) | 0.18 |
| stain inhibitor (Cpd-5) | 0.003 |
| coupler dispersing medium (Cpd-6) | 0.03 |
| solvent for coupler (Solv-1:Solv-2:Solv-3=1:1:1) | 0.12 |

The fourth layer (high speed red-sensitive layer)

| | |
|---|---|
| silver bromide (mean particle size: 0.60μ, size distribution: 15%, octahedron) spectrally sensitized with red sensitizing dyes (ExS-1,2,3) | 0.14 |
| gelatin | 1.00 |
| cyan coupler (ExC-1:ExC-2:ExC-3=1:1:0.2) | 0.30 |
| decoloration inhibitor (Cpd-1:Cpd-2:Cpd-3:Cpd4=1:1:1:1) | 0.18 |
| coupler dispersing medium (Cpd-6) | 0.03 |
| solvent for coupler (Solv-1:Solv-2:Solv-3=1:1:1) | 0.12 |

The fifth layer (interlayer)

| | |
|---|---|
| gelatin | 1.00 |
| color mixing inhibitor (Cpd-7) | 0.08 |
| solvent for color mixing inhibitor (Solv-4:Solv-5=1:1) | 0.16 |
| polymer latex (Cpd-8) | 0.10 |

The sixth layer (low speed green-sensitive layer)

| | |
|---|---|
| silver bromide (mean particle size: 0.25 μ, size distribution 8%, octahedron) spectrally sensitized with green sensitizing dye (ExS-4) | 0.04 |
| silver chlorobromide (silver chloride content: 5 mol.%, mean particle size: 0.04 μ, size distribution: 10%, octahedron) spectrally sensitized with green sensitizing dye (ExS-4) | 0.06 |
| gelatin | 0.80 |
| magenta coupler (ExM-1:ExM-2:ExM-3=1:1:1) | 0.11 |
| decoloration inhibitor (Cpd-9:Cpd-26=1:1) | 0.15 |
| stain inhibitor (Cpd-10:Cpd-11:Cpd-12:Cpd-13=10:7:7:1) | 0.025 |
| coupler dispersing medium (Cpd-6) | 0.05 |
| solvent for coupler (Solv-4:Solv-6=1:1) | 0.15 |

The seventh layer (high speed green-sensitive layer)

| | |
|---|---|
| silver bromide (mean particle size: 0.65 μ, size distribution: 6%, octahedron) spectrally sensitized with green sensitizing dye (ExS-4) | 0.10 |
| gelatin | 0.80 |

| | |
|---|---|
| magenta coupler (ExM-1:ExM-2:ExM-3=1:1:1) | 0.11 |
| decoloration inhibitor (Cpd-9:Cpd-26=1:1) | 0.15 |
| stain inhibitor (Cpd-10:Cpd-11:Cpd-12:Cpd-13=10:7:7:1) | 0.025 |
| coupler dispersing medium (Cpd-6) | 0.05 |
| solvent for coupler (Solv-4:Solv-6=1:1) | 0.15 |

The eighth layer (interlayer)

same as those of the fifth layer

The ninth layer (yellow filter layer)

| | |
|---|---|
| yellow colloidal silver (particle size: 100 Å) | 0.12 |
| gelatin | 0.70 |
| color mixing inhibitor (Cpd-7) | 0.03 |
| solvent for color mixing inhibitor (Solv-4:Solv-5=1:1) | 0.10 |
| polymer latex (Cpd-8) | 0.07 |

The tenth layer (interlayer)

same as those of the fifth layer

The eleventh layer (low speed blue-sensitive layer)

| | |
|---|---|
| silver bromide (mean particle size: 0.40 μ, size distribution 8%, octahedron) spectrally sensitized with blue sensitizing dye (ExS-5,6) | 0.07 |
| silver chlorobromide (silver chloride content: 8 mol.%, mean particle size: 0.60 μ, size distribution: 11%, octahedron) spectrally sensitized with blue sensitizing dye (ExS-5,6) | 0.14 |
| gelatin | 0.80 |
| yellow coupler (ExY-1:ExY-2=1:1) | 0.35 |
| decoloration inhibitor (Cpd-14) | 0.10 |

| | |
|---|---|
| stain inhibitor (Cpd-5:Cpd-15=1:5) | 0.007 |
| coupler dispersing medium (Cpd-6) | 0.05 |
| solvent for coupler (Solv-2) | 0.10 |

The twelfth layer (high speed blue-sensitive layer)

| | |
|---|---|
| silver bromide (mean particle size: 0.85 μ, size distribution 8%, octahedron) spectrally sensitized with blue sensitizing dyes (ExS-5,6) | 0.15 |
| gelatin | 0.60 |
| yellow coupler (ExY-1:ExY-2=1:1) | 0.30 |
| decoloration inhibitor (Cpd-14) | 0.10 |
| stain inhibitor (Cpd-5:Cpd-15=1:5) | 0.007 |
| coupler dispersing medium (Cpd-6) | 0.05 |
| solvent for coupler (Solv-2) | 0.10 |

The thirteenth layer (U.V. absorption layer)

| | |
|---|---|
| gelatin | 1.00 |
| U.V. absorber (Cpd-2:Cpd-4:Cpd-16=1:1:1) | 0.50 |
| color mixing inhibitor (Cpd-7:Cpd-17=1:1) | 0.03 |
| dispersing medium (Cpd-6) | 0.02 |
| solvent for U.V. absorber (Solv-2:Solv-7=1:1) | 0.08 |
| irradiation inhibiting dye (Cpd-18:Cpd-19:Cpd-20:Cpd-21:Cpd-27=10:10:13:15:20) | 0.05 |

The fourteenth layer (protective layer)

| | |
|---|---|
| silver chlorobromide fine particles (silver chloride: 97 mol.%, mean particle size: 0.1 μ) | 0.03 |
| acrylic-modified polyvinyl alcohol copolymer (molecular weight: 50,000) | 0.01 |
| polymethyl methacrylate particles (mean particle size: 2.4 μ): | |
| silica (mean particle size: 5 μ)=1:1 | 0.05 |
| gelatin | 1.80 |
| gelatin hardener (H-1:H-2=1:1) | 0.18 |

The fifteenth layer (back layer)

| | |
|---|---|
| gelatin | 2.50 |
| U.V. absorber (Cpd-2:Cpd-4:Cpd-16=1:1:1) | 0.50 |
| dye (Cpd-18:Cpd-19:Cpd-20:Cpd-21:Cpd-27 =1:1:1:1:1) | 0.06 |

The sixteenth layer (back side protective layer)

| | |
|---|---|
| polymethyl methacrylate particles (mean particle size: 2.4 μ): silica (mean particle size: 5 μ)=1:1 | 0.05 |
| gelatin | 2.00 |
| gelatin hardener (H-1:H-2=1:1) | 0.14 |

Preparation method of emulsion EM-1

An aqueous potassium bromide solution and an aqueous silver nitrate solution were simultaneously added to an aqueous gelatin solution at 75°C within 15 min. while the gelatin solution was stirred strongly to obtain an emulssion containing octahedron silver bromide particles (mean particle size: 0.35μ) (first step). In this case, 3,4-dimethyl-1,3-thiazolin-2-thione (3 g/l mol silver) were added to the solution. Then the resulting emulsion was subjected to a chemical sensitizing treatment by adding sodium thiosulfate (6 mg/l mol silver) and chloroauric acid (tetrahydrate) (7 mg/l mol silver) to the emulsion successively and heating at 75°C for 80 min. Silver bromide particles in the chemically sensitized emulsion were further grown under the same condition as those of the above first step to obtain an actahedron monodispersed core/shell type silver bromide emulsion (mean particle size: 0.7 μ). The degree of variability of the particle size was about 10%. Then sodium thiosulfate (1.5 mg/l mol silver) and chloroauric acid (tetrahydrate) (1.5 mg/l mol silver) were added to the obtained emulsion and the mixture was subjected to chemical sensitization by heating at 60°C for 60 min to obtain an internal latent image type silver halide emulsion.

$10^{-3}$% by weight (on the basis of the weight of silver halide) of ExZK-1 and $10^{-2}$% by weight of ExZK-2 were contained in each photosensitive layer as nucleus forming agents and $10^{-2}$% by weight of Cpd-22 was used as a nucleus formation accelerator. Further Alkanal XC (manufactured by Du pont) and sodium alkylbenzensulfonate as emulsion dispersing auxiliaries, and succinate and Magefac F120 (manufactured by Dainippon

ink) as coating auxiliaries were contained in each layer. Stabilizers (Cpd-23,24,25) were contained in layers containing silver halide and colloidal silver.

Compounds used in this example were shown below.

ExS-1

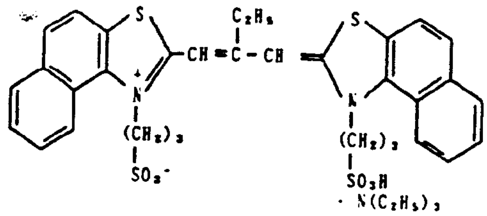

ExS-2

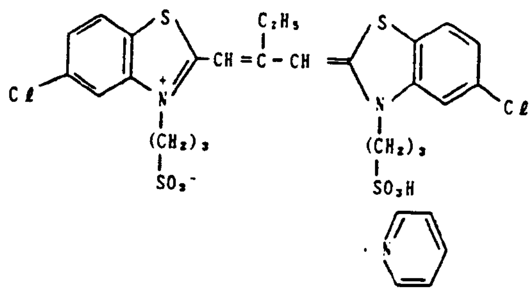

ExS-3

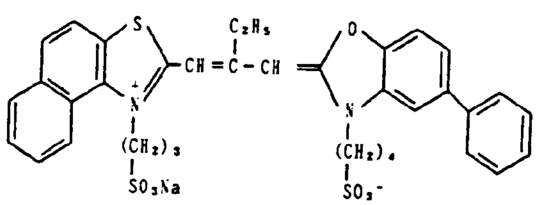

ExS-4

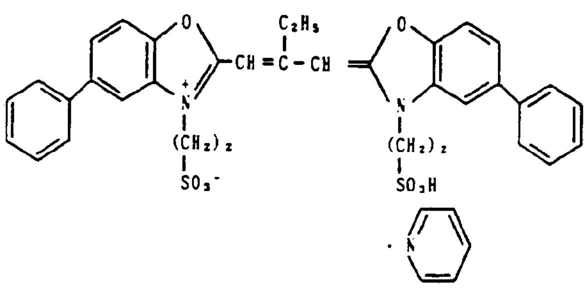

ExS-5

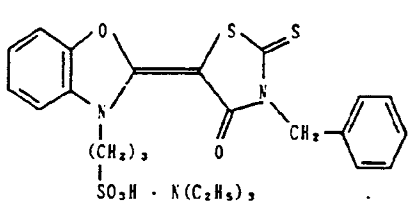

ExS-6

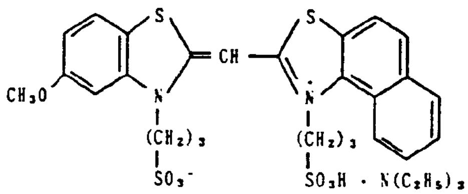

Cpd-1

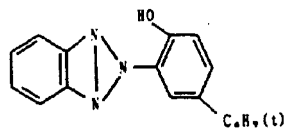

Cpd-2

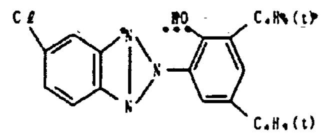

Cpd-3

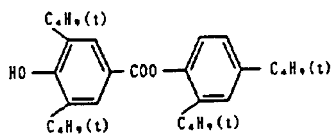

Cpd-4

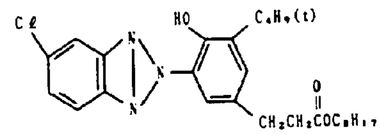

Cpd-5

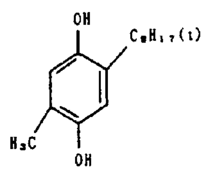

Cpd-6

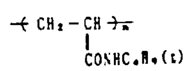

n=100~1000

Cpd-7

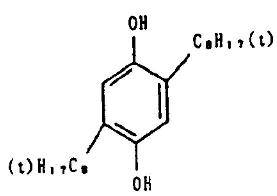

Cpd-8

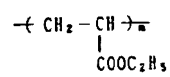

Cpd-9

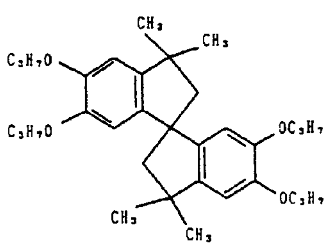

Cpd-10

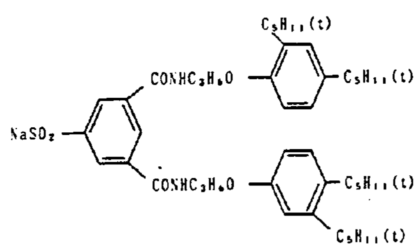

Cpd-11

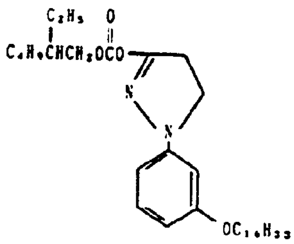

Cpd-12

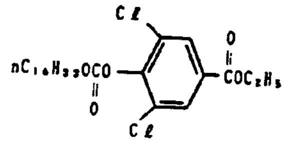

Cpd-13

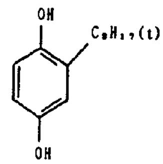

Cpd-14

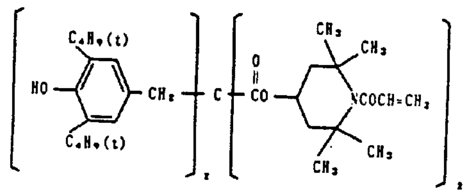

Cpd-15

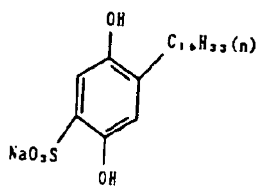

Cpd-16

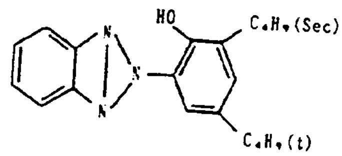

Cpd-17

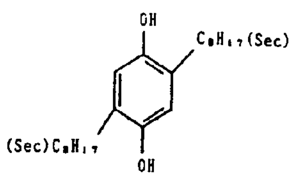

Cpd-18

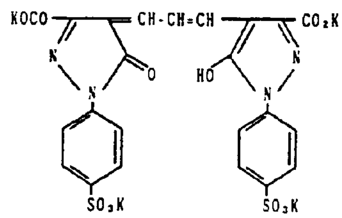

Cpd-19

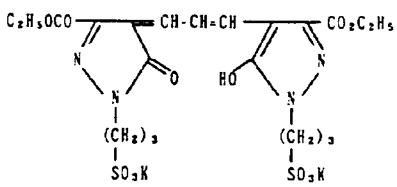

Cpd-20

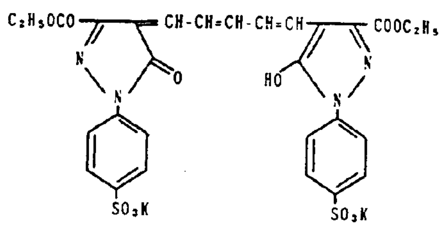

Cpd-21

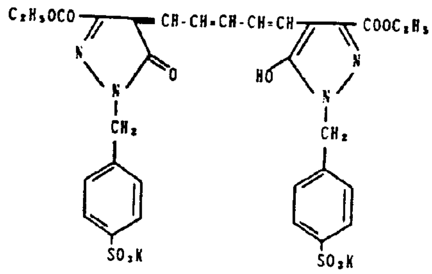

Cpd-22

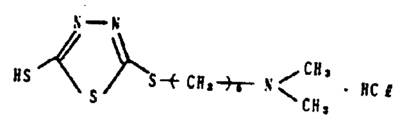

Cpd-23

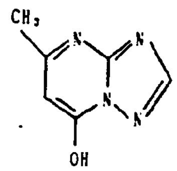

Cpd-24

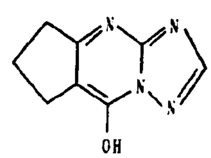

Cpd-25

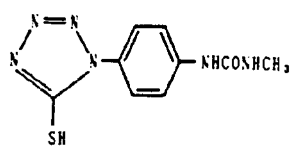

Cpd-26

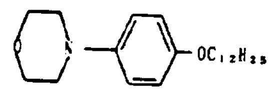

Cpd-27

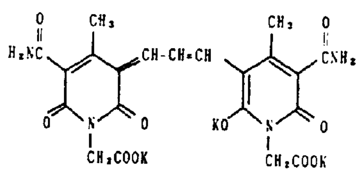

EXC-1

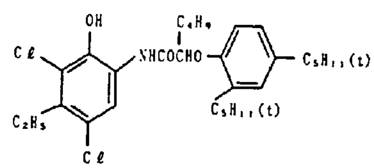

EXC-2

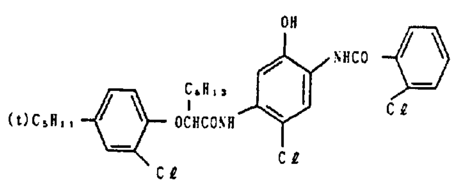

EXC-3

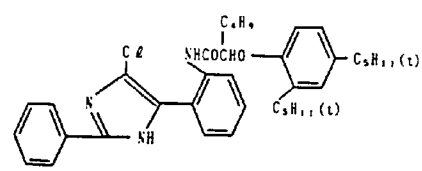

EXM-1

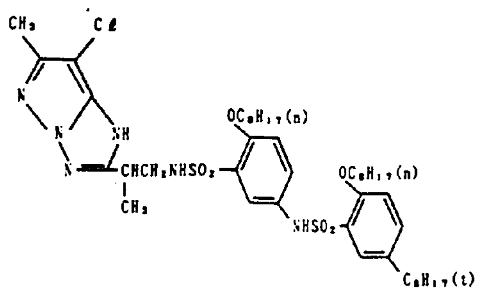

EXM-2

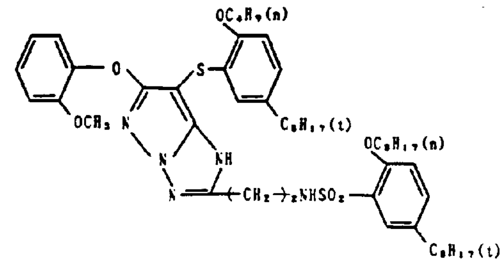

EXM-3

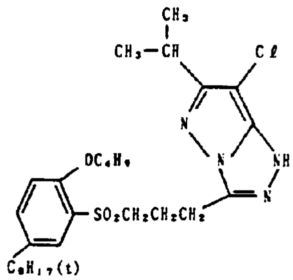

EXY-1

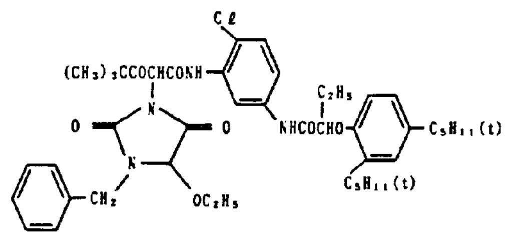

EXY-2

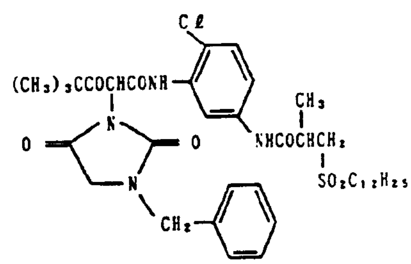

| | |
|---|---|
| Solv-1 | di(2-ethylhexyl) sebacate |
| Solv-2 | trinonyl phosphate |
| Solv-3 | di(3-methylhexyl) phthalate |
| Solv-4 | tricresylphosphate |
| Solv-5 | dibutylphthalate |
| Solv-6 | trioctylphthalate |
| Solv-7 | di(2-ethylhexyl)phthalate |
| H-1 | 1,2-bis(vinylsulfonylacetoamide)ethane |
| H-2 | sodium 4,6-dichloro-2-hydorxy-1,3,5-triazine |
| ExZK-1 | 7-(3-ethoxythiocarbonylaminobenzamido)-9-methyl-10-propargyl-1,2,3,4-tetrahydroacridinium trifluoromethanesulfonate |
| ExAK-2 | 2-[4-{3-[3-{3-[5-{3-[2-chlors-5-(1-dodecyloxycarbonylethoxycarbonyl) phenylcarbamoyl]-4-hydroxy-1-naphthylthio}tetrazol-1-yl]phenyl}ureido]benzensulfonamido}phenyl]-1-formylhydrazine |

A sample prepared as mentioned above was Sample 201.

A sample 202 was prepared according to the same procedures as those of Sample 201 except that an equimolar amount of cyan coupler (1) of the present invention was used in place of cyan couplers ExC-1,2 and 3 used in the third and fourth layers.

Samples 203 - 207 were respectively prepared in the same manner as that of the preparation of Sample

201 except that cyan couplers ExC-1, 2 and 3 were replaced with an equimolar amount of cyan couplers (5), (6), (9)(, (16) and (17).

The resulting Samples 201 - 207 were exposed through a red filter and then treated in the following steps.

The spectral absorption of the treated samples was measured and it was found that a spectral absorption property of the samples (202 -207) using the cyan couplers of the present invention is sharper than that of Sample 201.

| Treatment steps | Time | Temperature | Tank volume of mother liquid | Replenishing amount |
|---|---|---|---|---|
| Color development | 80 sec | 38°C | 8 ℓ | 300 ml/m$^2$ |
| Bleach-fixing | 40 sec | 33°C | 3 ℓ | 300 " |
| Washing (1) | 40 sec | 33°C | 3 ℓ | - |
| Washing (2) | 40 sec | 33°C | 3 ℓ | - |
| Washing (3) | 15 sec | 33°C | 0.5 ℓ | 320 ml/m$^2$ |
| Drying | 30 sec | 80°C | - | - |

(The countercurrent system was employed in the three tanks in the washing steps (1) to (3). Washing water was replenished to washing tank (3), washing water overflowed from washing tank (3) was introduced into washing tank (2), and washing water overflowed from washing tank (2) was introduced into wshing tank (1).)

The amount of bleach-fixing solution carried by a photosensitive material from a bleach-fixing tank to washing tank (1) was 35 ml/m$^2$ and the ratio of amount of the replenished washing water to amount of the carried bleach-fixing solution was 9.1.

The compositions of the treating solutions were as follows:

| Color developer | Mother liquid | Replenisher |
|---|---|---|
| ethylenediaminetetrakismethylene phosphonic acid | 0.5 g | 0.5 g |
| diethylene glycol | 10 ml | 10 ml |
| benzyl alcohol | 12.0 ml | 14.4 ml |
| potassium bromide | 0.52 g | - |
| sodium chloride | 0.06 g | - |
| sodium sulfite | 2.4 g | 2.9 g |
| N,N-diethylhydroxylamine | 4.0 g | 4.8 g |
| triethylenediamine(1,4-diaza-bicyclo [2,2,2] octane) | 4.0 g | 4.8 g |
| 3-methyl-4-amino-N-ethyl-N (ß-methanesulfonamidoethyl) aniline sulfate | 5.6 g | 6.6 g |
| potassium carbonate | 27.0 g | 25.0 g |
| fluorescent brightening agent agent (4,4'-diaminostilbene) | 1.0 g | 1.2 g |
| water | ad 1000 ml | 1000 ml |
| pH (25°C) | 10.50 | 10.80 |

| Bleach-fixing solution | Mother liquid | Replenisher |
|---|---|---|
| disodium ethylenediamine-tetraacetate · $2H_2O$ | 4.0 g | Same as mother liquid |

| | |
|---|---|
| iron (III) ammonium ethylenediaminetetra acetate · $2H_2O$ | 46.0 g |
| ammonium thiosulfate (700 g/l) | 155 ml |
| sodium p-toluenesulfinate | 20.0 g |
| sodium hydrogensulfite | 12.0 g |
| ammonium bromide | 50.0 g |
| ammonium nitrate | 30.0 g |
| water | ad 1000 ml |
| pH (25°C) | 6.20 |

Washing water (The same water was used as mother liquid and replenisher)

Tap water was passed through a column filled with H-type strong acid cation exchange resin (Rohm & Hass, Amberlite IR-120B) and OH-type anion exchange resin (Amberlite IR-400) to reduce calcium and magnesium ions concentration to 3 mg/l or less, and then sodium isocyanurate dichloride (20 mg/l) and sodium sulfate (1.5 g/l) were addd to the ion-exchanged water. The pH of this water ranged from 6.5 to 7.5.

Example 5

A multilayer silver halide photosensitive material 301 having the following layer structure was formed on a cellulose triacetate film support having prime-coating.

(layer structure)

The compositions of the respective layers will be shown below. The numerals stands for the applied amount ($g/m^2$). The amount of the silver halide emulsion is given in terms of silver.

The first layer (anti-halation layer)

| | | |
|---|---|---|
| black colloidal silver | silver | 0.18 |
| gelatin | | 1.40 |

The second layer (interlayer)

| | | |
|---|---|---|
| 2,5-di-t-pentadecyl hydroquinone | | 0.18 |
| EX-1 | | 0.07 |
| EX-3 | | 0.02 |
| EX-12 | | 0.002 |
| U-1 | | 0.06 |
| U-2 | | 0.08 |
| U-3 | | 0.10 |
| HBS-1 | | 0.10 |
| HBS-2 | | 0.02 |
| gelatin | | 1.04 |

The third layer (the first red-sensitive layer)

| | | |
|---|---|---|
| emulsion A | silver | 0.25 |
| emulsion B | silver | 0.25 |
| sensitizing dye I | | $6.9x10^{-5}$ |
| sensitizing dye II | | $1.8x10^{-5}$ |
| sensitizing dye III | | $3.1x10^{-4}$ |
| EX-2 | | 0.335 |
| EX-10 | | 0.020 |
| HBS-1 | | 0.060 |
| gelatin | | 0.87 |

The fourth layer (the second red-sensitive layer)

| | | |
|---|---|---|
| emulsion G | silver | 1.0 |
| sensitizing dye I | | $5.1 \times 10^{-5}$ |
| sensitizing dye II | | $1.4 \times 10^{-5}$ |
| sensitizing dye III | | $2.3 \times 10^{-4}$ |
| EX-2 | | 0.400 |
| EX-3 | | 0.050 |
| EX-10 | | 0.015 |
| HBS-1 | | 0.060 |
| gelatin | | 1.30 |

The fifth layer (the third red-sensitive layer)

| | | |
|---|---|---|
| emulsion D | silver | 1.60 |
| sensitizing dye I | | $5.4 \times 10^{-5}$ |
| sensitizing dye II | | $1.4 \times 10^{-5}$ |
| sensitizing dye III | | $2.4 \times 10^{-4}$ |
| EX-3 | | 0.010 |
| EX-4 | | 0.080 |
| EX-2 | | 0.097 |
| HBS-1 | | 0.22 |
| HBS-2 | | 0.10 |
| gelatin | | 1.63 |

The sixth layer (interlayer)

| | |
|---|---|
| EX-5 | 0.040 |
| HBS-1 | 0.020 |
| gelatin | 0.80 |

The seventh layer (the first green-sensitive layer)

| | | |
|---|---|---|
| emulsion A | silver | 0.15 |
| emulsion B | silver | 0.15 |
| sensitizing dye V | | $3.0x10^{-5}$ |
| sensitizing dye VI | | $1.0x10^{-4}$ |
| sensitizing dye VII | | $3.8x10^{-4}$ |
| EX-6 | | 0.260 |
| EX-1 | | 0.021 |
| EX-7 | | 0.030 |
| EX-8 | | 0.025 |
| HBS-1 | | 0.100 |
| HBS-3 | | 0.010 |
| gelatin | | 0.63 |

The eighth layer (the second green-sensitive layer)

| | | |
|---|---|---|
| emulsion C | silver | 0.45 |
| sensitizing dye V | | $2.1x10^{-5}$ |
| sensitizing dye VI | | $7.0x10^{-5}$ |
| sensitizing dye VII | | $2.6x10^{-4}$ |
| EX-6 | | 0.094 |
| EX-8 | | 0.018 |
| EX-7 | | 0.026 |
| HBS-1 | | 0.160 |
| HBS-3 | | 0.008 |
| gelatin | | 0.50 |

The ninth layer (the third green-sensitive layer)

| | | |
|---|---|---|
| emulsion E | silver | 1.2 |
| sensitizing dye V | | $3.5 \times 10^{-5}$ |
| sensitizing dye VI | | $8.0 \times 10^{-5}$ |
| sensitizing dye VII | | $3.0 \times 10^{-4}$ |
| EX-13 | | 0.015 |
| EX-11 | | 0.100 |
| EX-1 | | 0.025 |
| HBS-1 | | 0.25 |
| HBS-2 | | 0.10 |
| gelatin | | 1.54 |

The tenth layer (yellow filter layer)

| | | |
|---|---|---|
| yellow colloidal silver | silver | 0.05 |
| EX-5 | | 0.08 |
| HBS-1 | | 0.03 |
| gelatin | | 0.95 |

The eleventh layer (the first blue-sensitive layer)

| | | |
|---|---|---|
| emulsion A | silver | 0.08 |
| emulsion B | silver | 0.07 |
| emulsion F | silver | 0.07 |
| sensitizing dye VIII | | $3.5 \times 10^{-4}$ |
| EX-9 | | 0.721 |
| EX-8 | | 0.042 |
| HBS-1 | | 0.28 |
| gelatin | | 1.10 |

The twelfth layer (the second blue-sensitive layer)

| | | |
|---|---|---|
| emulsion G | silver | 0.45 |
| sensitizing dye VIII | | $2.1 \times 10^{-4}$ |
| EX-9 | | 0.154 |
| EX-10 | | 0.007 |
| HBS-1 | | 0.05 |
| gelatin | | 0.78 |

The thirteenth layer (the third blue-sensitive layer)

| | | |
|---|---|---|
| emulsion H | silver | 0.77 |
| sensitizing dye VIII | | $2.2 \times 10^{-4}$ |
| EX-9 | | 0.20 |
| HBS-1 | | 0.07 |
| gelatin | | 0.69 |

The fourteenth layer (the first protective layer)

| | | |
|---|---|---|
| emulsion I | silver | 0.5 |
| U-4 | | 0.11 |
| U-5 | | 0.17 |
| HBS-1 | | 0.05 |
| gelatin | | 1.00 |

The fifteenth layer (the second protective layer)

| | |
|---|---|
| polymethylmethacryate particles (diameter: about 1.5 μm) | 0.54 |
| S-1 | 0.20 |
| gelatin | 1.20 |

A hardener for gelatin H-1 and a surfactant were added to each layer in addition to the above-mentioned components.

| | Mean content of AgI (%) | Mean particle size (μm) | Degree of variability concerning particle size(%) | Ratio (diameter/ thickness) | Ratio of amount of Silver (AgI content %) |
|---|---|---|---|---|---|
| Emulsion A | 4.1 | 0.45 | 27 | 1 | Core/Shell=1/3(13/1), double layer structure grain |
| Emulsion B | 8.9 | 0.70 | 14 | 1 | Core/Shell=3/7(25/2), double layer structure grain |
| Emulsion C | 10 | 0.75 | 30 | 2 | Core/Shell=1/2(24/3), double layer structure grain |
| Emulsion D | 16 | 1.05 | 35 | 2 | Core/Shell=1/2(40/0), double layer structure grain |
| Emulsion E | 10 | 1.05 | 35 | 3 | Core/Shell=1/2(24/3), double layer structure grain |
| Emulsion F | 4.1 | 0.25 | 28 | 1 | Core/Shell=1/3(13/1), double layer structure grain |
| Emulsion G | 13.6 | 0.75 | 25 | 2 | Core/Shell=1/2(40/0), double layer structure grain |
| Emulsion H | 14 | 1.30 | 25 | 3 | Core/Shell=37/63(34/3), double layer structure grain |
| Emulsion I | 1 | 0.07 | 15 | 1 | homogeneous grain |

EX-1

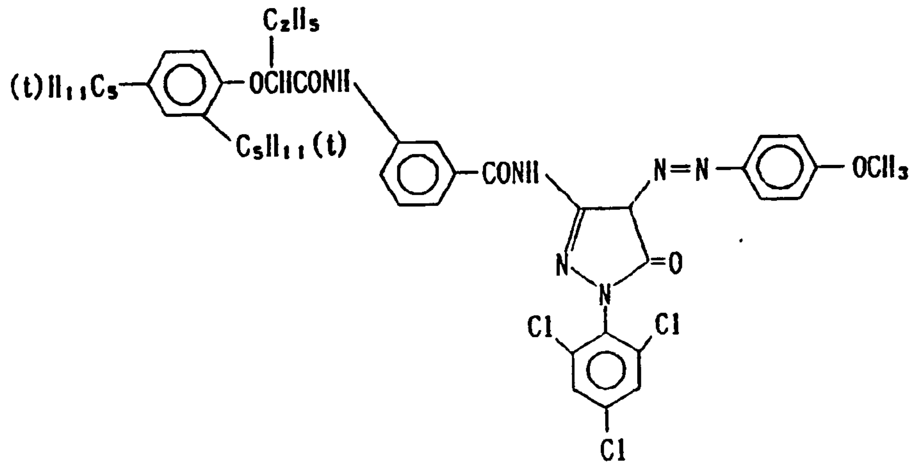

EX-2

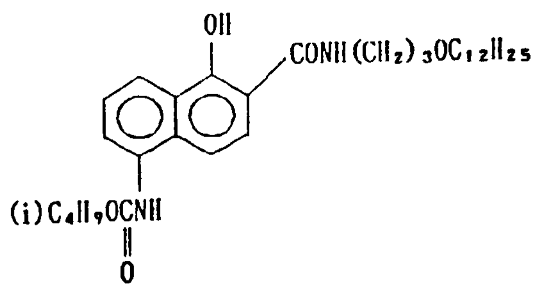

EX-3

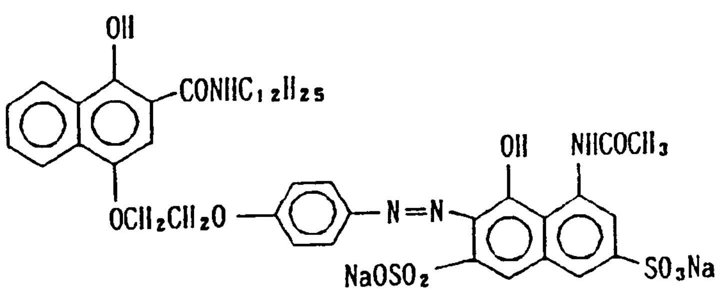

EX-4

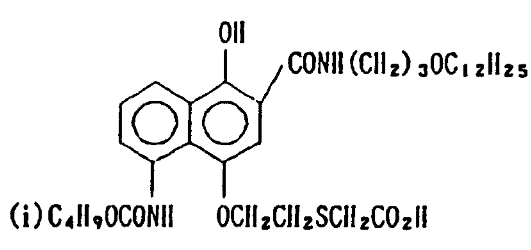

EX-5

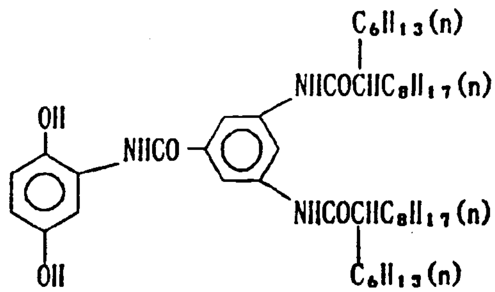

EX-6

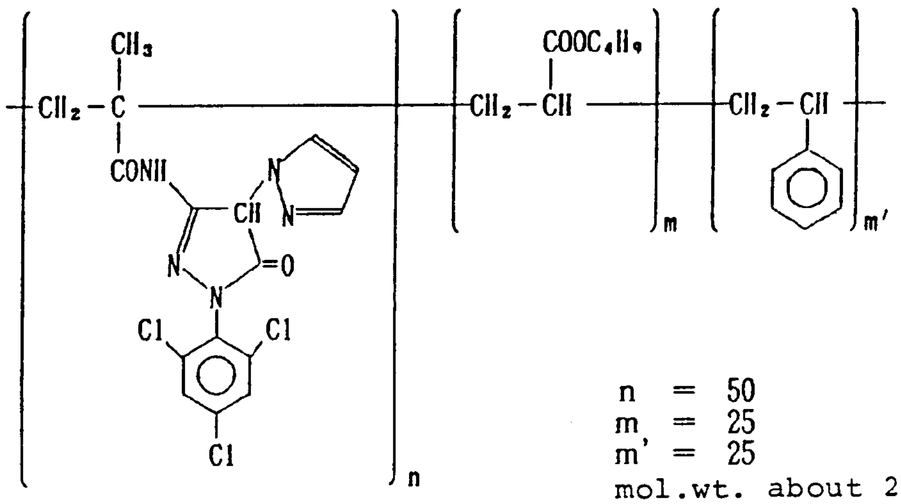

n = 50
m = 25
m' = 25
mol.wt. about 20,000

EX-7

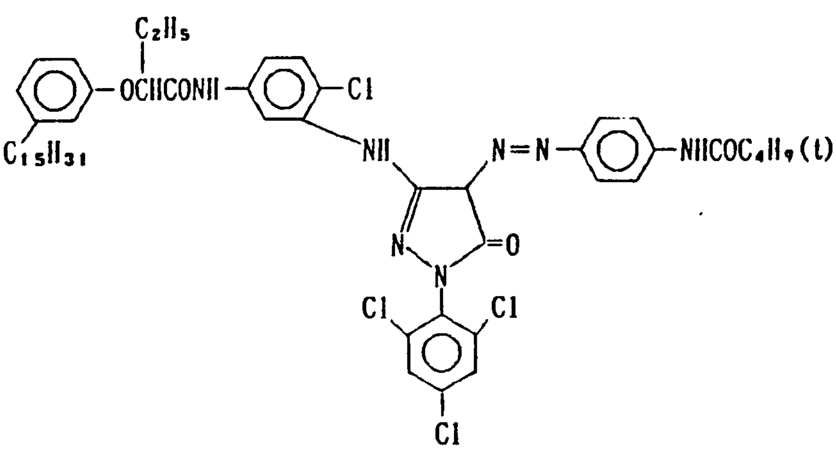

EX-8

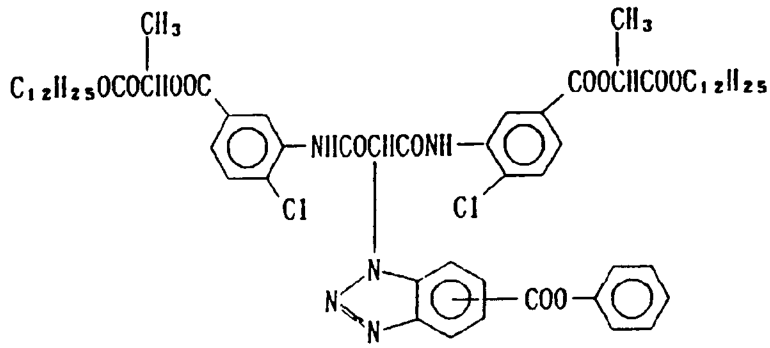

EX-9

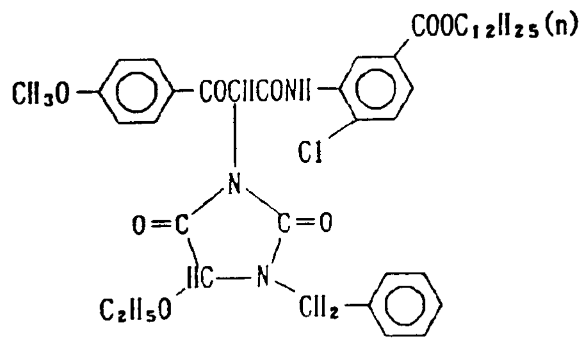

EX-10

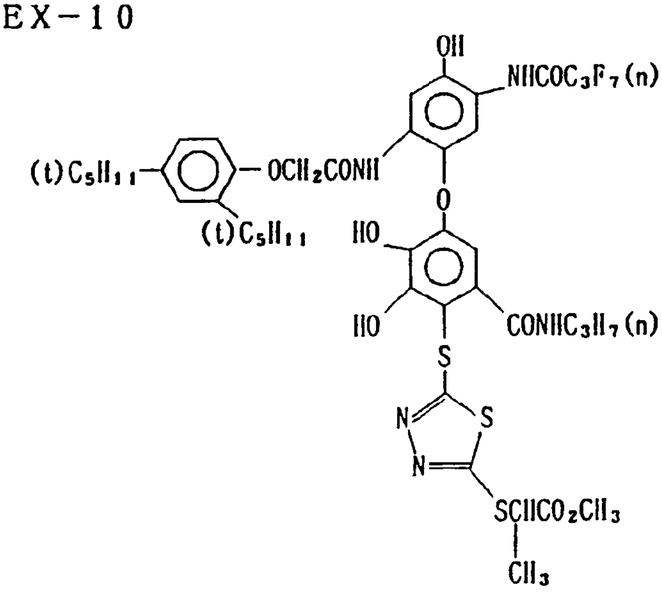

EX-11

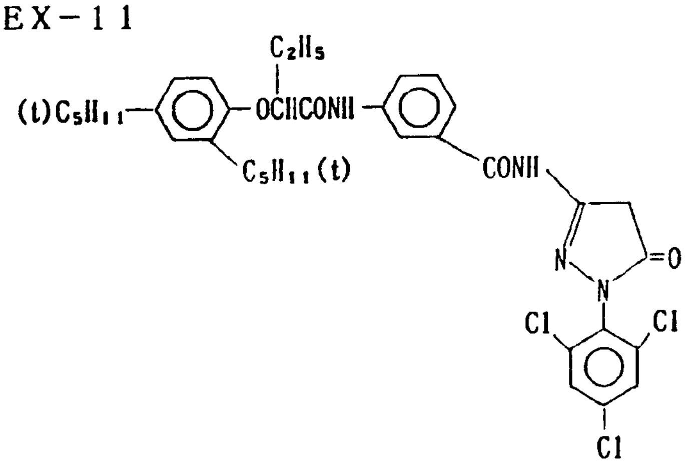

EX-12

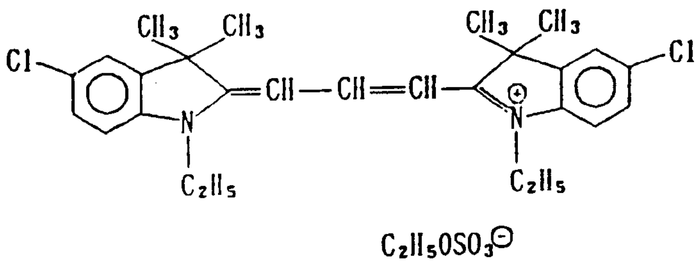

EX-13

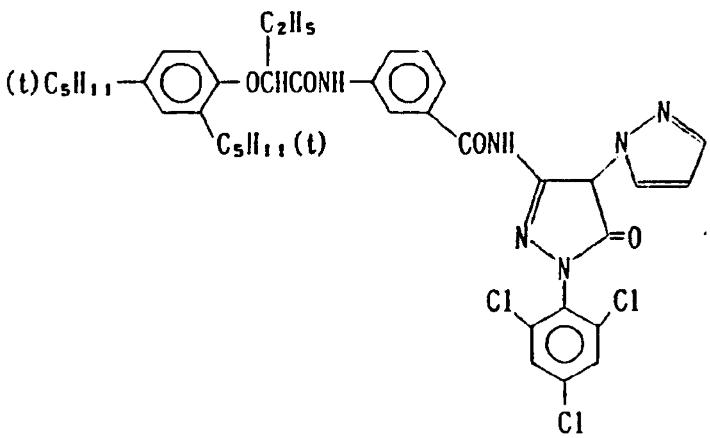

U-1

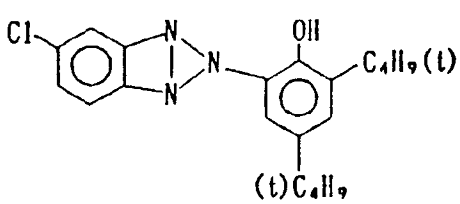

U-2

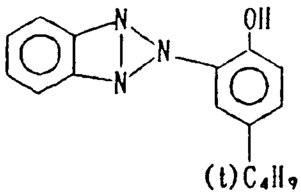

U-3

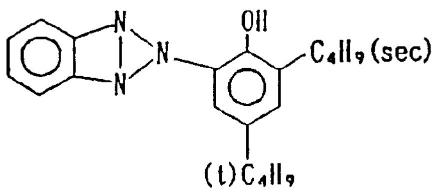

U-4

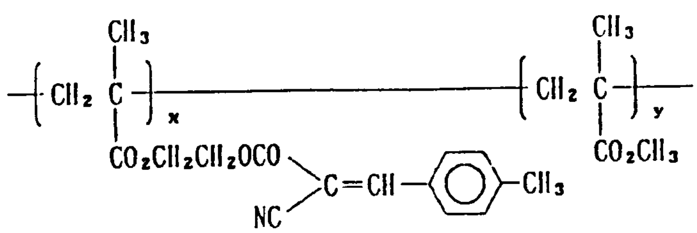

x : y = 70 : 30 (wt%)

UV-5

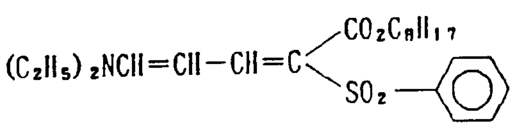

HBS-1 tricresylphosphate

HBS-2 di-n-butylphthalate

HBS-3

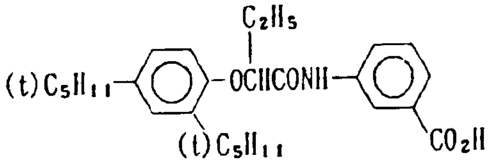

Sensitizing dye I

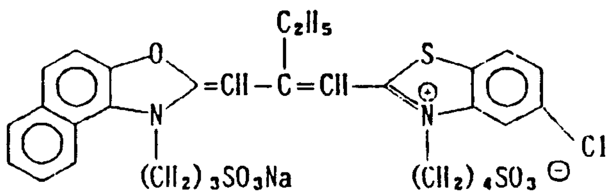

Sensitizing dye II

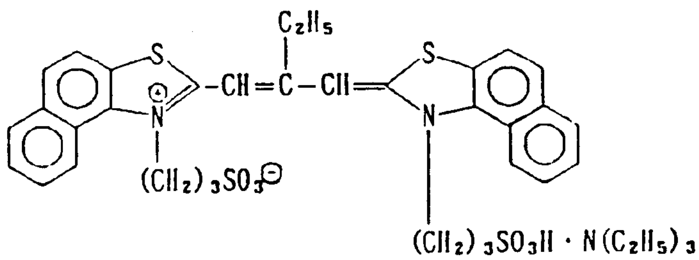

Sensitizing dye III

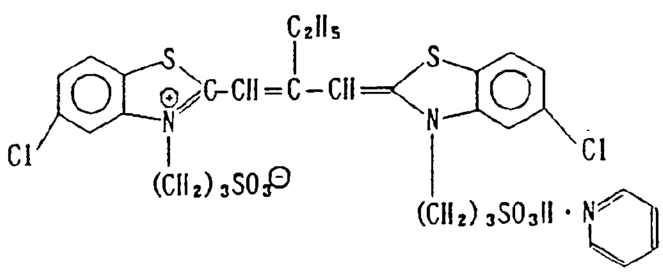

Sensitizing dye V

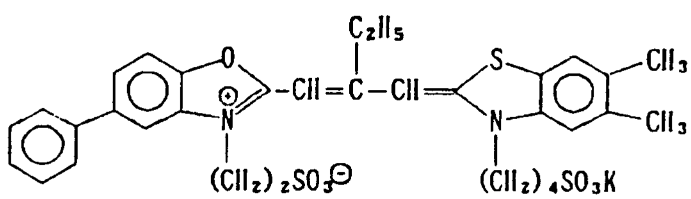

Sensitizing dye VI

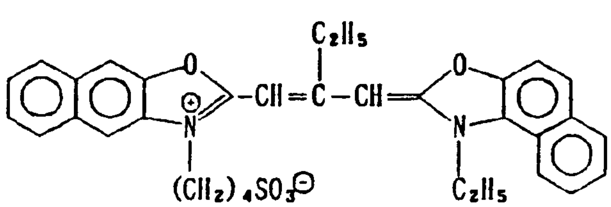

Sensitizing dye VII

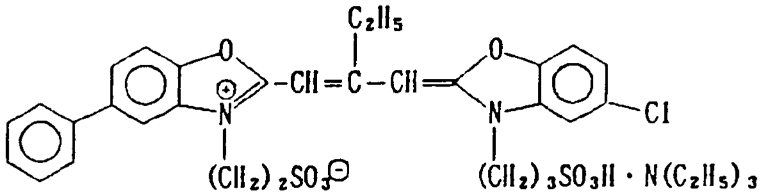

Sensitizing dye VIII

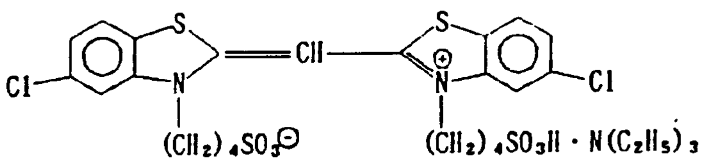

S − 1

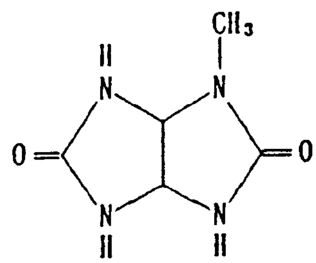

H − 1

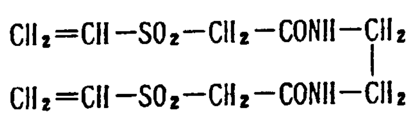

A sample prepared as mentioned above was sample 301.

Sample 302 was prepared according to the same procedures as those of Sample 301 except that an equimolar amount of cyan coupler (1) of the present invention was used in place of cyan couplers Ex-2 and Ex-4 used in the third, fourth and fifth layers.

Samples 303 - 307 were respectively prepared in the same manner as that of the preparation of Sample 301 except that cyan couplers Ex-2 and Ex-4 were replaced with an equimolar amount of cyan couplers (5), (6), (9), (16) and (17).

The resulting Samples 301 - 307 were exposed through a red filter and then treated in the following steps.

The spectral absorption of the treated samples was measured and it was found that a spectral absorption property of the samples (302 - 307) using the cyan couplers of the present invention is sharper than that of Sample 301.

Treatment steps

| Steps | Temperature | Time | Replenisher* | Tank volume |
|---|---|---|---|---|
| Color development | 37.8°C | 3'15" | 21 ℓ | 5 ℓ |
| Bleach-fixing | 38.0°C | 45" | 4.5 | 2 ℓ |
| Fixing (1) | 38.0°C | 45" | ** 30 | 2 ℓ |
| Fixing (2) | 38.0°C | 45" | | 2 ℓ |
| Stabilizing (1) | 38.0°C | 20" | *** 35 | 1 ℓ |
| Stabilizing (2) | 38.0°C | 20" | | 1 ℓ |
| Stabilizing (3) | 38.0°C | 20" | | 1 |
| Drying | 55°C | 1'00" | | |

* Amount of replenisher are expressed on the basis of 1 $m^3$ of photosensitive material.

** Two-tank countercurrent system

*** Three-tank countercurrent system

A jet agitator disclosed in JP-A- 62-183460 (page 3) was set in the fixing tanks in the automatical developing machine used.

The above treatment was conducted while a jet of a fixing solution was sprayed on the emulsion surface of the treated photosensitive material.

| Color developer | | Mother liquid (g) | Replenisher (g) |
|---|---|---|---|
| hydroxyethyliminodiacetatic acid | | 5.0 | 6.0 |
| sodium sulfite | | 4.0 | 3.0 |
| potassium carbonate | | 30.0 | 37.0 |
| potassium bromide | | 1.3 | 0.5 |
| potassium iodide | | 2.0 | 3.6 |
| hydroxyamine sulfate | | 1.2 mg | - |
| 4-[N-ethyl-N-β-hydroxyethyl-amino]-2-methylaniline sulfate | | $1.0 \times 10^{-2}$ mol | $1.3 \times 10^{-2}$ mol |
| water | ad | 1.0 ℓ | 1.0 ℓ |
| | | 10.00 | 10.15 |

**Bleaching solution**

| | | Mother liquid (g) | Replenisher (g) |
|---|---|---|---|
| iron (III) 1,3-diaminopropane tetraacetate | | 130 | 190 |
| 1,3-diaminopropane tetraacetatic acid | | 3.0 | 4.0 |
| ammonium bromide | | 85 | 120 |
| acetic acid | | 50 | 70 |
| ammonium nitrate | | 30 | 40 |
| water | ad | 1.0 ℓ | 1.0 ℓ |
| pH (adjusted with acetic acid or ammonia) | | 4.3 | 3.5 |

| Fixing solution | | Mother liquid (g) | Replenisher (g) |
|---|---|---|---|
| 1-hydroxyethylidene-1,1-diphosphonic acid | | 5.0 | 7.0 |
| disodium ethylenediamine tetraacetate | | 0.5 | 0.7 |
| sodium sulfite | | 10.0 | 12.0 |
| sodium hydrogensulfite | | 8.0 | 10.0 |
| aqueous ammonium thiosulfate solution (700 g/ℓ) | | 170.0 ml | 200.0 ml |
| Rhodanammon | | 100.0 | 150.0 |
| thiourea | | 3.0 | 5.0 |
| 3,6-dithia-1,8-octanediol | | 3.0 | 5.0 |
| water | ad | 1.0 ℓ | 1.0 ℓ |
| pH (adjusted with acetic acid or ammonia) | | 6.5 | 6.7 |

Stabilizing solution (Composition of replenisher is the same as that of mother liquid)

| | | |
|---|---|---|
| formalin | | 1.2 ml |
| 5-chloro-2-methyl-4-isothiazolin-3-one | | 6.0 mg |
| 2-methyl-4-isothiazolin-3-one | | 3.0 mg |
| surfactant $(C_{10}H_{21}-O-(CH_2CH_2O)_{10}-H)$ | | 0.4 |
| ethylene glycol | | 1.0 |
| water | ad | 1.0 ℓ |
| pH | | 5.0 - 7.0 |

Example 6

A multilayer silver halide photosensitive material 401 having the following layer structure was formed on a cellulose triacetate film support (thickness: 127μ) with prime-coating.
The first layer (anti-halation layer)
gelatin layer (thickness of dried one: 2μ) containing:

| | | |
|---|---|---|
| black colloidal silver | | 0.25 $g/m^2$ |
| U.V. absorber | U-1 | 0.04 $g/m^2$ |
| U.V. absorber | U-2 | 0.1 $g/m^2$ |
| U.V. absorber | U-3 | 0.1 $g/m^2$ |
| high b.p. solvent | O-1 | 0.1 $cc/m^2$ |

The second layer (interlayer)
gelatin layer (thickness of dried one: 1μ) containing:

| | |
|---|---|
| A-14 | 2.5 $mg/m^2$ |
| compound H-1 | 0.05 $g/m^2$ |

| | | |
|---|---|---|
| emulsion A | silver | 0.05 $g/m^2$ |
| high b.p. solvent O-2 | | 0.05 $cc/m^2$ |

The third layer (the first red-sensitive layer)
gelatin layer (thickness of dried one: 0.7μ) containing:

| | | |
|---|---|---|
| monodispersed silver bromoiodid emulsion spectrally sensitized with sensitizing dyes S-1 (0.47 $mg/m^2$) and S-2 (0.02 $mg/m^2$) | silver | 0.15 $g/m^2$ |

(iodine content: 4 mol%, mean particle size: 0.20μ, degree of variabulity: 12%)

| | | |
|---|---|---|
| monodispersed internal latent type silver bromoiodide emulsion spectrally sensitized with sensitizing dyes S-1 (0.51 $mg/m^2$) and S-2 (0.03 $mg/m^2$) | silver | 0.20 $g/m^2$ |

(iodine content: 4 mol%, mean particle size: 0.40 μ, distance from latent image to particle surface: 100 Å, degree of variability: 14%)

| | | |
|---|---|---|
| emulsion B | silver | 0.05 g/m$^2$ |
| A-1 | | 0.60 mg/m$^2$ |
| compound H-6 | | 0.01 g/m$^2$ |
| coupler C-1 | | 0.13 g/m$^2$ |
| coupler C-2 | | 0.033 g/m$^2$ |
| coupler C-10 | | 0.1 g/m$^2$ |
| high b.p. solvent O-2 | | 0.02 cc/m$^2$ |

The fourth layer (the second red-sensitive layer)
gelatin layer (thickness of dried one: 1.7μ) containing:

| | | |
|---|---|---|
| monodispersed silver bromoiodide emulsion spectrally sensitized with sensitizing dyes S-1 (1.1 mg/m$^2$) and S-2 (0.04 mg/m$^2$) | silver | 0.53 g/m$^2$ |

(iodine cntent: 3 mol%, mean particle size: 0.55μ, degree of variability: 16%)

| | |
|---|---|
| A-4 | 0.02 mg/m$^2$ |
| coupler C-1 | 0.40 g/m$^2$ |
| coupler C-2 | 0.07 g/m$^2$ |
| coupler C-9 | 0.05 g/m$^2$ |
| high b.p. solvent O-2 | 0.22 cc/m$^2$ |

The fifth layer (the third red-sensitive layer)
gelatin layer (thickness of dried one: 1.7μ containing:

| | | |
|---|---|---|
| monodispersed silver bromoiodide emulsion spectrally sensitized with sensitizing dyes S-1 (1.1 mg/m$^2$) and S-2 (0.04 mg/m$^2$) | silver | 0.53 g/m$^2$ |

(iodine content: 2 mol%, mean particle size: 0.07 μ, degree of variability: 17%)

| | |
|---|---|
| A-7 | 1.2 mg/m$^2$ |
| coupler C-6 | 0.35 g/m$^2$ |
| coupler C-8 | 0.20 g/m$^2$ |
| high b.p. solvent O-2 | 0.06 cc/m$^2$ |

The sixth layer (interlayer)
gelatin layer (thickness of dried one: 1μ) containing:

| | |
|---|---|
| A-10 | 10 mg/m$^2$ |
| A-11 | 5 mg/m$^2$ |
| compound H-1 | 0.1 g/m$^2$ |
| high b.p. solvent O-2 | 0.1 cc/m$^2$ |

The seventh layer (the first green-sensitive layer)
gelatin layer (thickness of dried one: 0.7 μ) containing:

| | | |
|---|---|---|
| monodispersed silver bromoiodide emulsion spectrally sensitized with sensitizing dyes S-3 (2.2 mg/m$^2$) and S-2 (1.0 mg/m$^2$) | silver | 0.5 g/m$^2$ |

(iodine content: 3 mol%, mean particle size: 0.35 μ, degree of variability: 19%)

| | | |
|---|---|---|
| emulsion B | silver | 0.05 g/m$^2$ |
| A-5 | | 0.12 mg/m$^2$ |
| compound H-6 | | 0.01 g/m$^2$ |
| compound H-5 | | 0.005 g/m$^2$ |
| coupler C-3 | | 0.27 g/m$^2$ |
| high b.p. solvent O-2 | | 0.05 cc/m$^2$ |

The eighth layer (the second green-sensitive layer)
gelatin layer (thickness of dried one: 1.7μ containing:

| | | |
|---|---|---|
| monodispersed internal latent image type silver bromoiodide emulsion spectrally sensitized with sensitizing dyes S-3 (0.29 g/m$^2$) and S-4 (0.3 mg/m$^2$) | silver | 0.5 g/m$^2$ |

(iodine content: 2.5 mol%, mean particle size: 0.5 μ, distance from latent image to particle surface: 100 Å, degree of variability: 18%)

| | |
|---|---|
| A-6 | 0.2 mg/m$^2$ |
| compound H-6 | 0.01 g/m$^2$ |
| coupler C-3 | 0.2 g/m$^2$ |
| high b.p. solvent O-2 | 0.13 cc/m$^2$ |

The ninth layer (the third green-sensitive layer)
gelatin layer (thickness of dried one: 1.7μ) containing:

| | | |
|---|---|---|
| tabular silver bromoiodide emulsion spectrally sensitized with sensitizing dyes S-3 (0.9 $g/m^2$) and S-4 (0.3 $mg/m^2$) | silver | 0.5 $g/m^2$ |

(iodine content: 2 mol% content of grains having 7 or more of diameter/thickness ratio: 50% (on the basis of projected area of the whole grains), mean thickness of grains: 0.10 μ)

| | |
|---|---|
| A-2 | 1.5 $mg/m^2$ |
| coupler C-4 | 0.2 $g/m^2$ |
| high b.p. solvent O-2 | 0.03 $cc/m^2$ |

The tenth layer (interlayer)
gelatin layer (thickness of dried one: 0.05μ) containing:

| | |
|---|---|
| compound H-4 | 0.1 $g/m^2$ |
| high b.p. solvent O-2 | 0.1 $cc/m^2$ |

The eleventh layer (yellow filter layer)
gelatin layer (thickness of dried one: 1μ) containing:

| | | |
|---|---|---|
| yellow colloidal silver | silver | 0.12 $g/m^2$ |
| compound A-15 | | 0.22 $g/m^2$ |
| compound H-1 | | 0.02 $g/m^2$ |
| compound H-2 | | 0.03 $g/m^2$ |
| high b.p. solvent O-2 | | 0.04 $cc/m^2$ |

The twelfth layer (the first blue-sensitive layer)
gelatin layer (thickness of dried one: 1.5μ) containing:

| | | |
|---|---|---|
| tabular silver bromoiodide emulsion spectrally sensitized with sensitizing dye S-5 (1.0 $g/m^2$) | silver | 0.6 $g/m^2$ |

(iodine content: 3 mol%, content of grains having 7 or more of diameter/thickness ratio: 50% (on the basis of projected area of the whole grains), mean thickness of grains: 0.10 μ)

| | | |
|---|---|---|
| emulsion A | silver | 0.1 g/$m^2$ |
| A-7 | | 0.5 mg/$m^2$ |
| coupler C-5 | | 0.5 g/$m^2$ |
| high b.p. solvent O-2 | | 0.1 cc/$m^2$ |

The thirteenth layer (the second blue-sensitive layer)
gelatin layer (thickness of dried one: 3μ) containing:

| | | |
|---|---|---|
| tabular silver bromoiodide emulsion spectrally sensitized with sensitizing dye S-5 (2.0 g/$m^2$) | silver | 1.1 g/$m^2$ |

(iodine content: 2.5 mol%, content of grains having 7 or more of diameter/thickness ratio: 50% (on the basis of projected area of the whole grains), mean thickness of grains: 0.15 μ)

| | |
|---|---|
| A-12 | 10 mg/$m^2$ |
| coupler C-7 | 1.2 g/$m^2$ |
| coupler C-8 | 0.2 g/$m^2$ |
| high b.p. solvent O-2 | 0.07 cc/$m^2$ |

The fourteenth layer (the first protective layer)
gelatin layer (thickness of dried one: 2μ) containing:

| | |
|---|---|
| A-13 | 0.10 mg/$m^2$ |
| U.V. absorber U-1 | 0.02 g/$m^2$ |
| U.V. absorber U-2 | 0.03 g/$m^2$ |
| U.V. absorber U-3 | 0.03 g/$m^2$ |
| U.V. absorber U-4 | 0.29 g/$m^2$ |
| high b.p. solvent O-2 | 0.28 cc/$m^2$ |

The fifteenth layer (the second protective layer)
gelatin layer (thickness of dried one: 0.8 μ) containing:

| | | |
|---|---|---|
| emulsion of silver bromoiodide fine particles of which surface has been fogged | silver | 0.1 g/$m^2$ |

(iodine content: 1 mol%, mean particle size: 0.06 μ)

| | |
|---|---|
| A-8 | 10 $mg/m^2$ |
| polymethylmethacrylate particles (mean particle size: 1.5 μ) | 0.1 $g/m^2$ |
| A-3 | 0.2 $g/m^2$ |
| A-9 | 1.0 $mg/m^2$ |

Anti-foggant A-16, hardener for gelatin H-3 and a surfactant were added to each layer in addition to the above-mentioned components.

Preparation method of emulsions A and B

Emulsion A was prepared by preparing cubic silver bromide (mean particle size: 0.15 μ) by the controlled double-jet method and fogging the resulting silver bromide with hydradine and a complex of gold under the low pAg condition.

Emulsion B was prepared by providing a silver bromide shell (thickness: 250 Å) on the surface of silver bromide grains of emulsion A.

Compounds used in this example were shown below:

C - 1

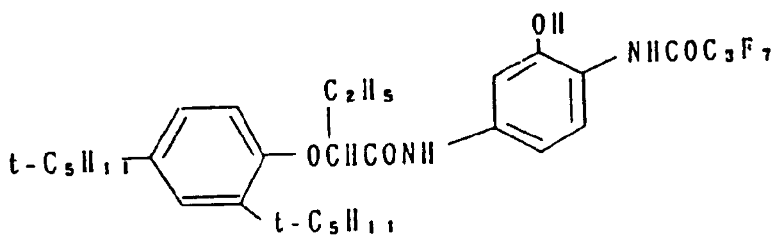

C - 2

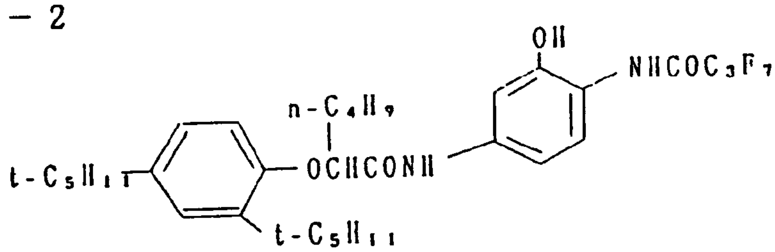

C - 3

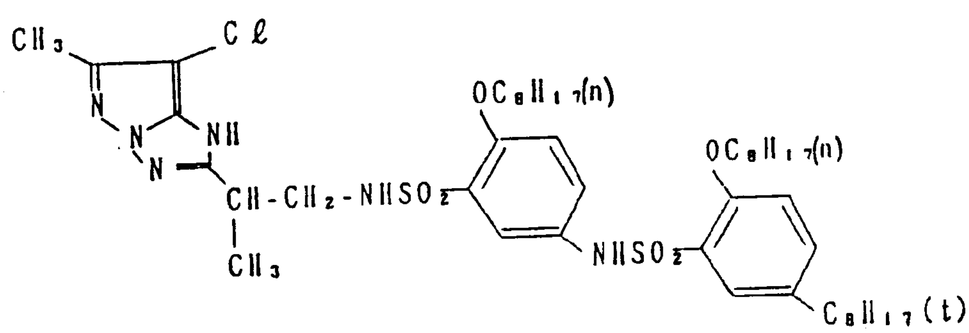

C - 4

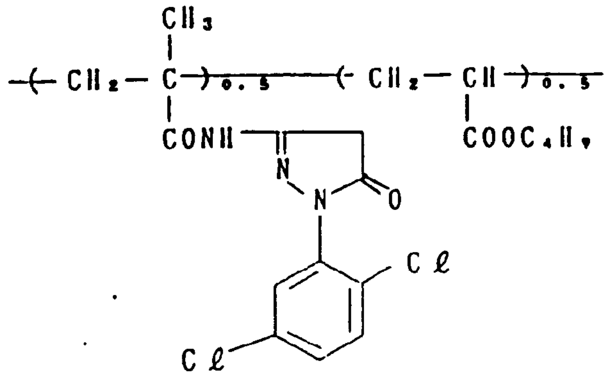

C - 5

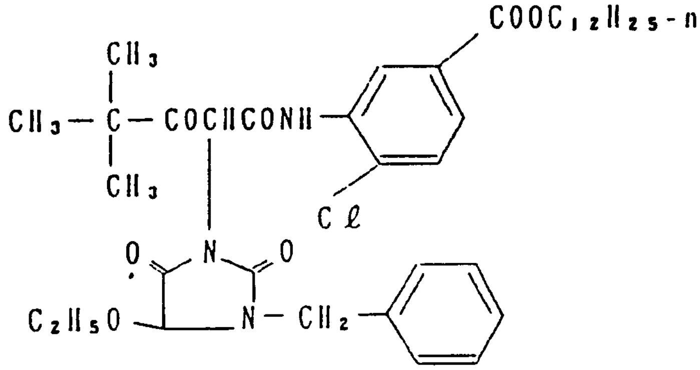

C - 6

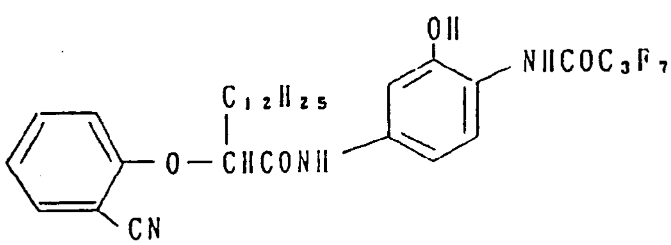

C - 7

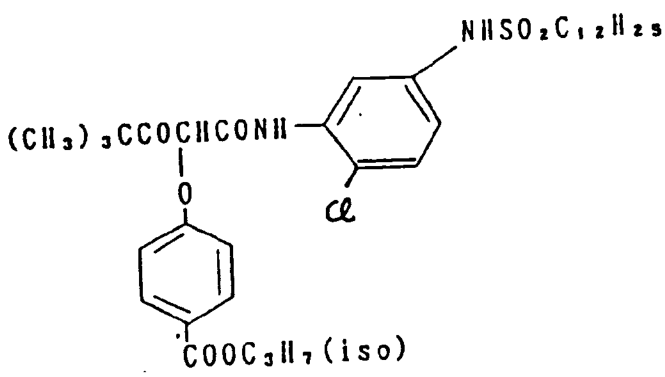

C − 8

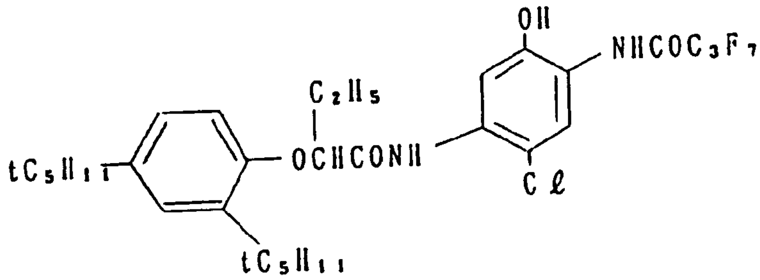

C − 9

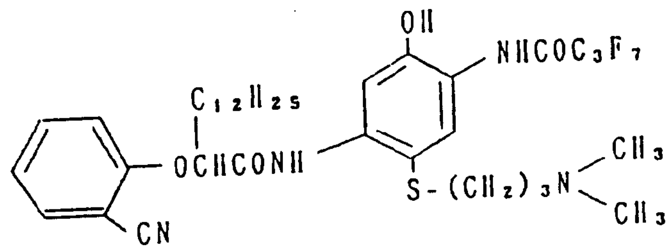

C − 1 0

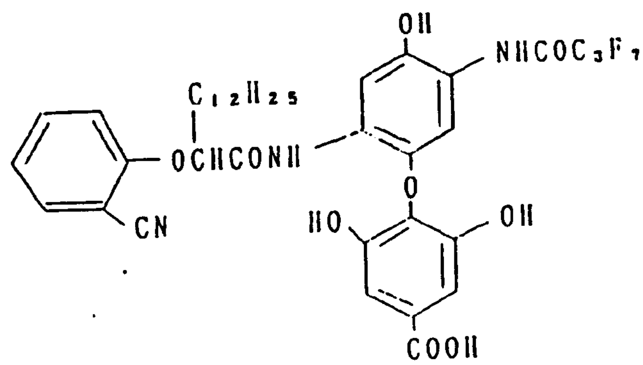

U − 1

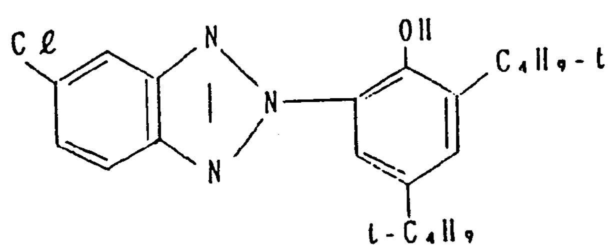

U − 2

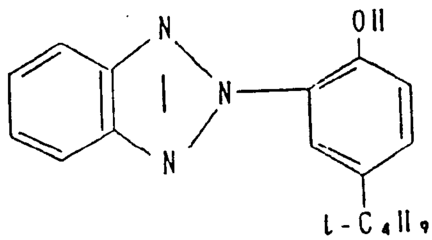

U − 3

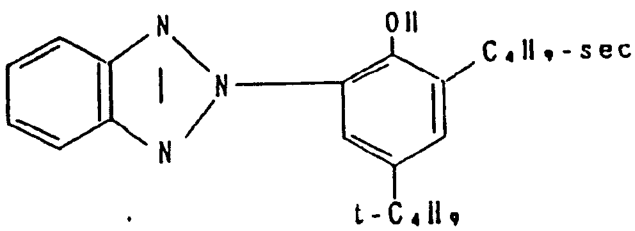

U − 4

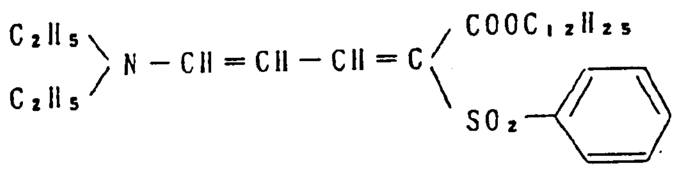

H − 1

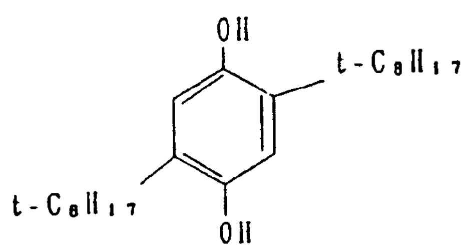

H − 2

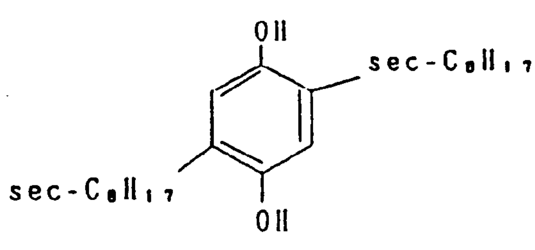

H − 3

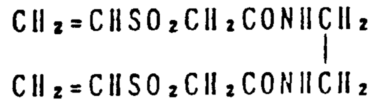

H − 4

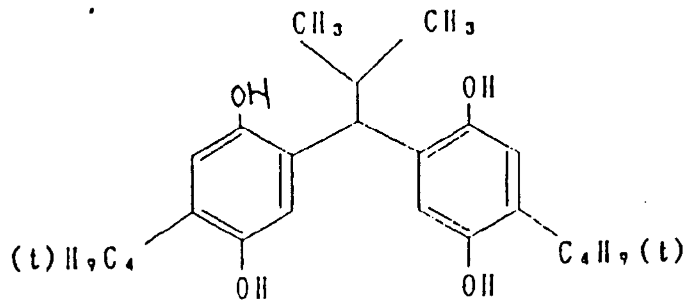

H − 5

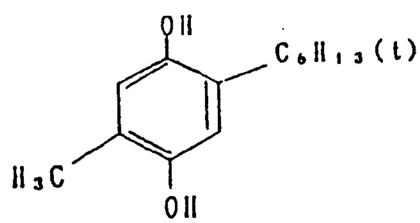

H − 6

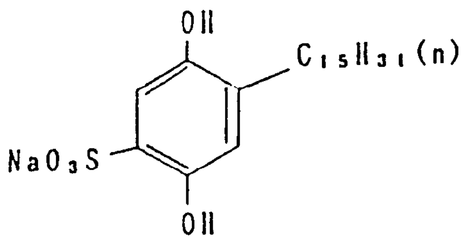

O − 1

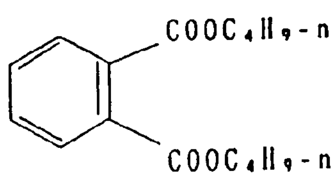

O − 2

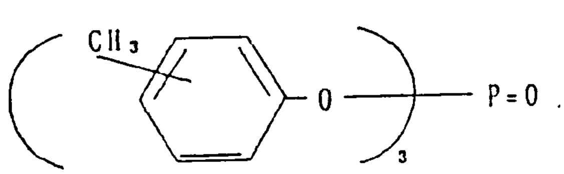

S - 1

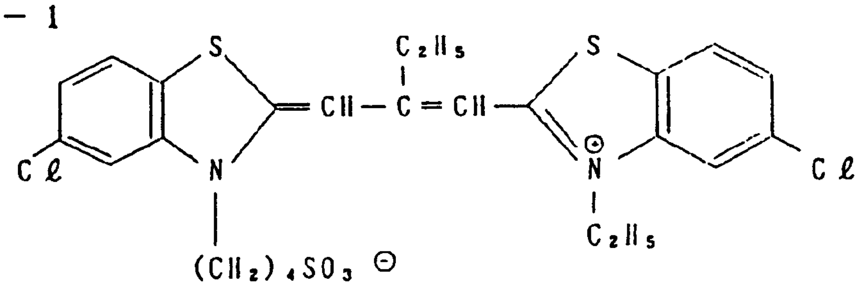

S - 2

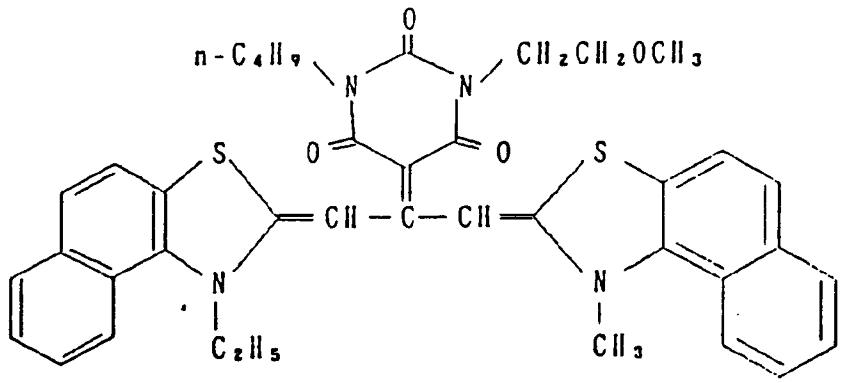

S - 3

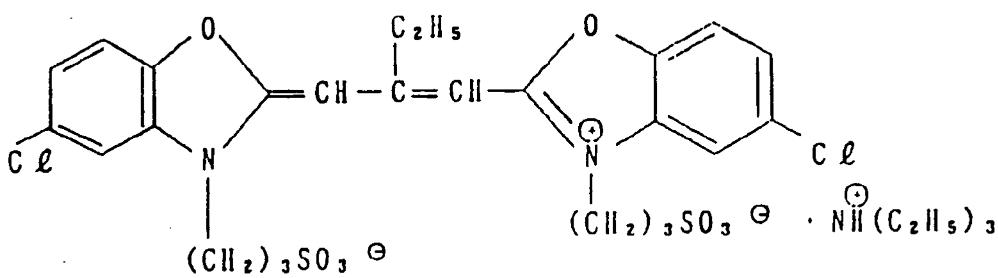

S - 4

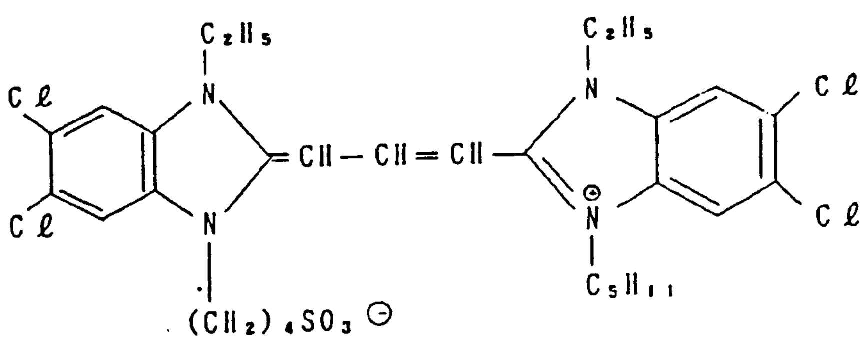

S - 5

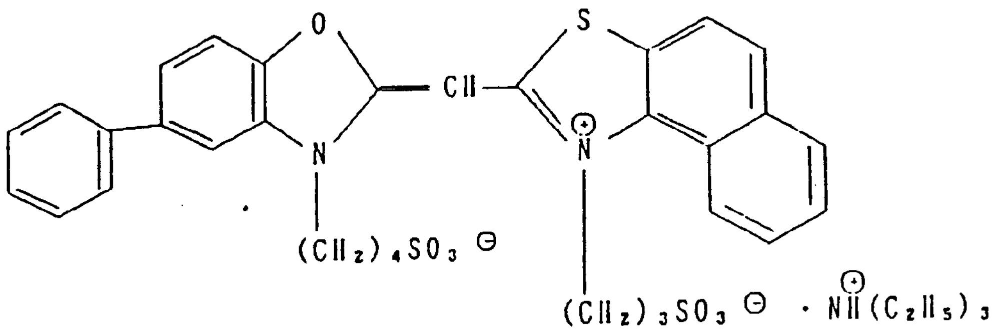

A - 1

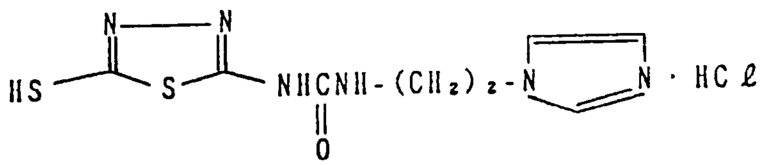

A - 2

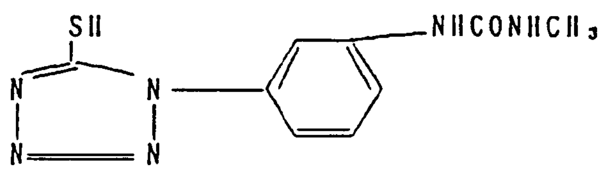

A - 3

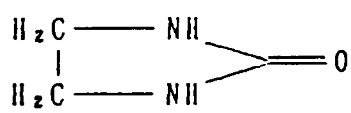

A - 4

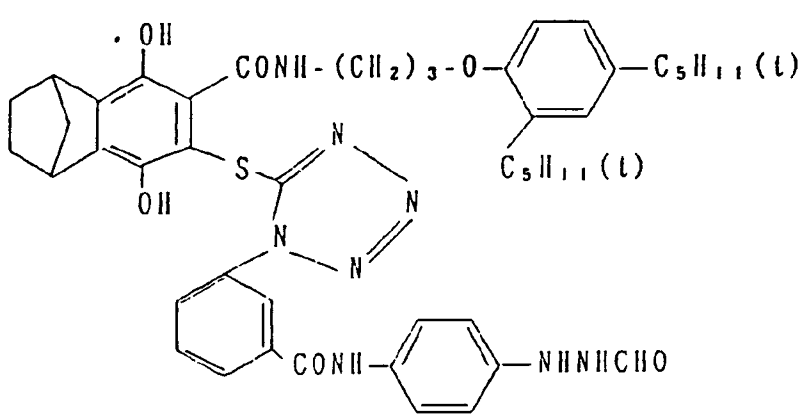

A - 5

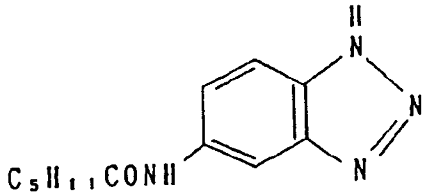

A - 6

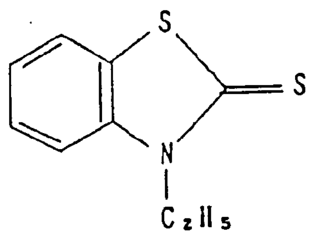

A - 7

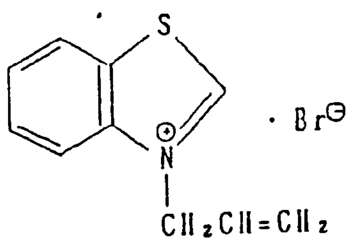

A - 8

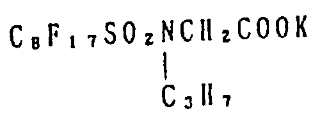

A - 1 2

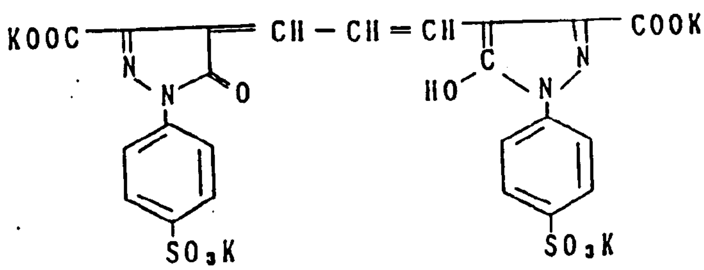

A－13

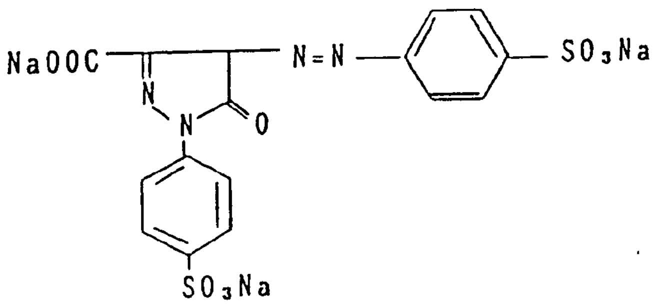

A－14

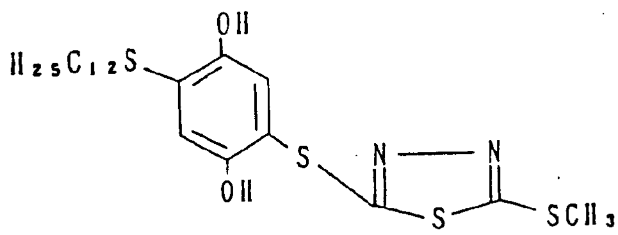

A－9

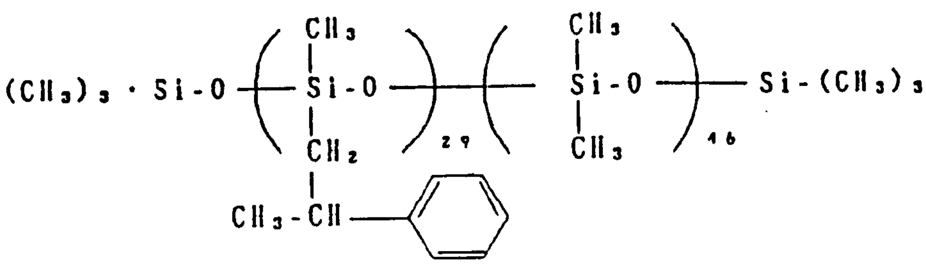

A－10

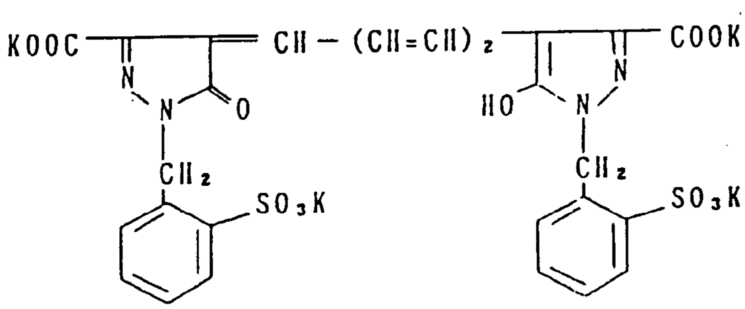

A - 11

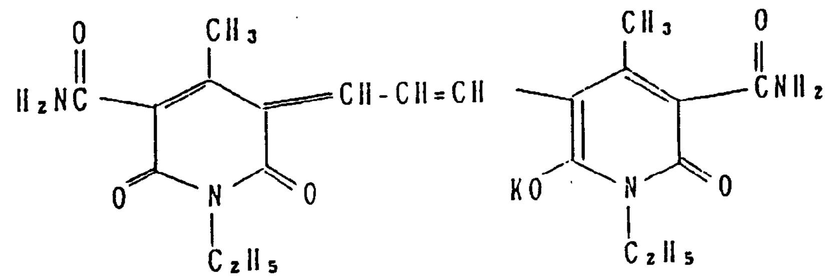

A - 15

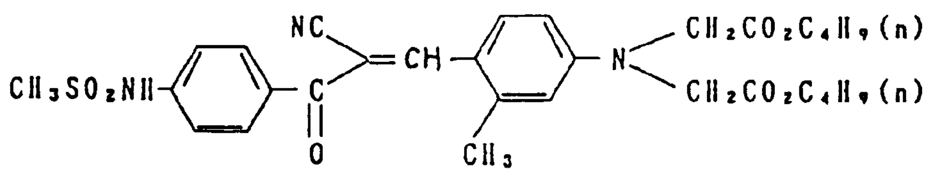

A - 16

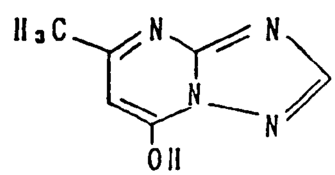

A sample prepared as mentioned above was Sample 401.

Sample 402 was prepared according to the same procedures as those of Sample 401 except that an equimolar amount of cyan coupler (1) of the present invention was used in place of cyan couplers ExC-1, 2, 6, and 8 used in the third, fourth and fifth layers.

Samples 403 - 407 were respectively prepared in the same manner as that of the preparation of Sample 401 except that cyan couplers ExC-1, 2, 6 and 8 were replaced with an equimolar amount of cyan couplers (5), (6), (9), (16) and (17).

The resulting Samples 401 - 407 were exposed through a red filter and then treated in the following steps.

The spectral absorption of the treated samples was measured and it was found that a spectral absorption property of the samples (402 - 407) using the cyan couplers of the present invention is sharper than that of Sample 401.

| Treatment steps | Time | Temperature |
|---|---|---|
| First development | 6' | 38°C |
| Washing | 2' | 38°C |
| Reversal | 2' | 38°C |
| Color developing | 6' | 38°C |
| Adjusting | 2' | 38°C |
| Bleaching | 6' | 38°C |
| Fixing | 4' | 38°C |
| Washing | 4' | 38°C |
| Stabilizing | 1' | 25°C |

The compositions of the treating solutions were as follows:

First developer

| | | |
|---|---|---|
| penta sodium nitrilo-N,N,N-trimethylene phosphonate | | 2.0 g |
| sodium sulfite | | 30 g |
| hydroquinone·potassium monosulfonate | | 20 g |
| potassium carbonate | | 33 g |
| 1-phenyl-4-methyl-4-hydroxymethyl-3-pyrazolidone | | 2.0 g |
| potassium bromide | | 2.5 g |
| potassium thiocyanate | | 1.2 g |
| potassium iodide | | 2.0 mg |
| water | ad | 1000 ml |
| pH (adjusted with HCl or KOH) | | 9.60 |

Reversal solution

| | | |
|---|---|---|
| penta sodium nitrilo-N,N,N-trimethylene phosphonate | | 3.0 g |
| $SnCl_4 \cdot 2H_2O$ | | 1.0 g |
| p-aminophenol | | 0.1 g |
| sodium hydroxide | | 8 g |
| glacial acetic acid | | 15 ml |
| water | ad | 1000 ml |
| pH (adjusted with HCl or NaOH) | | 6.00 |

Color developer

| | | |
|---|---|---|
| pentasodium nitrilo-N,N,N-trimethylene phosphonate | | 2.0 g |
| sodium sulfite | | 7.0 g |
| $Na_3PO_4 \cdot 12H_2O$ | | 36 g |
| potassium bromide | | 1.0 g |
| potassium iodide | | 90 mg |
| sodium hydroxide | | 3.0 g |
| citrazinic acid | | 1.5 g |
| N-ethyl-N-(β-methanesulfoneamidoethyl)-3-methyl-4-aminoaniline sulfate | | 11 g |
| 3,6-dithiaoctane-1,8-diol | | 1.0 g |
| water | ad | 1000 ml |
| pH (adjusted with HCl or KOH) | | 11.80 |

Adjusting solution

| | | |
|---|---|---|
| disodium ethylenediaminetetraacetate·$2H_2O$ | | 8.0 g |
| sodium sulfite | | 12 g |
| 1-thioglycerol | | 0.4 ml |
| water | ad | 1000 ml |
| pH (adjusted with HCl or NaOH) | | 6.20 |

Bleaching solution

| | | |
|---|---|---|
| disodium ethylenediaminetetraacetate·$2H_2O$ | | 2.0 g |
| Fe (III) ammonium ethylenediamine tetraacetate·$2H_2O$ | | 120 g |
| potassium bromide | | 100 g |
| ammonium nitrate | | 10 g |
| water | ad | 1000 ml |
| pH (adjusted with HCl or NaOH) | | 5.70 |

Fixing solution

| | | |
|---|---|---|
| sodium thiosulfate | | 80 g |
| sodium sulfite | | 5.0 g |
| sodium hydrogensulfite | | 5.0 g |
| water | ad | 1000 ml |
| pH (adjusted with HCl or aqueous ammonia) | | 6.60 |

Stabilizing solution

| | | |
|---|---|---|
| formalin (37%) | | 5.0 ml |
| polyoxyethylene-p-monononylphenyl ether (mean polymerization degree: 10) | | 0.5 ml |
| water | ad | 1000 ml |
| pH (not adjusted) | | |

## Claims

1. Cyan dye-forming couplers of the following formula (I) :

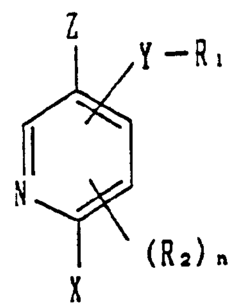

( I )

wherein $R_1$ represents an aliphatic group, an aromatic group or a heterocyclic group; $R_2$ represents a group or atom which can be bonded with the pyridine ring; X represents a hydrogen atom or a group which can be coupled off; Y represents a divalent bonding group having at least one amido bond and/or ester bond;
Z represents a dissociation group having an acid dissociation constant PKa of 5 to 12; and n represents 0, 1 or 2 with the proviso that when n is 2, two $R_2$'s are either the same or different groups or atoms and they do or do not combine to form a ring; when n is 1 or more, $R_1$ may form a ring together with $R_2$; at least one of $R_1$, $R_2$ and X may have one or more pyridyl residues of general formula (I) from which $R_1$, $R_2$ or X has been removed as the substituent(s); when n is 2 and one of $R_2$'s is a hydroxyl group and the hydroxyl group is at o- or p-position with respect to the nitrogen atom of the pyridyl ring, another $R_2$ can be bonded with the nitrogen atom of the pyridyl group and the hydroxyl group at o- or p-position can form a ketone by the tautomerism.

2. Cyan dye-forming couplers of claim 1 wherein the aliphatic group has 1 to 36 carbon atoms, the aromatic group has 6 to 36 carbon atoms and the heterocyclic group comprises a 3 to 8-membered ring.

3. Cyan dye-forming couplers of claim 1 wherein $R_2$ represents aliphatic groups, aromatic groups, heterocyclic groups, alkoxy groups, aryloxy groups, alkenyloxy groups, amino groups, acyl groups, ester groups, amido groups, sulfamido groups, imido groups, ureido group, aliphatic or aromatic sulfonyl groups, aliphatic or aromatic thio groups, hydroxyl group, cyano group, carboxyl group, or halogen atoms.

4. Cyan dye-forming couplers of claim 1 wherein $R_2$ is bonded with the nitrogen atom of the pyridine ring and R2 represents an aliphatic group, aromatic group, heterocyclic group, acyl group, sulfonyl group, aliphatic or aromatic oxy group or amino group.

5. Cyan dye-forming couplers of claim 1 wherein Y represents

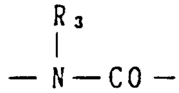 、 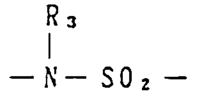 、 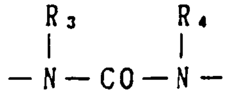 、

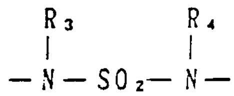 、 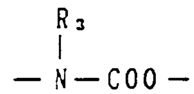 、

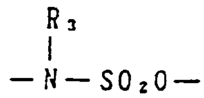 、 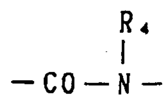

and

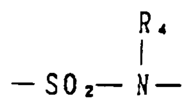

wherein $R_3$ and $R_4$ each represents a hydrogen atom, an aliphatic group, an aromatic group or a heterocyclic group.

6. Cyan dye-forming couplers of claim 5 wherein Y represents

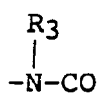 or 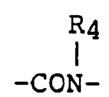 .

7. Cyan dye-forming couplers of claim 6 wherein Y represents -NHCO- which is bonded with the pyridine ring through the nitrogen atom.

8. Cyan dye-forming couplers of claim 1 wherein X is a hydrogen atom, halogen atom, aliphatic or aromatic oxy group, aliphatic or aromatic thio group, aliphatic or aromatic oxycarbonyloxy group, aliphatic or aromatic acyloxy group or aliphatic or aromatic sulfonyloxy group.

9. Cyan dye-forming couplers of claim 1 wherein $R_1$ and $R_2$ or $R_2$'s form a ring and the ring is 5 to 7-membered one.

10. Cyan dye-forming couplers of claim 1 wherein the dissociation group is hydroxyl group, sulfonamido group which can be substituted with an aliphatic, aromatic or heterocyclic group, or an active methine group having at least 1 electron-attractive group.

11. Cyan dye-forming couplers of claim 10 wherein the dissociation group is a hydroxyl group or aromatic sulfonamido group.

12. Cyan dye-forming coupers of claim 1 wherein -Y-$R_1$ is arranged in 2-position or 5-position with respect to N of the pyridine ring.

13. Cyan dye-forming couplers of claim 1 wherein -Y-$R_1$ is arranged at 2-position.

14. Cyan dye-forming couplers of claim 1 wherein the couplers have a so-called ballast group having 8 or more carbon atoms in at least one of $R_1$, $R_2$, X and Z.

15. Cyan dye-forming couplers of claim 1 wherein at least one of $R_1$, $R_2$ and X have one or more pyridyl groups of the general formula (I) from which $R_1$, $R_2$ or X has been removed.

**16.** Cyan dye-forming couplers of claim 15 wherein the pyridyl group has the following formula:

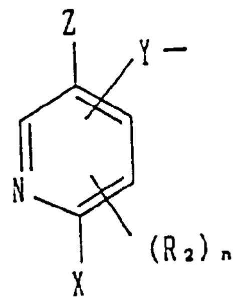

,

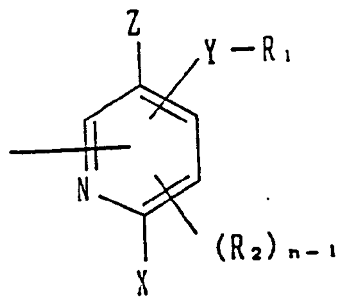

or

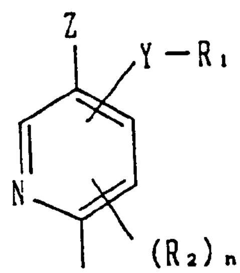

and the couplers are oligomers or polymers.

**17.** Silver halide photosensitive materials containing at least one cyan dye-forming coupler of general formula (I) set forth in claim 1.

**18.** Silver halide photosensitive materials of claim 17 wherein the coupler is dispersed by the oil-in-water dispersion method using a high-boiling organic solvent.

**19.** Silver halide photosensitive materials of claim 18 wherein the high-boiling organic solvent is at least one of the compounds of following formulae (VI) and (VII) or further with an assistant solvent.

Formula (VI):

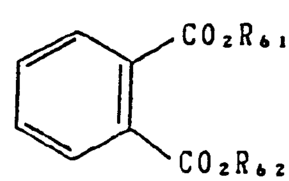

wherein $R_{61}$ and $R_{62}$ may be the same or different and they each represents an alkyl group, cycloalkyl group, alkenyl group or aryl group, with the proviso that the number of the total carbon atoms in $R_{61}$ and $R_{62}$ groups is 4 to 30;

Formula (VII):

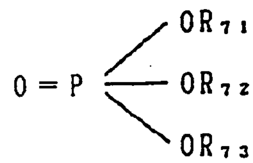

wherein $R_{71}$, $R_{72}$ and $R_{73}$ may be the same or different and they each represents an alkyl group, cycloalkyl group, alkenyl group or aryl group, with the proviso that the number of the total carbon atoms in $R_{71}$, $R_{72}$ and $R_{73}$ is 12 to 60.

## Patentansprüche

1. Cyanfarbstoff bildende Kuppler der folgenden Formel (I):

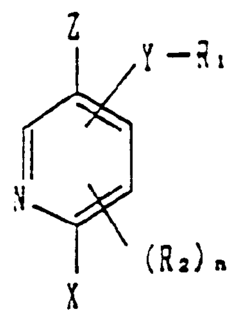

(I)

worin $R_1$ eine aliphatische Gruppe, eine aromatische Gruppe oder eine heterocyclische Gruppe bedeutet; $R_2$ bedeutet eine Gruppe oder ein Atom, welche(s) an den Pyridinring gebunden werden kann; X bedeutet ein Wasserstoffatom oder eine Gruppe, die abgekoppelt werden kann; Y bedeutet eine zweiwertige Bindungsgruppe mit mindestens einer Amidobindung und/oder Esterbindung; Z bedeutet eine Dissoziationsgruppe mit einer Säuredissoziationskonstante PKa von 5 bis 12; und n bedeutet 0, 1 oder 2, mit der Maßgabe, daß wenn n 2 ist, zwei $R_2$'s entweder gleiche oder verschiedene Gruppen oder Atome sind, und sie kombinieren oder kombinieren nicht zur Bildung eines Ringes; wenn n 1 oder mehr beträgt, kann $R_1$ zusammen mit $R_2$ einen Ring bilden; mindestens eines von $R_1$, $R_2$ und X kann einen oder mehrere Pyridylrest(e) der allgemeinen Formel (I) besitzen, von dem/denen $R_1$, $R_2$ oder X als Substituent(en) entfernt worden ist/sind; wenn n 2 ist und eines der $R_2$'s eine Hydroxylgruppe ist, und sich die Hydroxylgruppe bezüglich des Stickstoffatoms des Pyridylringes in o- oder p-Position befindet, kann ein anderes $R_2$ mit dem Stickstoffatom der Pyridylgruppe verbunden sein und die Hydroxylgruppe in der o- oder p-Position kann ein Keton durch Tautomerie bilden.

2. Cyanfarbstoff bildende Kuppler nach Anspruch 1, worin die aliphatische Gruppe 1 bis 36 Kohlenstoffatome besitzt, die aromatische Gruppe 6 bis 36 Kohlenstoffatome besitzt und die heterocyclische Gruppe einen 3 bis 8-gliedrigen Ring umfaßt.

3. Cyanfarbstoff bildende Kuppler nach Anspruch 1, worin $R_2$ aliphatische Gruppen, aromatische Gruppen, heterocyclische Gruppen, Alkoxygruppen, Aryloxygruppen, Alkenyloxygruppen, Aminogruppen, Acylgruppen, Estergruppen, Amidogruppen, Sulfamidogruppen, Imidogruppen, Ureidogruppen, aliphatische oder aromatische Sulfonylgruppen, aliphatische oder aromatische Thiogruppen, eine Hydroxylgruppe, Cyanogruppe, Carboxylgruppe oder Halogenatome bedeutet.

4. Cyanfarbstoff bildende Kuppler nach Anspruch 1, worin $R_2$ mit dem Stickstoffatom des Pyridinringes verbunden ist, und $R_2$ eine aliphatische Gruppe, aromatische Gruppe, heterocyclische Gruppe, Acylgruppe, Sulfonylgruppe, aliphatische oder aromatische Oxygruppe oder Aminogruppe bedeutet.

5. Cyanfarbstoff bildende Kuppler nach Anspruch 1, worin Y bedeutet:

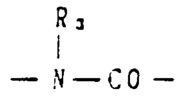 , 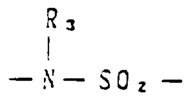 , 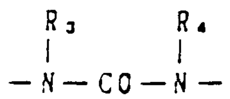 ,

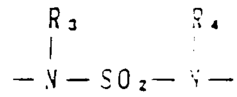 , 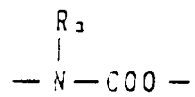 ,

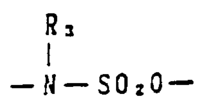 , 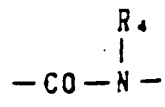

und

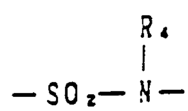

worin $R_3$ und $R_4$ jeweils ein Wasserstoffatom, eine aliphatische Gruppe, eine aromatische Gruppe oder eine heterocyclische Gruppe bedeuten.

6. Cyanfarbstoff bildende Kuppler nach Anspruch 5, worin Y bedeutet:

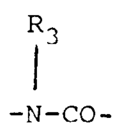 oder 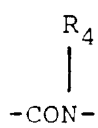 .

7. Cyanfarbstoff bildende Kuppler nach Anspruch 6, worin Y -NHCO- bedeutet, welches mit dem Pyridinring über das Stickstoffatom verbunden ist.

8. Cyanfarbstoff bildende Kuppler nach Anspruch 1, worin X ein Wasserstoffatom, Halogenatom, eine aliphatische oder aromatische Oxygruppe, aliphatische oder aromatische Thiogruppe, aliphatische oder aromatische Oxycarbonyloxygruppe, aliphatische oder aromatische Acyloxygruppe oder aliphatische oder aromatische Sulfonyloxygruppe ist.

9. Cyanfarbstoff bildende Kuppler nach Anspruch 1, worin $R_1$ und $R_2$ oder die $R_2$'s einen Ring bilden und der Ring 5- bis 7-gliedrig ist.

10. Cyanfarbstoff bildende Kuppler nach Anspruch 1, worin die Dissoziationsgruppe eine Hydroxylgruppe, eine Sulfonamidogruppe, die mit einer aliphatischen, aromatischen oder heterocyclischen Gruppe substituiert sein kann, oder eine aktive Methingruppe mit mindestens einer elektronenanziehenden Gruppe ist.

11. Cyanfarbstoff bildende Kuppler nach Anspruch 10, worin die Dissoziationsgruppe eine Hydroxylgruppe oder aromatische Sulfonamidogruppe ist.

12. Cyanfarbstoff bildende Kuppler nach Anspruch 1, worin -Y-$R_1$ bezüglich des N des Pyridinringes in 2-Position oder 5-Position angeordnet ist.

13. Cyanfarbstoff bildende Kuppler nach Anspruch 1, worin -Y-$R_1$ in 2-Position angeordnet ist.

**14.** Cyanfarbstoff bildende Kuppler nach Anspruch 1, worin die Kuppler eine sogenannte Ballastgruppe mit 8 oder mehr Kohlenstoffatomen in mindestens einem von $R_1$, $R_2$, X und Z besitzen.

**15.** Cyanfarbstoff bildende Kuppler nach Anspruch 1, worin mindestens eines von $R_1$, $R_2$ und X eine oder mehrere Pyridylgruppe(n) der allgemeinen Formel (I) besitzt/besitzen, von der/denen $R_1$, $R_2$ oder X entfernt worden ist/ sind.

**16.** Cyanfarbstoff bildende Kuppler nach Anspruch 15, worin die Pyridylgruppe die folgende Formel besitzt:

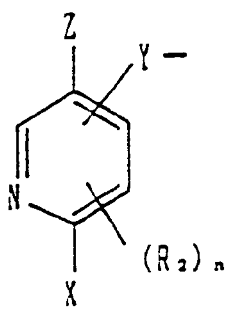

,

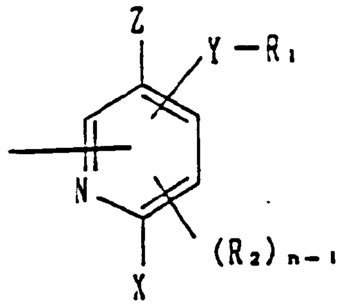

oder

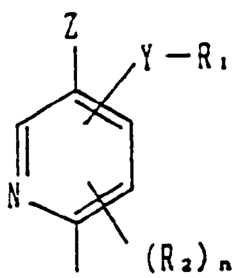

und die Kuppler Oligomere oder Polymere sind.

**17.** Lichtempfindliche Silberhalogenidmaterialien, die mindestens einen Cyanfarbstoff bildenden Kuppler der allgemeinen Formel (I), wie in Anspruch 1 beschrieben, enthalten.

**18.** Lichtempfindliche Silberhalogenidmaterialien nach Anspruch 17, worin der Kuppler mit einem Öl-in-Wasser-Dispersionsverfahren unter Verwendung eines hochsiedenden organischen Lösungsmittels dispergiert ist.

**19.** Lichtempfindliche Silberhalogenidmaterialien nach Anspruch 18, worin das hochsiedende organische Lösungsmittel mindestens eines der Verbindungen der folgenden Formeln (VI) und (VII) ist, oder weiterhin mit einem Hilfslösungsmittel vorliegt:

Formel (VI):

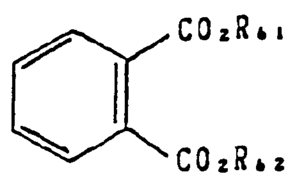

worin $R_{61}$ und $R_{62}$ gleich oder verschieden sein können, und sie bedeuten jeweils eine Alkylgruppe, Cycloalkylgruppe, Alkenylgruppe oder Arylgruppe, mit der Maßgabe, daß die Zahl der gesamten Kohlenstoffatome in den Gruppen $R_{61}$ und $R_{62}$ 4 bis 30 beträgt;

Formel (VII):

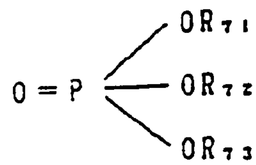

worin $R_{71}$, $R_{72}$ und $R_{73}$ gleich oder verschieden sein können, und sie bedeuten jeweils eine Alkylgruppe, Cycloalkylgruppe, Alkenylgruppe oder Arylgruppe, mit der Maßgabe, daß die Zahl der gesamten Kohlenstoffatome in $R_{71}$, $R_{72}$ und $R_{73}$ 12 bis 60 beträgt.

## Revendications

1. Coupleurs formant un colorant cyan de formule suivante (I):

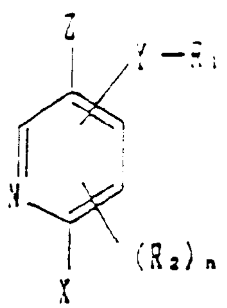

( I )

dans laquelle $R_1$ représente un groupe aliphatique, un groupe aromatique ou un groupe hétérocyclique ; $R_2$ représente un groupe ou un atome qu'on peut fixer au cycle pyridine; X représente un atome d'hydrogène ou un groupe qu'on peut éliminer; Y représente un groupe de fixation divalent qui possède au moins une liaison amido et/ou une liaison ester;
Z représente un groupe de dissociation ayant une constante acide de dissociation pKa de 5 à 12; et n représente 0, 1 ou 2 à la condition que lorsque n est 2, deux groupes $R_2$ représentent soit des groupes identiques soit des groupes différents ou soit des atomes identiques soit des atomes différents et ils se combinent ou non pour former un cycle; quand n est 1 ou plus, $R_1$ peut former un cycle avec $R_2$; au moins l'un des groupes $R_1$, $R_2$ et X peuvent posséder un ou plusieurs restes pyridyles de formule générale (I) dont on a éliminé un ou plusieurs substituants $R_1$, $R_2$ ou X; quand n est 2 et si l'un des groupes $R_2$ est un groupe hydroxyle et si le groupe hydroxyle est en position o ou p par rapport à l'atome d'azote du cycle pyridyle, on peut fixer un autre groupe $R_2$ à l'atome d'azote du groupe pyridyle et le groupe hydroxyle en position o ou p peut former une cétone par tautomérisme.

2. Coupleurs formant un colorant cyan selon la revendication 1, dans lesquels le groupe aliphatique a de 1 à 36 atomes de carbone, le groupe aromatique a de 6 à 36 atomes de carbone et le groupe hétérocyclique comprend un cycle de 3 à 8 maillons.

3. Coupleurs formant un colorant cyan selon la revendication 1, dans lesquels $R_2$ représente des groupes aliphatiques, des groupes aromatiques, des groupes hétérocycliques, des groupes alcoxy, des groupes aryloxy, des groupes alcényloxy, des groupes amino, des groupes acyles, des groupes esters, des groupes amido, des groupes sulfamido, des groupes imido, des groupes uréido, des groupes aliphatiques ou

aromatiques sulfonyles, des groupes thio aliphatiques ou aromatiques, un groupe hydroxyle, un groupe cyano, un groupe carboxyle ou des atomes d'halogène.

4. Coupleurs formant un colorant cyan selon la revendication 1, dans lesquels $R_2$ est lié à l'atome d'azote du cycle pyridine et $R_2$ représente un groupe aliphatique, un groupe aromatique, un groupe hétérocyclique, un groupe acyle, un groupe sulfonyle, un groupe oxy aliphatique ou aromatique ou un groupe amino.

5. Coupleurs formant un colorant cyan selon la revendication 1, dans lesquels Y représente :

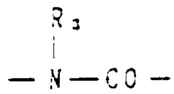 , 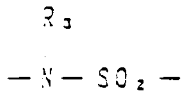 , 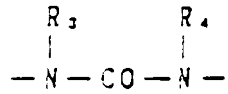 ,

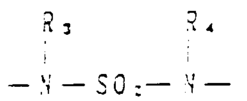 , 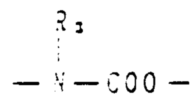 ,

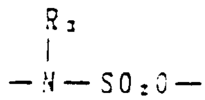 , 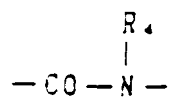

et

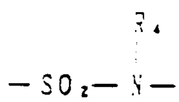

dans lesquelles $R_3$ et $R_4$ représentent chacun un atome d'hydrogène, un groupe aliphatique. un groupe aromatique ou un groupe hétérocyclique.

6. Coupleurs formant un colorant cyan selon la revendication 5, dans lesquels Y représente :

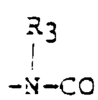 ou 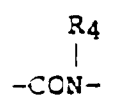 .

7. Coupleurs formant un colorant cyan selon la revendication 6, dans lesquels Y représente -NHCO- qui est lié au cycle pyridine par l'atome d'azote.

8. Coupleurs formant un colorant cyan selon la revendication 1, dans lesquels X est un atome d'hydrogène. un atome d'halogène, un groupe oxy aliphatique ou aromatique, un groupe thio aliphatique ou aromatique, un groupe oxycarbonyloxy aliphatique ou aromatique, un groupe acyloxy aliphatique ou aromatique ou un groupe sulfonyloxy aliphatique ou aromatique.

9. Coupleurs formant un colorant cyan selon la revendication 1, dans lesquels $R_1$ et $R_2$ ou les groupes $R_2$ forment un cycle et le cycle est un cycle de 5 à 7 maillons.

10. Coupleurs formant un colorant cyan selon la revendication 1, dans lesquels le groupe de dissociation est un groupe hydroxyle, un groupe sulfonamido qu'on peut substituer par un groupe aliphatique, aromatique ou hétérocyclique, ou un groupe méthine actif ayant au moins un groupe qui attire les électrons.

11. Coupleurs formant un colorant cyan selon la revendication 10, dans lesquels le groupe de dissociation est un groupe hydroxyle ou un groupe sulfonamido aromatique.

**12.** Coupleurs formant un colorant cyan selon la revendication 1, dans lesquels -Y-$R_1$ est placé en position 2 ou en position 5 par rapport à N du cycle pyridine.

**13.** Coupleurs formant un colorant cyan selon la revendication 1, dans lesquels -Y-$R_1$ est placé en position 2.

**14.** Coupleurs formant un colorant cyan selon la revendication 1, dans lesquels les coupleurs ont ce qu'on appelle un groupe ballast qui a 8 atomes de carbone ou plus dans au moins l'un des groupes $R_1$, $R_2$, X et Z.

**15.** Coupleurs formant un colorant cyan selon la revendication 1, dans lesquels au moins l'un des groupes $R_1$, $R_2$ et X a un ou plusieurs groupes pyridyles de formule générale (I) dont on a éliminé $R_1$, $R_2$ ou X.

**16.** Coupleurs formant un colorant cyan selon la revendication 15, dans lesquels le groupe pyridyle a la formule suivante :

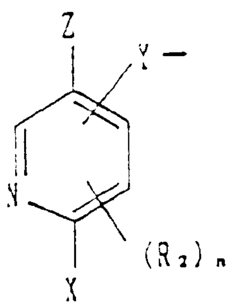 , 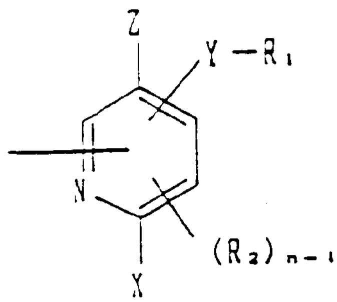

ou

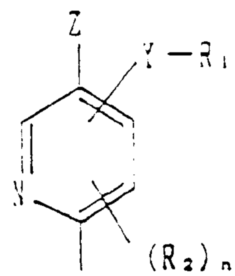

et les coupleurs sont des oligomères ou des polymères.

**17.** Matériaux photosensibles d'halogénure d'argent contenant au moins un coupleur formant un colorant cyan selon la formule générale (I) indiquée à la revendication 1.

**18.** Matériaux photosensibles d'halogénure d'argent selon la revendication 17, dans lesquels le coupleur est dispersé à l'aide d'un procédé de dispersion huile-dans-eau en utilisant un solvant organique à haut point d'ébullition.

**19.** Matériaux photosensibles d'halogénure d'argent selon la revendication 18, dans lesquels le solvant organique à haut point d'ébullition est au moins l'un des composés de formules suivantes (VI) et (VII) en présence éventuellement d'un solvant auxiliaire.

Formule (VI) :

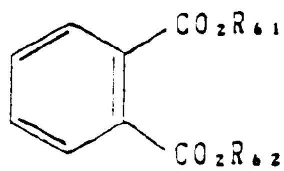

dans laquelle $R_{61}$ et $R_{62}$ peuvent être identiques ou différents et ils représentent chacun un groupe alkyle, un groupe cycloalkyle, un groupe alcényle ou un groupe aryle, à la condition que le nombre total d'atomes de carbone dans les groupes $R_{61}$ et $R_{62}$ soit compris entre 4 et 30:

Formule (VII) :

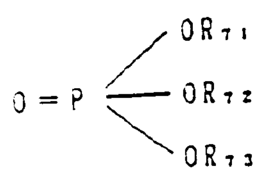

dans laquelle $R_{71}$, $R_{72}$ et $R_{73}$ peuvent être identiques ou différents et ils représentent chacun un groupe alkyle, un groupe cycloalkyle, un groupe alcényle ou un groupe aryle, à la condition que le nombre total des atomes de carbone dans $R_{71}$, $R_{72}$ et $R_{73}$ soit compris entre 12 et 60.

Fig. 1

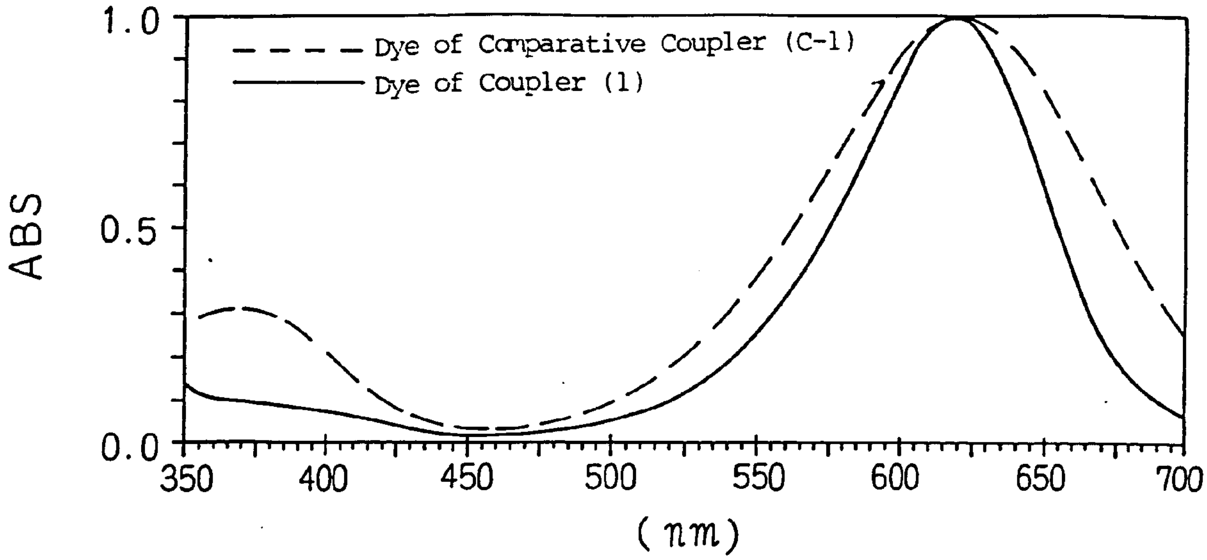
1.0
0.5
0.0
ABS
Dye of Comparative Coupler (C-1)
Dye of Coupler (1)
350
400
450
500
550
600
650
700
(nm)